# EUROPEAN PATENT APPLICATION

(11) **EP 4 726 035 A1**
(43) Date of publication of application: **15.04.2026**
(21) Application number: 24818760.1
(22) Date of filing: 07.06.2024
(51) Int. Cl.: C12N 9/22, C07K 19/00, C12N 15/113

(54) **CAS ENZYME AND SYSTEM AND USE THEREOF**

(30) Priority: 09.06.2023 CN 202310684902
(71) Applicant: Epigenic Therapeutics Pte. Ltd., Singapore 068908 (SG)
(72) Inventor: FENG, Zhengyan, Shanghai 200131 (CN); WU, Leilei, Shanghai 200131 (CN); MAO, Shaoshuai, Shanghai 200131 (CN); YANG, Changqing, Shanghai 200131 (CN); ZHU, Qi, Shanghai 200131 (CN); GUAN, Jingwen, Shanghai 200131 (CN); ZANG, Ying, Shanghai 200131 (CN)
(74) Representative: Weickmann & Weickmann PartmbB
(86) International application number: PCT/CN2024/097935
(87) International publication number: WO 2024/251229

(57) **Abstract**

The present application relates to the field of biomedicine, and specifically relates to a novel Cas enzyme, a system thereof and the use thereof.

## Description

### TECHNICAL FIELD

The present application relates to the field of biomedicine, and specifically relates to a Cas enzyme, a system thereof and the use thereof.

### BACKGROUND

Recent advances in genome sequencing technologies and analytical methods have significantly accelerated the understanding of the genetic basis of biological activities in different fields, from prokaryotic biosynthetic pathways to human pathology. In order to fully understand and evaluate the huge amount of information generated by gene sequencing technology, it is necessary to improve the scale, efficiency and ease of use of genome and epigenome manipulation technology accordingly. These new genome and epigenome engineering techniques will accelerate the development of new applications in many fields, including biotechnology, agriculture, and human therapeutics.

Clustered regularly interspaced short palindromic repeats (CRISPR) and CRISPR-associated (Cas) genes, collectively referred to as CRISPR-Cas or CRISPR/Cas systems, are currently understood to provide immunity against phage infection in bacteria and archaea. The CRISPR-Cas system for adaptive immunity in prokaryotes is an extremely diverse set of protein effectors, noncoding elements, as well as loci structures, some examples of which have been engineered and adapted to generate important biotechnologies. Components of the system involved in host defense include one or more effector proteins capable of modifying DNA or RNA and RNA guide elements responsible for targeting the activity of these proteins to specific sequences on phage DNA or RNA. These guide elements can be reprogrammed to target alternative DNA or RNA targets.

CRISPR-Cas systems can be broadly divided into two categories: class 1 systems composed of multiple effector proteins, and class 2 systems composed of a single effector protein that complexes with an RNA guide to target DNA or RNA substrates. The single-subunit effector composition of class 2 systems provides a simpler set of components for engineering and application transformations, and has been an important source of programmable effectors to date. Characterization and engineering of class 2 CRISPR-Cas systems using CRISPR-Cas9 as an example paves the way for diverse and wide-ranging biotechnological applications in genome editing and other aspects. However, in addition to current CRISPR-Cas systems that enable new applications through their unique properties, there remains a need to develop powerful genome engineering tools, i.e. alternative programmable effectors and systems for modifying nucleic acids and polynucleotides (i.e. DNA, RNA or any hybrid, derivative or modification thereof).

### SUMMARY

In one aspect, the present application provides an isolated Cas enzyme, the isolated Cas enzyme comprises an amino acid sequence as set forth in any one of SEQ ID NOs: 1-58 or a sequence having at least about 80% identity to the amino acid sequence as set forth in any one of SEQ ID NOs: 1-58. For example, the Cas enzyme comprises a sequence having about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identity to the amino acid sequence as set forth in any one of SEQ ID NOs: 1-58. For example, the present application provides a Cas enzyme comprising the amino acid sequence shown in the following table:

| **SEQ ID NO:** | **Number in the present application** | **Amino acid sequence (* may be any amino acid)** |
|---|---|---|
| 1 | EpiCas001 | MNNNGETKCYGNNLDRFIGLREVNKTLRNELIPTDSTRENIEKN GIISEDELRAEMGHELKAIMDDYYRNMIEETLSRKCNINWSTLF AAMDSVARSANKKDADKILEKVQKEKREEIHKLFKEQGNFEKL FKAELVSDVLPDFIGKYEGYSDEERISKLGTIKLFKKFMTSFKAY FDNRKNVFSKENISTSICHRVVNDNAFIFWDNLNAFKKIDKYAH DAVETIEGEISGKLGEWKISQIYSVDFFGMVIPQSGITFYNEVSG EINKYMNIYCQKEKQNSRQFKLKRLHKQILCIGETSFEIPEKYES |
| | | DEEVYQSVNDFIGMIKNNDIISRLEHLAENVNKYDADKIYINSK SYENVSKCIGGTWNTIKDCLRLWYNENIRGSGKNKEKKVGKA VEEDKYKSIAMLNQLLENYSDGDGRIYNVQLYVEHITKIIGDYK PEELVYDANIRVIEQDEKAEEIKSVLDIYIAILHWIKAFIIEDAVE KDTDFYYEIEEIFDKVQSIIPLYNMVRNYTTQKPYKQEKIKLKFN TPTLAEGWHKSKEYSNNAIILQRDGKYYLGIFNAKNKPDKKIIE GCSQKKNKADYAKMVYYLLPKPHMNLPHIFIKSKEAVKNYGL SNYIVEGYDKDKHIKSSNGFDIKYCRDLIDYFKDCISKHPEWKN FNFSFTDTALYNDIGEFYNEIKRQGYKIEWTYISEEEINRLDENG QIYLFQIYNKDFAEGSKGTPNLHTLYLKNLFSEDNLKDPVFMLN GNTEVFFRKSSIKKPVVHKKGSVLVNRNYIDLNGNRITIPEKEY KEIYEYCNGIGNVNLSESAMKYYDMAKHYTATMDIVKDYRYT VDKFFIHMPMTINFNAESNNNINELALNFVAKNDNIHIIGVDRG ERNLIYISVIDSKGNIIKQKSYNVVNDYDYKSKLKEREYDRGDA RKNWKRINNIKELKEGYLSQVVHEIAQLVIQYNAIIAMEDLNYG FKRGRFKVERQVYQKFENMLINKLNYLVDKRKNADEAGGVLK GYQLTYIPERLKNMGRQCGIIFYVPAAYTSKIDPTTGFVNLFNFN AITSQEGRRKFINKFKHIRYNLNMNLFEFEFDYNDFITHNTQIAK TEWSVYTNGPRIKKERVNGKWNDGYEADITEVMKQKLSDNGI DYADGGDILTRIAEADDKLVSGIFEIFRLTVQLRNSKSESCGEDY DRIISPVLNESNRFYDSDEYSGENEILPVDADANGAYCIALKGLY EVKQIRENWVEGDKLSGDLLKISNADWFDFIQNKRYE* |
| 2 | EpiCas002 | MNIKFNKFTGVKPVSKTLRNALIPTEFTMVNLERNGIISADELRA EKRQELKKIMDDYYRDLIERTLAENHSINWHILFELMKSKMHG DEKDTAKKLENEQKSKREEIHRLFAEQEEFGKIFSAKLVSDILPG FVASYAMYNDEDRTEKAEVVKLFNKFMSSFTEFFENRKNVFSK |
| | | ENISTSLCHRIVNDNALIFLNNMDSYKRIMENFKSESELVSKGLE TMLGEWKISQIYSEDFFGMTMTQSGIEFYNDICGELNLVLNLYC QKSKKNSARFKMKRLHKQILCTEKSSFEISYKYDNDKDVYTSV NGFIKNIKNNNIIERLLKLGTETEQYDLNKIYISSQSYETVSIAIGG TWETIRNCLTSWYDDNIKGSGKAKENKIKAVVKADKYQSIARV DYLTETYNASGTDLQKAESYLSHIVQIIGDYEPEELYFDESIHLIE NGDKISEIKKILDIYIDVVHWIKTFEISESVEKEPEFYYELDDIYD KILDAVPLYNKVRNYVTQKPYNQEKFKLNFDSPTLANGWSKSK ERDNNAIILSRDGKYYLGVFNVRNKPDKEILEGHSKPKNETDYA KMVYNLLPGASKMLPKVFLSKKGIDNFKPSDYILEGYIKKKHIK SSETFDIKYCRDLIDYFKNSISRHPEWKNFGFNFTETSKYNDISEF YKEVEKQGYKIEWTYITEEDINKLDEAGQIYLFQIFNKDFAVGS KGTPNLHTLYLKNLFSKENLDNVVLKLNGEAELFFRRSSIKTPVI HKKGSILVNRCYVDADDGKRKPIPEKEYMEIYNYCNGKRDTEL SESARKYYNISQRYEATMDIVKDYRYSVDKLFIHLPITINFKATE NKNVNDMVLEYIAKSDDIHIIGIDRGERNLIYVSVIDMHGHIVM QKSYNVVNEYDYKTKLKECEQGRDAARKNWKEIGKIKELKEG YLSMVIHEITQLIIEYNAIVMMEDLNFGFKRGRFKVERQVYQKF ENMLINKLNYLVDKHKAVDEPGGLLNGYQLTYIPDRMSELGR QCGIIFYVPAAYTSKIDPTTGFFNAFRFNLLTSAESRRDFLNRFK CIKYNGSMNMFEFDFDYNDFITHNTTIARTQWKVFTNGGRIKNI ISKSGWNGTKKIDDITALMKEIFDSRKIDFSDGGNLLPEILAYEG ADKDKFIVELLDIFKLTVQLRNSMPDGSDIEYDRIISPVINENNTF YDSDMYTEPNSPLPIDADANGAYCIALKGLYEVNQIKENWKEG ETFPKSVLKLNNANWFDFVQNKRYE* |
| 3 | EpiCas003 | MNLAFLNRGYNYGMINRIERGPFSELGDQQLLEQTRRLAANQR CLEVHILDHLDEIDRRGLALRRGFSSLFDYAVRELRFSDAAAQR RIQAMRLCRRHGCVRASLQSGELGMTSAAQLETTFAGAERAER KSHTGHRNVGQGRGPDQDQDSVGSTRQLGLPAAPGEADEQGE RGHRSAPEGAPPAAECSGDARERGHRSAPEGAAPAGEYTGEVA ARTTPAVAVAVASRSGAQPPGGVHAGALLPRPELNGNAAPVQ APSRAPVAAPGAPGAGAGAGPAAPASDEGPAAQLLPPTEFSAST PEPEPVTAPAELRHTASRAPEPPRDDPFAELVPGTVSAAPLLHPQ RQRELIEQAAGMSTRQVASLLAAAAPEVLPPRDTLRAVAPDRY TLKVSIDQECEQGLRLLKDLMAHLDPRMSWGDLVARVVREAV ARHDPRGGGRGQRRKGADSAGASPRRTPKETRAAGAAGGPAR RVGESTGREPNRTGAAGAAGGPAQREDESSGRAQNRTRAAVA PVAAARRRGGATSAPQSAPAVLVDGARAGGGPAGAPAAPPAP PGDTAATERQNPIEVLTSAAPPSQSAPAAPSDGAHAGGAPAGAS AATSAPQSAPAAQLDGARAGRVPSGAPAATSAPQFASGALLDG SNAGAGVPGAPVATAAPQPALGGSPDGGPSGASAGASAGAAG GASADGFSARAAGPGASVATSAPKSVLGGFPDGGPPDAAAGA AAHTSREPVHSRRQSPRRRHIPAAVRRHIWHRDGGRCCYRDPL TGRRCTSAHLLQIDHLVPVAEGGGPEPSNLALRCFAHHRMRHG YAPAASPQPPI* |
| 4 | EpiCas004 | MDGLARVDRAVTACLAAPAWALPEEALVEALDEAHRLGQRL AVLERVLVREVDGRGLARAQGATSTAGWLRDRLRVSIQSARR LVADAGWLDAADDPGTTDDPGTTGDPSGTTDDPGTTGDPSGT RTYASVEPGTGIGAAGAPSPADALPDAAGRTGLDGAVTTGPGP GAGGAGGAGGADRAGAPGLSAAAGCGTAAAGPVLREALATG RVNLEQARIIAGAVESVRKEAGPDTADKALYTLLADARRFEPR |
| | | ALRALADRILWHVAPDAAEEADRAALRRADDEAQRRRHLTLS ATDDGRMRLRGLLDSETSATLLAALDPLTRPAGLGDDRTSGQR RHDALGDICRLALRTGELPDNGGEPAQIVVTTEYAALTARLTA VGLPAARQPGATSGTRSSIARPSPEPAPAERGPTEPTPAEQGSIES TPAERRPTEHTPAERRRTERMPAERGAARRTQSGIGRLRRTTHE RGGCGPRPPQRAAHNCGQPGRTGRAPAAGSGQRRPERPVPRIR NGTAGTTGTATGRWQRPRGAPTETPTETAVSGGPPTPQSSPPAD PSTLGTQRGSPTTDQRPASTDRAPATPVHEATAPAQDALAVQD AAATVQRSPRLDHGCATADQQQDPGRDQDPGRDRAPGRDRHS RGPDRDQWQARDRDHGRQGRGRLGPGRLDNDLRLTPATVRRL ACDAAILPAVLGSQGQVLDLGRQRRLFTGPLRRALVLRDRGCA FPGCDRPPRWCDGHHIQHWADGGATALHNAVLLCRHHHRVIH QDDWTVRIAADGHPEFVPPAWIDPEQRPRRNRYHRRA* |
| 5 | EpiCas005 | MVVIECTVPDCEFKTTDASEAVAIALLANHNLVHRNEAQPRRL PANTAPKGPKLDRPKIDVGVTMEEWNIFTRRWEVFRAGSGIDE NSAPSQLFQCAGTELGDSILKANPNAVNESLTQLMEAMRSLAII PIATCVLRTELLQMRQERDESFRSFTAKVRGKAETCAYNAKCS CSRPVDYTDHVIRDVIINGLYDNEIRREVLGIDGILEKPINQVIAV VESKEMARNALPPSPSLSAVSSFQRQKTNSPHPAPSPPQADQAK EATCPGCQKTFKLFTEGARGWNKKPHQLCIDCHRVNRRARRPQ RQPQQRSPQAPTVHANQAEPIAQISSLHGETKQDTRPTTHATTA KRPVRLDHHIFSKGEWKRARFRDHPRVPITISLARKGRHRKETA DTTPDIHAAVSAIADTGAQSDLWSLADFLACGFSREELQPIRPSL TAANNSPISIEGAFFAKLSATSYSGQSTTSFSMVYVSSSVRDMYL SYDSLLNLDLLPHCFPRHDDVTEASNERRPASQNKTRPNYTSPT TNAARIVNEGCTKPASEHDVTCSCPQRETTPPRPSELPFPCTPAN |
| | | NTKMKAWLLNRYASSTFNTCPHRALPCMEGPPVEIHLDPTAKP TACHTPANIPLHWQKRVYEDLLRDEALGVVERVPYGEPVTWC HRMVVTRKHDGSPRRTVDLSPLNKYCQRETYATESPFQLARRI PNDTWKTVTDAWNGYHSVPLRSSDRHLTTFITPFGRWRYTRAP QGFLSSGDRYNRRFDAILAEFERKERCVDDTVHYDHDLEQHW WRTIEFLTRVGQAGIVLNPDKFQFAGRSVDFAGFHVSDHTIEPL PKYLDGIREFPTPTSTTDIRSWFGLVNQVSNYAQLRDLMAPFKP FLSPRHKFLWSSELEEAFQASKEKIVKAIREGVEIFDIHKRTCLRP DWSKRGIRYFLLQQHCSCSSGMPDCCPGGWGITLAGSRFLSPAE QRYAAIEGEALAVAWGLEQTRYFTQGCDNLVIITDHKPLVKIFG DRTLDEISNSRLFRLKQRTLPWRFEIHHLPGKSNYAADATSRHP SQSNTEEKAPLCPANPPDAAEIALMTAIRDTAQELGAISWTLLA QETAADARMGNLLHSLEQGTQLNKDDPTLANLGPIYESIYQEE GVLMYQDRVVIPPSLRQRVLQSLHAAHQGTSSMEQRARSIVYW PGMSNDIRVTREKCHDCNRNAPSQARTPPLPTAPPSTPFEAVFA DFFNYKGYHYLVVGDRLSGWVEVLSSPATTNLAGSTGLIRHLR TFFATFGVPEELSSDGGPEFTAKATEDFLRTWDVKQRLSSVSFP QSNGRAEVAVKTAKRLLLSNTGPTGSLDQDRFLRAILQLRNTP DPDCSLSPAQIIFGRPLRDTLAFVNRLEKFSNPHIRPLWRQAWA AKEDALRTRISRTTESLREHARPLRPLAIGTRVFLQNQQGPNPN KWDRSGVVVEAAGHDQYRVKVDGSGRVTLRNRRFLRAYTPA LPAIPQAPSGAPAKTPSMAPDAPSSPSQTCHHTNRTATPATTPG VPTPDLATAALALQVSPEEPDAAEEKQGPSTPEAMTLPSPPNAA ARPRRTIRRPKQFEPETGTWTE* |
| 6 | EpiCas006 | MASIASLAPDGWLERKELSDGVSDDGLSDDESLLEERDTLETIR DSLTATNPDEIDGLLGRPSLGSDGLPSPRTPSRRREFLGERKGSK |
| | | TRLSPTPPAAVPAPTCAPASTKNLWRGFALFAMAMACAALSLQ RSPELVEYGPSRRLQTSFGERVDEAADGATSRRLQNSNQFCAA RDGWEAFVGTARAGEAFGGEVCLLASTSTESSTLYGSHAAYSP PMRVRAEFLAADDLTRQLGLVFGVPAWPDAANLPATGYACVL EHKVTRWSSKSRLVLRRANADYDKNPDWINFKRSRWFDSEPN AWYEATAAIDDTSAAIAGKVGADVSCEVRDDGGAVVASVRKE DRWVMAHPGKVGFYSERDDLGERFVRALEVDGERAPEEPSFC CYWSPTSDMCGDCPDPQTDGWCAASRARCEGCGGTFCDAPTP PKEVPGRCCYDGAGCGSCSASDDDGFCAKSESNCGSCGGFFCS LAPTPAPTRVPSPRPTTPSPTAAPTVAPGHPTAAPATYAPSSSEPS SKPTTSTPSAQQGCACEEGPYEYDGKTFEGCTSYGWSVPWCGT TNCGICDGLVSTGCWDECEETYAPSSSEPSPKPTTSTPSAAPQGF PAIPLPTSATSPPTPTVFLTNADFRYGTYLITNPGVYVLREDIVFA PETPKMMPPLDSETYPPDRGFWLGFFAAISVAADDVVVDLNGH EIRSSREFLLRQRFFSIIELADKPFKANSGPPQFGALDAPLVPARR VHVKSGTLGLSSHMGIHGNDNEDVTVTDVVIRDFETGGVQLNG ASRVRLERVTVGPSLGHEHSAGPVPALATLSQATNLLRVAEGT RHERQEEMVLLSAAVERFVQETLAGEDTSASVFASEAAGYAGL PDGSATVWIPTTGLGGPHQTSELSSSVKSTSIRLIFGRIDCSRRVL EKQPKSLCRNRRIREN* |
| 7 | EpiCas007 | MAGNHFKGSDSGRPAVPPRPNGAGKAGTPGGAGQGGSGKRVS PGETAMFTALYEQSQKAKKTGRARGNVFAGGATFASQPSGAPS VPANNAGAANAANGAGNVNAGKADNNTPSAGGAGLTGNLGT IYTGASETGTFARLDDSGAEFAGSGSKEDYYDFGLNYGSDASSS STSDGLVRHRHRKGERKKRHTGAIIAAVVAVLLVALGASGFML LNSAKTVKSEAKEAVEIVGGLKDKVTSGDFSTLPDDAKKIDELC |
| | | DSMKAETSSPLWAAASFIPVYGSDINAARTMIDALSDVSSNALV PMADNLSQATPGKLFQDGMINVSALQAVADSLSSSSKVFKSAN EKVQGIGDTHISQVTELVDKVKDGFATLNGAVDAAEKVAPVLP QMLGANGQTRNYLMYAMNNVEIRACGGFGGSQGLISVTDGQ MSIGEFVPRIGLSEDEAVESVDEEDEALFGNHSNLYNSGNTYSP DWPRNSQRVAALWKSQYGQDVDGVIGIDPVFLQYLLGLVGNV SLPDGTVVDGTNAAKVLMHDVYWNYPVEESDGIFASVASAAF DKILGGIGDVDVTKLVSAVERGAEEGRLIAWMRNDDEQNAIKE TGIDASLPDPDDPSADPVAGVYFNNLSFSKLDWYLNADTQIGQ GIKNGDGTCSYRITVTLTNIMTQEEAGKLPDYVAASAPDAARD DERLNVSLFAPTGGNISDLTVEGTQFGLGAATWHGIPFYSGTVD LHAGETTTITYTLTTSAEAGDKPLTLRQTPTCQAARDSASA* |
| 8 | EpiCas008 | MEAGRDFIAFGQDVQGTDCCRGAGGAGLAGPAGRSGAGAKN VRPPCQTVGMEAGRDFIPFGQVVEGTDRDCCSGAGGAGLADA GAAGVSGVGGVLAGVRQLCAGVVEDSALWDLGAASDFAGGV EEISRAVEYLQLVAASAVDRARTQSAAAAAPGMSWTTGWRDE DPASDENSGTTAPVPVEPPSAAATGSDDGYRNTVEFLRARLRIG AAEARGRLALGAGVLPRQGLAGRSEAAVHAELGAAVAAGAV GSRAAAVIEAALGRVRHVGDAAAAARMEHALTRTAAENDTDF LARVARSWAEAMDQDGAEPSEELLRQRQGAFIRRQRNGLHHL EIFATAEQFEQLVTVMNTATNPRTGTGSGTGAGHGAMNAPAA DTPEDAGHGSEPATDGPAGPAAVQEPAQDWAPAATGGAGWET GPAAGAGWIVGPAAVSASDTRAGVVSTSAAGATGDEFVSDPGP GYGFGPVPDPGPGDHRAEAGDTGAGGLDRRTRAQKQLDGLVG ACQISLAAGTLPASGGLRPQVMVTIDYRDLLARLGNGTGTTSDS AAPAGTATDCPVHMAGTVDVAGTVDMAGTVDMAGTVGAAG |
| | | SGRRTGGLPGTGSLLFTGPVTDSTIRKIACDADIIPVLLGTADRV LDIGRASRIFPPHIRKALIARDQGCAFPGCTIPASWCEAHHIDYW SRGGSTGTENGTLLCSHHHHLIHKEHWTIRVRAGIPWFIPPPHID PAQTPRRNNYFHPARLDPAA* |
| 9 | EpiCas009 | MPNRKVTLTAFSRMDHRVEALEEGLNEVRASIAGVQATMRES YANIVALLEKHLGKSIATEGGTSEKKDKPESSEVKNNVTLEFRQ SAKKVELPSFDGDDPAGWISRAEVYFRVQGTTPAVKVSLAQLC MDGLTIHFFNSILRENEDLSWEELKEALLERYGGHGEGDVYEQ LTDLKQEAIDDYITEFEYLVAEIPRLPEKQYRGYFLHGLKPEIKG RVRSFAALSEMRRTKLLQVARVVEREVEGGSELKPNRGPRFGF GSGSHKNGSQSDWVMVRRREGSRNEGVKSNTDGPKGDRKRIG PRDRGYTQLTYNELMDYKQKGLCFKCKNPFGPMHRCPAKELR VLIVDDVENEEDEERILAVEVGEEEDEKGEMSILNLHHITHETH HTMKFQGTIRGVEVLILVDSGAIHNFISQKLVHQMDWPIEPTAE MNVKLGNGFQISTQGVCRGLEMCIGDLKMNPEMHLFELGGIDV VLGIDWLKTLGDTITNWKQQTMSFWKDKSWVTLKGHEGCRR QTVALQSILNRPKPTSQGLLWEMDNVKHKGKGVNELSIQQQAE MEVLFNKFEHVFQTPTGLPPKRNRDHAITLTDGHGPVNVRPYR YPHHHKNEIEKQVKEMLTAGIIRHSTSSFSSPVILVKKKDGTWR MCIDYRALNKATIPDKFPISVIEELLDELHGARYFSKLDLKSGYH QVRVKDSDVGKTAFRTHEGHYEFLVMPFGLTNAPATFQGLMN DIFRPLLRKNVLVFFDNILVCSKDWETHLHHLEEVLRLLAAHSL VANQKKCYFGQRSIEYLGHMITEEGVAVDSRKVDSVLSWPQPK NVKRVRGFLGLTGYYRKFIRDYGKIAKPLTELTKKDGFKWGVE AQKAFDELKKRLTTAPVLTLPNFSKEFVIECDASGSGIGAILMQ |
| | | DKKPIAYFSKALGVRNLTKSAYEKELMVVVLAIQHWRPYLLGR KFVVSTNQKSLKQLLQ* |
| 10 | EpiCas010 | MIKDFASLPPSHQHKLSLQCQLSTIDDHQTHVIPVLIDSGCTDSII DEAFVRQHNISTKPLPTRVIVSNADGTKNRTGPLTEYVTLRLTV AGRTELMDFLVTGQREARILLGHDWLRRTNPNINWQTNTITYP SDDTEYHPEDGDRVIAVDLQSRIDKISVESNVSQTKSQQIAEESE RAKSTLTFRDVVPPFYHHFEPVFTKASFDKLPPRRVWDHAIELL PEAKGLDCKTYPLNRDEQIHLDEFLKENLETGRIRVSKSPYASPF FFIKKKDGSLRPVQDYRKLNALTIKNRYTLPLISELINRLKGARF FTKLDVRWGYNNVRIKEGDEHKAAFRTNRGLFEPLVMFFGLTN SPATFQTMMDRLLRDLINEGSVIVYLDDIMIFTDSLEEHRKIVSK VLQILADNQLYLKPEKCEFEKKEDEYLGMIISHDTIRMDPLKVQ AVAEWPIPRTKKELQSFLGFSNFYRRFIRDYGKIAKPLTILTGKV DWEWAEDQQRAFDVLKKTITTSPTLAIPNDEDPFKVECDASNY ATGAVLSQQQEEKWRPVAFLSKSMNPAERNYAIWDKEMLAV MHALEEWRHLLMGAKHQFTIFTDHQNLTYFRDPQKVNRRQAR WMDTLARYDFKLEHVKGSANTKADLLSRRVDHPRGERDNEG QVLLPQLAEANIYTMEDVLEEHYLDELLASQHKLDDYVVRKL KEDPKVWKKRDGIVWIGNEKEDELTLGSFENKIYVPQDPKLRA RIIEEEHDTSLSGHPGERKTHEGVNRSFWWPSLRRDIRKYVAGC EICQRTKSHRQKPHAPLHPNPIPDRPWQIITTDLIGALPESNGYD AIQVFVDRFSKMAHFVPTHVTLDLLGYAKQLRDSVIRLHGIPEK IISDRGPQFMSNFTKDFYQTLGIEPGLSTGYHPQTDGQSERINQD LEHYLRTFINHRQDDWADWLAIAEFTYNNHLHSATNQTPFFVN YGSHPRKGTGQKPSGRSQAAEDFATSMTKLHEQTRQSLLLANQ NLKKYYDRKRTEAREYHKGDKVWLEGTHISTDRPMKKLDDKR |
| | | FGPFTVKAKVGPSAYLLDVPRSWKAIHPVFNEILLSPYHTPEFPN QPRTSKPPPIQVDGAVEYEAKEIIASRPTKLKGAKLDYFVHWKG YPITERTWEPDHHLKNSPSLITTFHRKHPAAPRVIASAALRFRPY ENFTTTTEKPRLFDWITNRFTDCGAKILGTRI* |
| 11 | EpiCas011 | MSITDEYFQLTTKYRAQYGERTIVLLQVGAFFEVYGLKNPRTK VVTGSQIAEFAQICQLSVSEKNVCVEQDHVVMAGFRDYTLEKY LQKLSENDYTAVVYTQTKEGKNVTRQLHAIYSAGTYISYDNDS SQQITNHIMCIWIEKYQALDKSARLSCGVATANIFTGKSEIFEYQ TAYYMNPTTFDELERCVSTYCPSEVLVLSTTLPQDVVQTIVQYV GITHPSRHIFCASNMTDAVKNCTQQKYIHHMLTTFFGEESQQLC AEFSNNEIATQAFCYLLHFVQEHNPDLVRKIEIPRFSSQSKTMLL ANHTLRQLNIIDDLSASGKQAGQYSSVLSFLNKCASPMGRRLFQ QQLIAPTFDQEWLQKEYNMTELMLQPDTEPMIPMLRKQLATIR DIEKICRQIVLHKIYPDSIAHLYDSLQTIQQIHVCFAELPKVLEYL HHSTEKIDQVLHTMQTYLLIDRCRGIQSVQSFPHSIIQPGVSSSLD KIIQTYEENLQHFREIHAFFNKIMRAKEGGDAEYIKIHTTDKSGV SLQITKKRATVLKSLLKGLADPKIQLSGGLYFCVEDIKFSSASTS ADEIEIPCLKNICKALLTSEESMNQEIAKSFQDFLTTFENLHLENL EKCALFVAKLDVLHCKAYLASNYNYCKPTIVADHQKSFVDAR DLRHVLIEHIQINETYVPNDFCLNLPDQPLGVLLYGTNAVGKTS LIRALGIAVIMAQAGLYVPCSAFTYQPYQSIFSRILGNDNLFKGL STFAVEMSELRMILRMADESSLILGDELCSGTETESALSIFTAGL LHLHAKGASFVFATHFHEILKFEEMQQMPKLALKHMAVHYDP QRDCLVYDRKLQDGPGNRMYGLEVCKSLYLPDDFLQSALAIR NKYYPTSRGELTHHTSTYNTQKIRGICEMCKTNMGEEIHHLQPQ |
| | | KEANDKGFIGTIHKNHPANLMTLCEACHLKQHKSSITQKRKKT TAGYAVL* |
| 12 | EpiCas012 | MEIFISDNTLNMSKKEDVEPVETNKIMKEYLDLTKQYTKIYGEK TIVLLQVGAFFELYGLRDDKGTILESQIEDICIICQLNYADKKLM YNSKQVIMAGFREYMLDKNIPKIIEAGYTAVVYVQEKDGKNIK RVLHSVCSQSTYLSCETDSSPVMTNNIMCIWLDTFKPMDRNVS TVSKTRDTIVYGVSTINIFTGKSSMFEYQTPFILNPTTFDELERYV SVFSPSEILFVSSLEKEINRTILQYIGVKCPSIHIYDSRDMENKTIIA CSKQVYVQHMLSTFFGEEAMNVCREFQMNTMATQAFCYLLN YIQEHDKNIVRKISIPEFNNTSDRLLLANHTLKQLNIIDDSSIDGK KTGNLSSVLSLLNKCCSPIGKRNFKMMLLNPSTNETWLQQEYD MIEYMLQSENIYFIDMFRKQLSQIRDIEKICRQLLVLKVYPSSIYQ LYKTIGVIHNLHICLAENKKITDYLCKDISKEQINSYHYINDLCL QINEFLENHFVLDDCKNIQSLTSFENNIIKKGISQPLDDLIKHHYE KIAIFEFVKKQLNEFMYSNGQNRDIEFIKTHETEKSGLFLQITKT RANILKNYIKAKLEKDADYSFHNKELQFSFYLKDIKFGVASSAN EEIHIPILQKICKELITIKDEIDVVIRETYLEILKKIEDKWLPSLEQL ANYVANVDILQCKTYIAKNYKYCKPEIVKSEKSFVSAQELRHC LIEHIQQNEIYVANDICLGNEKSGMLLYGTNAVGKTSFIRALGIS IIMAQSGMYVPCSRFQFKPYTAIFSRILGNDNIFKGLSTFAVEMS ELRIILKMADKRSLILGDELCSGTETESALSIFVAGLMDLHDKDA SFIFATHFHEIINYDEIKMMNKLSLNHMSVIYDREKDALVYDRK LKDGPGTKTYGLEVCKSLYLTDEFLDKAYAIRNKYFPETRGEL NFKSSTYNVNKIRSICEVCKNEIAEETHHLEEQHTADTDGFIGHF HKNHKANLVSICKNCHDKIHHDNSGKNTLNGKQRKKTTKGYD FM* |
| 13 | EpiCas013 | MLLTEEYLEEQKKLELKYGKNVVLFMELGSFYEIYEANGIGKV KEISNILNILQTKKNKNKPESQRNPLMCGVPSVSLDKHLNRLAQ EEKWTIAIMSQTATDNGFERKITEIISPGTNVRNNIKDDTNSYLA SLILDKNTKGIYSCGLSLIDVTTGNIKVYEGYGVHNDKEKILDE VNLILENYNIKELILLDNLDSNKLYYKFPSINMKTSNKSIHYKIQ LITETFNKDFSELGLEQSPYIIDSLVGLLEFLIDHNKSLVVKLNSIT FINNKEFMYLGNNPLKQLNIIGDDFSVEKLINKGISAIGRRYIREQ ILNPIINADEINNRYKYSLSLSKSNKLPTIEKSLKNIYDIERLSRKL ELKTIKPFELGNLVTSLYASSDIIKNIKMLDTLTTFESYINLDLIK DYNSIHWNFFKDEQLMTLKTELELIMDLSNYEFDSFIKYSDKDG YYYEFTNKKYENLSFTSTIEHTRKLKNSIKLFTSELSSINTRRLVI ESKMMEISNVLFKDVLDIFDITIINIEINKIQRIEFYLNNTKLLAKK YCIPTILDDVENIFDVTNLRHPLVEHLEQEVFVPNSIKFNKENKG YLLYGQNSSGKTVLSKSIGIAIILAQSGFFVPAESLAFTIKDSLFT RIIGADNINKGLSTFAVEMLELKNILNRSTPDSMIIGDEISHGTET TSGVSIVASAILHFNAPFIIATHLHQLDSINDIKEKPFIHFIHLALTF DTSTNSLIYNRTIQKGKGSSIYGLEFAKSLDMPFDFLDKAYAIRK EIADDISGLEQLTISKPSSYNSKLFLGTCYLCDNKGEEVHHIKHQ HLADDDGFIDTLRKNHKSNLMVLCSSCHNKQHEKKI* |
| 14 | EpiCas014 | MSAKKGFSMIDEYFNIYNEKIKEYGKDIAVLIECGSFMEIYQIDN NEEKIGNAYEISKILNICYANKNGDLSNNNRSYPNFVGFTTNSM SKYLPILLENNYTVILVNQLETNGNKKGKLIQRGVTAVYSKCLQ PLEMDTNADNQLLYISLDVKLIHSNVLKRNKTTMYELNTSICCV NNFTNSIEIYENNYTFTKDCIENCLNDFDHLLYKFFSKDINIHLV GENIINFESELLKFFNSRYDNMRFHNFDKQKHKEYIKTDYQNEY FKKVYNHINFGLLTPIEYLNITTMPSLSIVNLMYTIDFIGRHDLK |
| | | YIRNLSIPKMMNDSDHLFLALDTLNQLNIDKGVFDIVNCTKTAI GKRYLKSLLCKPLKNPNEIEYRYKLTDELESIINTNNEIDQLLSCI IDFEKLHRKMSLEVLHPFEFEKLHTCYKNVNKLINVLSNYPLFH QIIPNNVKLNVFNEYINSYMETFDFDKLKKYNLNTSKDEISNYF LKGVIVELDEIQLKIDNITEEIERMRVKYNTICENNCKTKNPDYI KIAYTETEGYSFSCTKIKYQTLINAIKESSNFKIKQTNNIVKFFTP ELVDLSNKLLTFKDLLKNKINLHYISTLKLYYYNYHDLFTSMTT FIQILDVVNSNLKCKNKYNYCRPIIDDNENDSSFFKAVGLRHPII EVITQSNKYIPNDIVLDDNNIGILLYGLNSSGKSSLLRAIGIAVIL AQCGLFVPCNSFIYKPFHTVISQVDLSDNMYIGKSSFITEMAGLK KILDLSGKNTLVLADELCRGTETYSAISIVCSTMIKLIQSNTKFFF TTHLHQIPQLKRINTLKSIDICHLSVTIKNSNIIFNRKIEKTSGDPL YGLEVCKTIINDNDFINDAFEIRNELTRNNTQILSHKKSNYNKKK ITSYCEICNSCNSLETHHITEQYLADKNGIMPDGNHKNNLYNLV TLCHDCHLKVTLGKIKTRGYIQTINGRILDYNFTEDTTEKV* |
| 15 | EpiCas015 | MTTEHKKYEYLKFNKSLNEEYLLLQEKYEKIYNKDFTVVLMQ VGSFHEIYATNDRGYDLHKLSNILNIIVSKKNKKNPEVSEKNPY MIGFPILATPKFINILVDNGFHVIKIDQVTPPPNPKRAITQIYSPGS YIEGISSYDSNYILSIYIEEIKQMNNSYILFAGLSIIDLSVGKCIIHE TFSTKDDEKYSLDEVLKFIYNFNPKEIIVYYKNLITYSENELLTY LELNDKKYLISEFSDNEYTKIIYQNNFLKEIYNLETCMTHIEELD LERINNGRLSFILLLNYCKKMNSTILNNIYKPEFYLNTKYLHYG NNALKQLNVQTYNSNEKSLFDVINYTSTSMGKRYLNFNICNPIS DINILNDRYDKISELKEKNIDLSNKLKKILDIERLHRKISLQNLNP HNFVDLHISYSTILSLYEDIKKTKLNKIYNEKVKMNLENYISYYS RLFNLDEMEKYNLNDITNSFYNENINEKIDILQKSIKKEYKLIEN |
| | | LRDELEKFIDCKKKDYFNSFNEDKSPIQINYNEKDKYHFSTTSK RGSILKNNIKNNSKYNYDSYKFKDQSNTMKITCTYVDNISDKLV ILNDKIRPLIKNTYLNDLKEMYEKYNDLYLKINNIIAEIDFINSGS ICSIRNKYCKPKIILKDKSFMNVKSIRHPIIEQICSNKYVPHNLNL DDNQIGILLYGLNSAGKSSLMKSIGLNVILAQIGYFVSASEFVFS PYKSLFTRISNIDNIYKGLSSFALELVELNAILKRSGSNTLVLSDE VCKGTEYNSALIIVSAMIKMLVESNTSFISATHLHELTKLDAIKC ISKIGIYHISVKYINNNIIFNRLLKKGNGTEEYGLDFAKYMIKNN KFIDICNNMKKDINNITANKSKYNKKLIVDKCQICKSIKNLETHH IEFQKNTDEYGFIIKKTLNHIHKDHISNLVILCSECHDDIHNNKII MKGYEDTIKGSILKYKRSKCIEEKKYNDDEIKFIINKPNISQKKV KDLFEKQFKKNITIKNISKIINNKY* |
| 16 | EpiCas016 | MYNIIYLKINIVMTLIEEYFEFQDKYTKIYGEDTIVLMEVGSFFEI YQVGDLGKAKEIASVLNMILTKKNKNINEVTFKNPLMTGFPTV SLEKNVSRINAQSKYTVVLVKQRGQSPNITREVDEILSPGIQYRI NTFKNNFLANIYVEYYRKNMFGIGISVVDITTGENYLMDSVSLE GFPDNPLSEIQQMVTKYGPSEVIVSMVDFESAESEATRIRVLLDE MIGKYSWKSCDSANYKVSFLNEMFSEIYKVKSILTPIEYLGLEH NQLAGNALIDSIGFIQKNNPCILDKMPRPEYINCAKYLTLNNDA LSQLNVVDNNKKANRKGSLFELLNTTVTAAGGRLYGRRLSNPI SSKEDLNAIYAEIDLFISDSKFAEVRREIAPILDINRIWRRLVMRK GDPMDIWNLYTSIKILADMKNDFSWIPNLENLSNSAQSANKYIE SIFDISRIEDSYLSNLEDNIFKKENPILDKLEAELAEIKYKSQIIAE MFANHSFFTEKTKLVYTDKEGFYIETTPSRYKQYLGLNTAPKSD ILAGFDVYKNKSKVRLTNDDLRKLSESKVIKIQKMSILIKEMFV KEVDEMYSAIGDDVESISAFISHIDVMAANAYNSVNLSLFKPELI |
| | | DNEEGYSSVECHALRHPIVERLTHSEYIQNDVLFSNNKYGKVIF GVNSTGKSTLLKATGLAIIMAQAGMHVPSKMKLSPYQSLYTRI TSGDDLFSNKSTFTVEMLELKQILEKADHNSMVLADELSHGTE TNSGVSILAATINTLSKRKATFLFTTHLHQITGVRSIMDNKSIDLS HLSVNINPITGVIEYDRKLKDGAGESVYGILVAKGLGLADDFIN DATSILNEVKSNRTQLDAITENNTNKYNSNKVNKNCEICGFQSS ETHHIKEQADFKNQKYLEIQKNAKYNLVNVCNDCHKKIHSGKI VNLMYKQTTEGVELKIT* |
| 17 | EpiCas017 | MPSLIKEYFKIQEESEKKFGDKTVIVMHVGGFYEVYSYEGLGK ADVVANECNMMLTSKDKSKEISITNPQMCGFPLASLLRYMYFL TSKGFTCVTVKQDEKVKEKRTIDRIFSPGTCVDDSESSIVASIYA DDGCIAVSWIDLTSGKVMFEEIYNDNEAYRREELTRIIETCGCK EIICMSKNGIMYDDAHEIDFCNIYEHISHQNDVISRAYPNDSTVS PIEFIDMEMFHIARVSLILLIEWVEDHHQSLIQRIRIPERINRKSLA LHNTSVHQLQLVSQDKKSLFDVINKTNTSMGKMLLKQTLLNPY SDVSKLNGMYEDIERISKVDVEVVKKVLKNIGNVDRLARRIAN KSINLEDIRHLILCFENGLDIIEHMRELSVHNMISENDVKRCYIA WHSTIDMEKDVFREGVYDDVDILKKERDTIMYMMDANAKRFS KTLGVNEGVKFELHKGTYGLITTPGRAKTLKNRVGNNVVFQA DCKQKVVISTPDIQSLIHKLHGAIDAYDECMEEKMKEFVEILYS YHEIICTFGTYIAQVDVLLSHYLVAKAFNYTKPVVKEGDKSYIT GKNIRHAIVERLNGNTLYMGNDVDLSNEKGLLLYGVNGSGKS CFSKSVGICVIMAQIGMFVPAETFEFCPYERIFTRISSEDNIYKGQ SSFYVEMMEMNTILRFANEKSLIIGDEICKGTENISAISIVASMCN MISKIGATFVFATHLHGLVDLEPIKQIIDTIQIKHIGVTIESGVVTY DRHIKNGEGEHLYGLEIATHILGNKEFASLALETRNALLKKTPP |
| | | KKSKYNARVLKKVCHICGRKDEQLDTHHINYQCDAHENIKNT KGNLVVLCKKCHDDEHHGFLSIKGWKDTSKGVILDYEFVSEK* |
| 18 | EpiCas018 | MPQLLVDEYIELTNEYRKKYGEKTIIWMQTGSFYEVYAWNEED EQIKVSQNILQIRITTKKSGNNRSCLMAGFPDHSYKRFEKRLLKE NYTIVYVDQIKKDPIERGVTKISSPGCVFDSDDDSKESILMSVLIE IEDNDFYGYYTIYDSNIGEIRMLSIKNEENQTIDHIFDNIGDIISEN RVNEVLLNIVSEQYIKLPETNSKILFHYNKIDKIDELLKPLLQNDI LSKYFKKYLSIYENVIDSLELSKCLPQEIVNLIYMLDFLKEHEPIF VKNLQRPIVDGPNQIKYMEFYNHALQKLQVISNEGNDLIKFIDK TLTYGGSKKLKYLLKNPSNNIEELKKRYKSVDLFVNNRQLLNE TKTYLKICDLHRFYRRFAIGKINVYNDIPRIVNINENINILINIFKN VDNSIYWLPNDKIIQNFKDYCGDLIYTFCIDNCKSSGNVFNKDIV PELDDLYLKSESLNQELESIRTYLCNLINEPIYLKNTDKDGFFFET TKKRGTKIAKQLKENNKFDLEISNSTSVSKISSAGVKKISVQLCD LQTKIIDLTQKKVSEKVLFYYDTYFESSIATIIESMAWLDVYYSY SKSAIEWNYVKPNLVECENSYIDAKQLRHPIIEQLLSIEKQAFIPN DIRIGFKESYLLYGVNSVGKSSLLKSVGLSVIMAQAGMFVPASD YKLCPYDKLIVRIGNTDNLFEAHSSFICEIREANQCVSNTNNRSL VLADEFCASTERESATQIVTSMLLWLNEKKSSFIFATHLFELIEA PIVNVKICHLKVRIESDSLVFDRKLTSGPPTERNYGILVAKKIFK NNKFLKMMDRCQTKTSTRTDINRSRYNRGVLLSECAICAYSPID EMSLPLDTHHINMQCMANNDGFIGNYHKDIVSNLVVLCKKCHI MAHNGKLNIKCWEQRDCGNVLNYEIN* |
| 19 | EpiCas019 | MLHPRLQEMLAARGIRRLYTHQAQAANLALGGAHVVVVTPTA SGKTLCYQLPVLDRLLRRPQSRALYLFPTKALAQDQHHGLTQD LLEAGTDAAAMIYDGDTPQAQRRAARRIAQVLITNPDMLHVGI |
| | | LPYHTQWRELLAELDYVVIDEMHAYRGVFGSHVAHVLRRLQR LCAFYGSAPQFILTSATIANPAELATNLIGEPVEVVAESGAPSGE RHVALVNPPVIEARLGIRRSPLVDARQLALSLAAQGEQVVCFTR SRLAVEQLVIALRERAGRVGLAPEAIRGYRAGYLAAERREIEAG LREGRVRCVVATSALELGIDIGGLTVAVLVGYPGSVASTWQRI GRVGRGQAPSAAFLIASASPLDQYLMAHPEHLFGGGAEAAHIN PDNLYVRSGHEQAALYELPMAEGEAAQDDDRRAILAALEDAG EARLSRGRWFWSGQGFPAGEISLRSAEAARVSIVARRAEEGRDI TIGEVERSSAPRWLHEEAIYLHEGQPYLVRTLDLEGGVAQVEPA TTDYFTVASEREEIEIERVVASTDGERMTTAHGEIRLTRQVTGY RRQRFETQEVLGWGVVDLPEQVILTSAFWLGVKPELVARLDAQ GLWTADRGGARGPDWEQQRDRARLRDGYRCAHCGAPERPER AHDVHHIVPFREFGWEPGRNDAYRYANRLANLVTLCPRCHQL AERQVALGSTLSDMGHVLRYLAPLWLLCDPEDIGIHADSAGRR AERPTLFIYDQLPGGAGLSDQLPRLIVPLLKSAAQVVSDCPCET GCPACIGPVLAPAIGRKRRAVGLLEALAW* |
| 20 | EpiCas020 | MSWNEEITPLAALLQRWRQDPAFRRRVAAWARAPAREAQWA PIPEDLHPRWREALRQAGFSMLYTHQVEAYRAARSGAHVALFT GTASGKTLAFLLPILQALCEDPQTTALFIYPTKALAHDQLAVLR AWLSALRLPAAALPYDGDTPGEARRFVRERGRLLLTNPDMLHL GILPQHPRWAAFLSHLRFVVIDEAHVYRGVFGSHMVNVLRRLR RLAAAYGASPQFLLASATVANGPELAEGLVEGSVRIIEEDGAPY GERHFILYRPPLLDAATGLRQSALTAAAGLIWTLLEHGIPTIAFA RSRMAVERLLRMVRSAWAERGGDPEEVHGYRGGYLPEERRAI EKGLREGRIRAVIATSALELGMDIGTLEACVMVGYPGTIASVRQ QAGRAGRRAPLSVAIMVLTATPLDEYLAGHPEFLFRNPPEAARF |
| | | DPDNLAILADHLRCAAFERPLDPGEPFARTLDTGWIVERLIADG EPLYRNPTDGRVYFVADRYPAQGVSLRTSTGDRVKIRLGAGKG GRIIGEVDRPSAPRLVHPGAVYLHEGAAYRIVSVDWEQGEAMA EPFDGDEITEPIVEARWYLDAERARRTEPWGIGAWGEVTIVHQ VVGYRRLRWPGGELLGWAEVETPEEILSTTAWWLTIELGLVER LKEEGIQIGPLDYGPEWEAIRRQVRARDGYRCRVCGAPEPPGQR HDVHHLRPLRTFRDPAEAYALENLITVCRRCHRRLEQMLGTRS ALSGLAHLLHGLAPIFLMSDPADLGLLVDPESREGGGPTVLLYE GVPGGVGFAAHLWAVQEALLEAALEVVRRCPCERGCPACVGP PGEMPDLKGETRRLLECIFAADEAAASKVQ* |
| 21 | EpiCas021 | MADLERLLERLRRDPTFGPNVTAWRRREGRPARYGETPAWLD PRLQEALRAGGIERLYTHQVAALQAVRQGEHTVVASGTASGKS LCHHLPVLQALLEDPSDCALYLFPTKALAHDQEAVLRALVERIP DLPDSAKPFPASYDGDTPGQARRAIRERVRIVLSNPDMLHAGIL PSHPHWAPFLHHLRYVILDELHTYRGLFGSHIANLLRRLRRVCA FYGSRPQFLCASATIANPGDLAGRLIGEPATVIEEDGAPRGPMH LLLYNPPLLDPALGIRRSALQEARRLAVRLIGAGLQTVVFGRSR RGVELLLTYLRQDLPPGLRPALRGYRGGYLPALRREIEAGLRSG EVRGVVATNALELGVDIGTLAACIMAGYPGSIASTWQQAGRAG RRDQPGLAVLVAGANPLDQYLVTHPDYFFGRSPEQARINPDNP FILAAHLRCSAFELALEDGAPWGTPAPPDLLEDLVREGSLLHEG GRYYWIGVGSPAREVSLRTASGDTVTIIEESSDGIGTIGQVDRPG AAMLVHEQAVYIHEGTFYRVERLDWEAGLAYVRPAQVDYYT QAHLRVDWRVLSEEAQAPQGRGSRHTGEVEVHAQATAYRRV HLHTQESLGWGAISLPPQEFRTQAFWLTLPWPAQEHLRPDYGP NWEEQRRRARERDRFTCRHCGAREEVAGREHDVHHLRPFRTF |
| | | GYIPGENDAYLEANRLENLVTLCRVCHARVDHGPRQETVAAL QGLGHLLAHVAPLYLLCDRGDIRETTRLRALPEGRPVVLIYDDC PGGAGLSPTLFSCADALLQTARERLQECPCAGGCPSCIGPPEEPA RDKVIHILEFLLEEEAPPPGTA* |
| 22 | EpiCas022 | MNKLWQHPNPDWVPVSHFGELRYESIDVVMARWDWDKLIWG KVRRFVFNLQKRIYKATKSGRYRKAKSLMYLLQNSYYATLLA VRTVTTENFRGKCFCARAKTFTSEGKRTAGVDRVKANTPKRK MALVKDMLDTIQRGWDKYRPMPAKRIYIPKANGKPKVSEAKL RPLGIPTIKDRAMQAVTKIALEPFYEAKFESCSYGFRPAMGCHD AIDRITAALLHKQKWVLDADIKGCFDNIDHKFLASQIDTHWRPI VKRWLKAGYMEKSDYNQTNMGTPQGGTISPLLANIALDQMET DLIEHLRGIKGWKKKIGVTTVSIVEKTEKPQKEKKTQKKPKKA NNKGTKEPQKKQKNVYEGAKKSYKCRRNLLIDLVRYADDFVV LHKDKEVIEESKRFIGKWLEVRGLTLSEEKTKIVHSTEGFDFLG HHIRHYENRIKGTYKLKLLKGTPTEQRRAKATHVLRVEPTKDKI KGHYRVIGDTIWKLKAATPSELIRILQPKISGWANYYKTVHSSE AFGKLDHLLWKRLYQWAQRRHPNKGKRWVTNKYFATVNGR RWTFAEIVGKGKDKKIVKHLKGYSKHKERTGSYARVGYDRSY YDGDTAYWAERLSKGYGNITPSKAKMLKRQDGICPVCKCRFG NEDLPEAHHIHPRHLGGEDKYTNLVLVHLHCHDKIHRENPGIK GKLVSQTSPELQGFLNNGMAPAFQAEGMKPPANTSTVLRTGKG AGIITPASDEMKSPVGTSTVLRTGKGAGIITPAPDDNKET* |
| 23 | EpiCas023 | MDTLQLSNPDGRVRSQHSLTGLPSDKKHRDHTDSSGRLVPSCY RVINSSDMCDWDWDSIIWGKIRRLVFNLQKRIYKLTKAGKYRK ARSLMYLLQHSHYAMLLGVRKVTTENKGKKTPGVDKVKVTTP RQKMNLVVDVLDTIQMGWDKYEAKPAKRIYIPKANGKERPLGI |
| | | PTVLDRAMQAVTKSALEPFYEAKFESASYGFRPGMGCHDAIDK IASVLLTKQKWILDADIKGFFDNIDHKFIVEQTDKHWRPIVKKW LKAGYMEENKYNPTKNGTPQGGIISPLLANIALDQMETCLIEHL RGIEGWKTRIGNTKISIITDKKTQKKYESRKHLQLSVIRYADDFV VIHEEKEVIEESERFIKEWLGLRGLTLSEEKTKIGNETDGFDFLG HHIRHYENRIKGTYKAKLLNGTPTEQRKAVTPHVLRVEPTKAK VNAHYQELSDIIGKMKATTPSELIGILQPKISGFANYYRTVHSSK AFSRLDHLLWKRLYQWALRRHSNKGKRWVAAKYFCTVKGRK WTFAEIKGKGKDREIVKSLKGYSKHKVRTGSHARVGYDRSYY DGDTAYWAGRLSKGYGNISPSKAKLLKRQDGLCPVCKCALTN EDLPEAHHIKPRQYGGLWEFKNMVLLHLHCHDKIHREYGEQK RKLCKGTREYDELVKKGVFVSPEVEAKYGPDIAKMRKGPEVN KGSSKILRLGSDPGIISRDKDTVPQETHSNGNNEDDELIQVKSW KSGEIKMIPRSKMYRKTKRVRDKITNKWKLVKMWFCK* |
| 24 | EpiCas024 | MNILWQHPNPSEVNVFARAQKQFTSEWIPVSHLDRHRYESIESV MAKWDWDKLVWGKVRRLVFNLQKRIYKATKAGRYSKARNL MYLLQNSYYATLLAVRTVTTENKGKRTAGVDRVKVNTPKRK MALVKDMLDTIQRGWDKYRPMPAKRIYIPKANGKLRPLGIPTI KDRAMQAVTKIALEPYYEAKFESCSYGFRPAMGCHDAIDKIAG TLLNKQKWVLDADIKGCFDNIDHKFLASQLLPLQHWRPIVKRW LKAGYMAKSEATKKSEYYRTDMGTPQGGTISPLLANIALDQME TDLIEHLRSLKGWKTKIGTTTIGIDINKKTKKSYKSRKNLKIDLV RYADDFVVLHEEKEVIEESKRFIGKWLEIRGLTLSEEKTKIVHST EGFDFLGHHIRHYENRIKGTYKLKLLNGTPTEQRRAKATHVLR VEPTKEKIKGHYQDISDTIWKAKAATPVQLIALLQPKISGWANY YKAVHSSEAFNKLDMLLWRRLYQWACRRHPHKGKRWVANK |
| | | YFATVSGRKWTFAEIVGKGKDRKIVKYLKGYSKHKERTGSHA RVGYDRSYYDGDTVYWAERLSKGYGNITPSKAKMLKRQDGIC PVCKCRFGNEDLPEAHHIHPRHLGGEDKYTNLVLVHLHCHDKI HRENPGIKGKLVGQTSSELQGFLNNGMAPAFQAEGMKPIGTST VLRIGKDAGIITPVSDGNKET* |
| 25 | EpiCas025 | MNILWQHPNPEWIPVSHLGRYRYESIESVMAKWDWDKLIWGK VRRLVFNLQKRIYKATKSGRYSKARNLMYLLQNSYYATLLAV RTVTTENKGKRTAGVDRVKANTPKRKMALVKDVLDTIQRGW DKYRPMPAKRIYIPKANGKLRPLGIPTIKDRAMQAVTKIALEPF YEAKFESCSYGFRPAMGCHDAIEKIAAALLKKQKWVLDADIKG CFDNIDHKFLAVQIDTHWRPIVKRWLKAGYMEKSDYNQTNMG TPQGGTISPLLANIALDQMETNLIEHLRGIKGWKKKIGTTTVSIV VGGKKAKRPQKENEKSQKEPKKANNKGTKESQDKPKKVYEG AEKSYKARKHLHIDLVRYADDFVVLHEDKEVIEESKRFIGKWL EVRGLTLSEEKTKIVHSTDGFDFLGHHIRHYENRVKGTYKLKLL NGTPTEQRRAKATHVLRVEPTKEKVKLHYREISDTIWKLKAAT PVQLIAILQPKISGWANYYKTVHSSRAFGKLDMLLWRRLYQW ACRRHPNKGKRWVANKYFATVNGRKWTFAQIVGKGKDRKIV KYLKGYSKHKERTGSYAKVGYDRSYYDGDTAYWAERLSKGY GNITPSKAKMLKRQDGICPVCKCRFGNEDLPEAHHIHPRHLGGE DTYLNLVLVHLHCHDKIHRENKGIKGKKVSQTSPELQGVLNNG MAPAFQANGMKTPTLTDTVFRVGEGAGIITPALDEKKPNADTS TVLRTGKCAGIITPAPDDNKET* |
| 26 | EpiCas026 | MKQNGIDEITIKSSGEINPKSGTNTRILYEHQKNAMECLDIMNK EKAYSTLVVLPTGGGKTYTASMWLLKNAIDRKKKILWIAHRQ MLLEQAAESFQKFAYAEVIPHISSFHFRIVSGASSHDQTIHIRPTD |
| | | QLLIVSKDSIGRNLQRLDAWMAGENEIYMIVDEAHHSTAKTYR KVISYIKEKVPNVKLIGLTATPFRTAEEEQGLLAKIYTDGVQNG QAVHGDIGITYQIGLKELINRRILSKPVFESYYTDEFYGKELGLK AWESIQHLDMIPDDLAKKMAESSARNKLIVETYQANQEKYGQT ILFAVSVEHAIQLTAQFRKAGVAADYVVSSIKDMITGVTLSREE NEKKINAYRVGDLQVLINVNILTEGVDLPKTQTVFLTRPTVSTIL MTQMIGRALRGTAAGGTANAYIVSFIDAWDEHIAWVNPESLFV GNNDFQENAYEQKQRDLRMIAISKIEEFARILDDSTDTSLLEKVP FTQRIPVGMYAFTYLEENGGIEHAYQVMVYNSSQKAYENMMD SLPDLFQSFHAEEEYLSEHQLMEMEKQCRDTFFCGEMVPPYEP KDVIHILKYYAQYESVPKFYTFDEVDRSKLDVGMIAQHIWAED MGERKKAEYIDKIWNDADDNMLRLFFGKKLYFLRQLDIELMKI RYPDIYEETANVVYETRKLEELSLHDIGKVNPKLEMQLRDAAF EKAKTSDGKYQCAVCGMKNRSKTCFHVDHIIPMNQGGKSVPE NLQILCRQCNGIKGDQI* |
| 27 | EpiCas027 | MARDYIEVKSFGKVFPKKGANTRMPYEHQRDAMENLDIINKSN KFSTMIVLPTGGGKTYTASMWLLRNAIDKKKKILWIAHRQILLD QAAESFQKFAYSEVIPHIPFFQYRIVSGASCHDRVINIQALDNLLI ISKDSIGRNLNGLDVWLKDEKEIFIIVDEAHHSTAKTYRKVIEYV KSKVSNVKLIGLTATPFRTASDEQGLLAKIYEDGVENGKAIHGN IGITYQIGLKELINHRILSKPIFESYYTDEMYGESLGLNSWKSIQH LDNLPEDVAEQMADSAARNKLIVNTYKAKQKEYGQTIVFAIN MVHAVHLSALFNKEGIKSEFIVSDVRDSVTGVRISREENERKLE EYRSGNVKVLVNVNILTEGVDLPQTKTVFLARPTVSTVLMTQM IGRALRGEAAGGTTLAYIVSFVDNWNEHIAWVNPDSLFSGNNE FTDNPVEHVKHELRMIAISKIEEFASVLDDSVDTTMLEKVPFIQR |
| | | IPIGMYAFTYLEENGVDFSYQVMVYDSTRKAYEDLMASLPQLF KIFDCKDEYPSEELLEEIAEHCRDTFFLGDMIPPYEEKDCLRILK YYAQYEIPPQFYLFDDIDRNKLDVAVIAKYIWDEDMGQRRKTE YINSIWENSDENLLRLFFGRKLYFWRQLDIELTKLSNPNIYKKEE NVQLGIRKLEDLSLFEIGKVNPEMEKNLRDTAFEKAKDSEGNYI CACCGQKRTDRIFFHVDHIIPMNKGGKTVPENLQILCRQCNGM KGDKFA* |
| 28 | EpiCas028 | MVAINKRALSEEDIKMKYITPAIEKAGWDIKTQVRAEYTFTDGR IIVRGNLVARGKKKRADYILSYKPNMPLAIIEAKDNKKPLGAG MQQALEYGEILDIPFIYSSNGDGFLEHDKFTGKERVLSLDEFPSP NELWERYKKGKGIQPHEEKIIKEDYFFDMSGKTPRYYQRIAINR TIEAIAKGQKRILLVMATGTGKTYTAFQIIYRLWKSRTKKRILFL ADRNILIDQTMANDFKHFGDKMIKVNRKNISKAHEIYLALYQS MTGSEEWQDIYKQFSPDFFDLVIVDECHRGSARDNSAWREVLE YFSSATQIGLTATPKETRDVSTSTYFGEPVYTYSLKQGIEDGFLA PYKVVRVSLDKDLEGYRPAKGKVDKYGNLIEDRIYNVTDFDK NLILEKRTEIVAKKITEFLKKTNRFDKTIVFCVDIEHAERMRQVL INENSDLVKENPKYIMRITGDDEEGKAQLDNFIDPASKYPTIVTT SKLLTTGVDAQTCKLIVLDSNINSMTEFKQIIGRGTRIKEEYGKY FFTIMDFRGVTRLFADPEFDGDPVVVYEPKKGEDIVPPAEGDYG DFGDSVDGTVGEPDSDENETGTRIKYVVDDVEVMVVNERVEY YDKDGKLVTESLKDYTKKNIRNEYADLNEFINRWNSEDKKSAII EELKERGVFLDAIKEEVGKDLDEFDLILHLAFDQKPLTRKERAY NVRKRNYFSKYSDVAREVLEALLDKYMNEGITELESMEILKLD PFRKIGTPKKIIKAFGGKEKYIQTIKELEREIYA* |
| 29 | EpiCas029 | MSNPGNSVLSTRYRDARTLVRRVCRLPGEDRAPFKEKVARLRE HFEQFNVDVSELCQWFMGLRKAHAAAHAANAATSPAGFGPLG DFLLVPALDGVDADESECDRWRLAVFDAVAGFRDLTAPIREAV PPALHAAIDATARNGPHTNPKNVAMKRLFERLRTLDSAHRLVL LKSAAEWVVARYRRGMENWVRQREEWEKEKTAWETARPQLT PEVRERFTQVFRSLVWDRDKRPGVTNKRPRICPYERLKGNLDN CIYAGQKGHGPLCWKYAGFKKTREEQFSRFNDRGFEKDAAEFL ARRKDGVRRHEVLRQMFRGRSADEQRFTDNWSAYLRALSLNE DTVVEHGRLPHCLRIGETFEKSKCEWNPHTELCKQYKRALDNP DNGFDEETLRLEPLYRQWRRDYLAGPRKPSFRYPSSRDLPMPKI FGAGFHEIDFDRSIVRLRLDDMSAGQWIEFGFTPWPRGYKPSRD EIKAAGRVTSVHVHFIGTRARLGFRFSVRPKPSRFTCSQDELDEL RSRCFPRQAQDQQFLDAARQRLLDSFPGGAEEAKRNMRLLAV DLGETGACAAVYQGTTHQTDCPLAIIKINRLYAGRPDALEPDV KGRPSKEKRKFDKGDPRGVRKEHVARHLARIAQKTAEITQHPR RQPAVPQTVTPGAHDLRSLKRHTAWMIRDWTRLNAAQIVKVA EDQRCNLIVFESLRGFLAPGYDKLCPKEEDKKRWLAMFAYGRV RRKVVEKAVERGMRVVTVPYYKSSQVCSTCGAVQENTNLHRE NKRDRKFICEKNTCAVELNSDANAARVLARIFWGEITLPSPDEK TVRRS* |
| 30 | EpiCas030 | MTRIKYRQERTLVRGLQRLPNQDKAEFNKNVLGLRRHFEQFNL NVAELCQWLISFKPENPGSVCETKLFWEFMLEPENFCPDTYDR GPDWLRHEIFVLAAGWRTFEDIKAHNMPESLFESIKIASSKPRSK TAEALFIRLKSLEASHVMVLLKSASEWLSTRYVRQSENWKQNE QEWQKKKAAWEDKHPELTPAIRDKYNAIFVELGISEKRPHVCS WKKLSENKDNCDWAGERKNIGDKWISHSDLCIKYHEFARKLR |
| | | SKQRQHFVDNANQYLELRRRYPQWTRDMAMNGLFKNVPLAR NWFRNAWTNYLNALNILETTILENYSGHLPHCEKLSDECVFKK HTDNCRRYKLLLGEKLSNQERELEETYREWRREFLAPPNKPFL RYPSAQKLPTPKLFGRGYYDLDFTRHVVKLRLDDMPADNFVSF GFKPWPRGYDKKPGEINITSVHVHFIGTRARVGFRFAVPHSDSR FSVSQDKIDELRSGGFPGKSQDQEFLNEARQRLLDGMNENQKS ALRIMAVDLGTHRAAAAFFTGCIFNKAKLLKLKKIDLLTEPKTD TTKPEKLSADEKKIQREKGLTQHHVGKHFETLEARTKEIISKRQ NMKMAPSDDTPDIVGDHDLRHLTSHIRRMIRDWVRLNARQITE LAEEENVDLIVFESMRGFRAPGYDKLDLKKKRRLAFFAYGQIR RKVAEKAVERGMRVITVPYFKSSQICAQCGRSQNDKNKLRDN KWKQSFQCEFSDCNYKTHSDENAARVLGRVFWGEITLPTD* |
| 31 | EpiCas032 | MQQGCDSRFDTTIKEQEVSLFNTYTKYVDCVNKLPNAQLVDA DFSLKLLSMVIANALEVLNGIKKTVDNDKKYLLLSGGFKLPEV VDVLARLTSNVRQDGAIEIANFGLNSRAVRKRDILNSKKNELDE IESELGALTNKIKKSAQKPKKEDINSKGRLTQERNSLVAEIKKLQ AMNKYGLIFRRKNCDGDFEYFLALVDRDHMENLSDKSKFKSA QSTGYQYMEYNCLKLKSLLQIIFESDSDNLSGVRKLERYGTVKP LFLSKEHSRELEKSKTLIAWLDENIQKIVDTIAGTTNPKITYTEY GNTDAAKNAADVWRDFERHFNRQSYSISWCHLNFEKLKTTKS VELFQIANKDHSIHPDFTSDERDRKKLNRSSGRPNLFTTYLDNV LTDEIGNHYQLLGEGVEYFKHKCGAEPSTPRRKSIDRYYVEFRV AFVPNKSESWEETNSSVKNAISQSDSPKHFIGIDRGENAIANIVVI DENNRAILKQDMSCLAIFSDITPHSAKKSYIFLTGDEPVYIATKS ATDALTDKTEIKKLSDVMFTEYRDKLYKLLPPSEDKKVIPSKSS PCLDEMNIEDLKKLISKLESPEYNPASRKMYLAKTNTPIIDYVYI |
| | | RRYNILLLQAKYMLGKKQVRVTDSNFEQEKIQRKEYLLHATNS IKDNYSAYFVHHVCKLAEKYNAYIVFENLNKKNGDTREINFLS GEQISGLTPKQQNQLKGVSFYVNMEQKLINKLNYLATKDAQSH SLTLKGQFTQSIDYKGVITSMTNEQINLITSPKKQEQVEALKSGL GKGKKNCYQIGCILYVDAANTSKECCNCGFSVNTALTRCERTD EGLSFSGSSNTSYKWGDTRLTERGIDESFDLNNKSDGVKLSVRP GKVVNAKLSTRDVYDPISCLACGFDGRGTGHDHLHENDNDAY QKIKLLQTCDELAAFNIAKRGGKLIKGQEEV* |
| 32 | EpiCas033 | MNYSDFVGLYSISKTLRFELKPTPATKKLLDDSGVKETDKIRHE HFKQAKKILDEVHREFINDALKNVSLSTSDLQDYIKLTLDKASK KADVQKAQKKIFSSFEQAYKITNYEWQEKLDAKKFFLQSPGIQL EIASQLAEKIAKNLEMNEDDVKNTLKQFDKFSTYFKGYAENRK NCYSFDGKATEIANRTIAQNLITFYKNIEKFNKYYSGNSLLADD VSKYFLLENYNQFITQQSIAQNYNVKIGELNYIINQERQKQKKD AKANLPLFKVLYKQILGDATKEVPFEFIKDDAEYKAIIHNLKDK MPKWIEEIEDHFVSQAKQLQNPEGVYLGKKAINTLLQKYISIDV RPLINEELPELAKNKSSKTDDDESLRNQFISVKDFFEALDKVQE QSETSILRSDESAALLPSQFIWNKLCNDIEKDVKVAKDSAPKIDT SNKNSVKSALDSFNDLFRMYSYFELRYKNQPVAVMDEDAKFY QIFNAEDTPFANEKDKRFSQVYDLIRNYLTKKQYSLDKWKLNF DCSILLGGWDLNKEPEKHGVLLRHKQQYYLAIIDNSAKNAFDK KENLDLYTAGADDWQKMEYKLLPGANKMLPKCLFSKSFRENF GVPSDIDVLYKNDEYKKGDKFDINKMHKLIDFYKDAIKKYEG WSMYEFVFSDTKTYQSIDQFYHEVDRFAYRLDFVNINNQVLSK MNENGQIYLFEISNKDWLKEARGKANLHTQYFLNTFRGGDFKL NGEAEIFFRPKSIEAKVDKERKSKHEIIENRRFSEDKILFHVPITFN |
| | | RNSKLSSNFAFNQHAIERIKTNGVNAVIGIDRGEKHLAYVAVVD KNGKLLDKPVSLNQIHSICKGRDFPKDYFDELKKREDERRSNRQ DWDAVRRIKDLKDGYVGHCVKQVVDLMVKHNAVVALENLNF GFKQGRSKIERSTYNQFEQALLKKLQYVVDKNKDDDEIFGVQK GVQLAPPDVAPGKISNHMGFVFYVDPSYTSAIDPKTGYRQHVR LDDRVKTKNFSKFVDDAFDKIYFKDDKLIFEFNWRGLAKAQNG LGVSKNKKTDIKQDKKWGITLDVPRTVFHKNVAGKSETEIVNP QQLLICSLNEAGIKLNEDIKLQLVKNTNLSSKFVKSFVYCFNLA NKLRNQIDGEDAIISPVYPNGFDSRNQKLDGKYKWNGDANGV YNIARKGAILLQKLNKASTPKEFNCKVSSEEYDGFVGGEK* |
| 33 | EpiCas034 | MIFNCILTEHQETQNTKVLVEKFVNDFKDLMGDMQKQEEYLA ENYGVDKSLYKIESIKNEGDEKSKMDTATFYIYPYFDFDKNLSI EECIKRVDSFIGDKKLNRNVEELIGVSDNAGGFSFYFNNGLQLL QNNRIDEIKNAILQASSLWNGKENELQERLEFLNKKAKQVLPV RIVKNWHEYRSDIGGSIQSWVSNSLRQDIEIKKLLYGYKEKKVK RGGEIEEHDIEGHIQEIEKVLGDAELGGYKQYFENKEDEGGIRQ TEEVIAKVKQLKKHLDDEKEKIDTEQPKINPAILNDYRDVLADF RNTFNHLYQKRYGLEEQDEGIEQKDEKGKKIQTVNKKYPKLFK ELPKIPKFLGAVKIEEKGVYDKYICSYNGLKKGVEFLGGVTFTG SKRIDDYYTYRNDSSKDKEEIEKARKRSIERVQRVLENILRLYRS TDAKQLKGQKIKSLLAKYCKDISLLDGEYNYIYRNERYARDKR GQKIELKRVDDELVEDDFIQGLISDLQSFEWRLDNIKQGKWYER VAGWGALVEIEKIKIGLMAWFYEISVPHNFQEIEKNKLHFRKVE AIVKRYNQDEVLDNKEKNTIIQQGIVSEMRGMIARMSKQEIIAR YVVQPVNAEQKYPLVMDEGKYYIHPKEILDKDEIKEQPVNMIDI SKEKDLTEEKNSRIKVRRDSLFAITSSKYQTQFLNNSLNRQLEK |
| | | WQNLNPQLSEYSFIVEDTHKIKWVQDGDSYTPNCDEVLLSRLF VSVPFKIGVENKDKTVAIKQQNKFIGIDIGEYGVAYYGLDGSDF SAKPFVAGFFFQPDMRKIREGIAKNKERQQAGTFTNSNTQIKRV RDNATTKIRNQVHQLVVENNALPIYEKEVSAFESGSGKISAIYN SIKRSDVYAESKADDMEHKLIWGNDKIMVGAEIGAYATSYMCS VCYESIYAYIAKDDYEREYEVIDIVGEQKQIKDKWNATVKFDIE GKEMYAYIEQKDKPVGKGDTFNGKGTINFVRKYERPPLDVVR KIERLPSAIQQELNECTKKERVKSNGEKEKISVWENRRGSQGVF RCAFCNKCSDADVQASMWIALKGYLQNVKNAVQKKENKEQY KMYEGSIGKEVVGEIAKGFSQGGKEQVRFILDFAKDNSIMLR* |
| 34 | EpiCas035 | MATKTYRARLSSIDNRTQQAVVRTHYFFNHCLRQMIERYLDM RKGKYGPFCKQLGDLLLTRSHTDAHGIMDQLTRGAPSTQTDQE WAVLVKHHHREYGPLFLQHEGFAVVDGVVVHTKPQGRHEPTP GRLAVTAKFWHQICDMACAFLKSNQELMAVWRKDKTEWLKT KNAWEVDNPEFMTFWNGPYADFERACEAARVAAQEAAGQTP TARKRDSRVRGKRIDRWHLWYEWLVAHPETVGWRQRAEAGD FKPVPDAVQAAIRKKTKRQDKYVGKYLDWLRENNPELKALDS LRRTYVRRYLRFKRPPTLTLPSANKHPYWFTLELNEFYKGADFE KGCVKMRLIDCDADGLLEMKWFDVRMGCDHRLKPAWRKEKF ASDGRYPPYLGGKIGRKLDRPAECAADRKAGVKGVKLVLDKG RRELFFTLVEQDCPPRLKFKKTKNRRCTADNLFDAGDQRVPIRV MSVDLGIRHIGAYAICEGTKQDDHWEMKYLKKGLLSDSSLPEL GQIRLHDRHLKKLRSRQGDAPTGERNFVALQDHRTGMADDRF KKAAHLLIETARRAGVDIIVFEKLDTLKPDAFDERWINRQLRDM NRRQIVTHVAQQAGEFGLLCKDDISPWLTSQVCSRCYRPGWRF SIKPKAPYKEQCSRAACQDFGYPIWDPGGHLFRCPHCGYKVNA |
| | | DINAAGNLAAKFFGHGFWFDNNLSRDKASGCFIWTDDQRRCF DAREAFEQWAAEASRRQALKEAPF* |
| 35 | EpiCas036 | MATKSYRARILTDSRLAAALDRTHVVFVESLKQMINTYLRMQN GKFGPDHKKLAQIMLSRSNTFAHGVMDQITRDQPTSTLDEEWT DLARRIHKTTGPLFLQAERFATVKNRAIHTKSRGKVIPSPETLAV PAKFWHQVCDSASAYIRSNRELMQQWRKDRAAWLKDKNEWQ QKHPEFMQFYNGPYQNFLKLCDDDRITSQLAAEQQPTASKNNR PRKTGKRFARWHLWYKWLSENPEIIEWRNKASASDFKTVTDD VRKQIITKYPQQNKYITRLLDWLEDNNPELKTLENLRRTYVKKF DSFKRPPTLTLPSPYRHPYWFTMELDQFYKKADFENGTIQLLLI DEDDDGNWFFNWMPASLKPDPRLVPSWRAETFETEGRFPPYLG GKIGKKLSRPAPTDAERKAGIAGAKLMIKNNRSELLFTVFEQDC PPRVKWAKTKNRKCPADNAFSSDGKTRKPLRILSIDLGIRHIGA FALTQGTRNDSAWQTESLKKGIINSPSIPPLRQVRRHDYDLKRK RRRHGKPVKGQRSNANLQAHRTNMAQDRFKKGASAIVSLARE HSADLILFENLHSLKFSAFDERWMNRQLRDMNRRHIVELVSEQ APEFGITVKDDINPWMTSRICSNCNLPGFRFSMKKKNPYREKLP REKCTDFGYPVWEPGGHLFRCPHCDHRVNADINAAANLANKF FGLGYWNNGLKYDAETKTFTVHTDKKTPPLIFKPRPQFDLWAD SVKTRKQLGPDPF* |
| 36 | EpiCas037 | MENKNCNFDKFTKQYQVSKTVRFALTPIGRTEEHIIEKQYIEAA QRKDQAYKIVKPVIDKKLCVMIDDVLTHCEKQDWEKLNELTL QYQNDKTSVEALGEQQGKIRKNISKEFENSIEYKNFFGKDSEKL FKTFLPQCLEEINASEADKEAVKEFQKFKTYFNNFLTVRKDIFN DDSKQNTIPYRIVNENFMIFAGNRKTFSNIVKLIPDASAEIAKEGI ERQEWEFYDVQNIDSCFEPDSFQQCMSQLGIQKYNFIVGLVNSY |
| | | MNLYIQQHPEALEVKRSRLKLRMLHKQILSDRTNPSWLPEQFA EGEEGEKQIYEAIQALEDDLNKNHFQEKFDNLLQLIDTENPRIYI AASEMPRVSSALNYGWNGLNNVRMNVLQELNSEAKIEKILKQ DVSLNELSDALNKYADIYKKEQVPTLQQYIEYGKELLQDCAVA CNDYHASIRNNVERLNQNEKLIEVLKTYLDCYQRVMHFMNVF VVGDELDKDVDFYAELDELIEDISQIIPLYNKVRNYVTRKAYSL DKIRIMFEKSDFLGGWGQDFDTKKALIFEKDGLYYIGIIEKKYT NTDVQYLYEEIEEGNKAVHFVYNFQKIDNKNFPRIFIYSKGTNY APAVRKYNLPIQNIIDIYNERKFQTDYKKVNEQEYYESLEKMID YFKDGIMKNETYRKFHFNWKPSNEYENIKEFYDDTDNSCFLLE KEEINFNHLKEQADQGKIYLFQISSKDFNKGSTGTPNLQTMYW RELFSDQNCKEGIIKLCGGASIYMRDVSIKQPIIHKKDSWLINKW YMEEGQNKVIPDDTYVKFSQIVQGRMSEAELNVEEQQLWNSG LIQKKKATHDITKDRRFTRKQYMLHAPLIINYKQQDIPTPRRFNE EVRSFLKNNPNINIIGIaRGEKNLIYITVIDQKGNILSGMQKSFNQI EEKGANSRIVDYYGKLQSVEDRHTEARKNWKQIGTIRELKEGY LSQVVHEIAQLMIQYNAIIVMaNLNMGFKKGRMKVEKNIYQRF EKMLIDKMNYLAFKKNIEGKAINPYEIGGVMNGYQLTNKFTNF ADMGSQNGFIFYVPAAYTSVIDPVTGFVDVFQRAEFKTGDFLQ KFEDISWDDKEQSFVFTFDYQKFKCNGTCYQNKWSLYADVNRI ETVKKNNQVYRQEQCNPNQRLIELFDSKGIAYRDGHNLVNDLG NYDSKLLSDILYNFRLILQLRNSMRDYSTGEVIDYIASPVMYHR ERFDSQKKNPSLPLDADANGAYHIALKGLMCLQKINEYADPDG KMDIKNMKITNEEWFKFMQTRRESNV* |
| 37 | EpiCas038 | MRRFFMTKMTDFTKLYQLSHTLKFELRPIGKTSENLQNSGLLEE DFKRSQDYVEVKSVLDEVHKKFLQDVLSDCDLSWQELADALK |
| | | IFQVNKDRKVLEDVQKKYRKLLADRFNKNKFFKTLTEATPSKL FKSLLAEPDMAERKVETFKRFACYFKGYQETRQNIYSAEAQQT AAAYRAVNENFVKYLRVVELYENFRKNFPELAEEIGRRTAELT NGELSQILRIENYNRFIAQSGIEYLNSLIAQINSAVNLYRQAHNV KLSFVPLLFKQILSDREQAFTLERIEKDADLDEKLRAFCERHIGN VEIRGETGDLFSRLKVLLTTLNDDCELYIDAASLSAIGNALTGN WSCFRDAMTAYANETLKNKSLREKYCKQPVYSFSEIKSWRVV RTDDDGNVQPVCLSDYWHGEKTLELFAAEKILRNRILEMVRVP HDKNFRERKNDVALVKDYLDAVVEILHLLKPLNVGSENGGDL DLLGILTEFYEALSPVIPLYNQIRNYVTQKPSDIGKIKLMFNNPTL ADGWDLNKEKDNTAVIFCRNGMYYLGIMNPKSKTDFNSLATD SSTDSYQKMVYKQLQNPNQMLPKVFFSAEKIKFFAPSEELLRRY EAGEYKKGEKFDLAYCHELIDFFKRSIDRHEKWSKFEFKFSETS AYSGINEFYREVAEQGYKMSFVNIPVKAVDQLVEDGKLFLFQI WNKDFSEKSTGTPNKFTLYWKALFDRQNLSDVVFKLNGEAELF LREPAITEPVVHRPGGKLVNKTVVAAVDGNGVAIRNSIPDAVY TEIYKHVNGMEEGTLSPAAREHLNRFPRLDWEPGMGLEATVKR TVVKDVKFQLVKDKRFTERKFSFHVPITVNFKSPRLKGAKLNE MVWEFLKDNPDVNVIGIaRGERNLIYVNCIDLKGRILLQKSFNIV GGFDYHQKLDLRERERGQARKSWNEIGTIKELKAGYLSGVVYE IARLMVEYNAIVVMaDLNGGFKRSRTGIEKQIYQKFEKALIDKL NYLVFKNESDWHVPGGLLNGYQLTGPLESFAKLGKQCGWLFY VPAAYTSKIDPSTGFVSLFKLSNYTNTEKRKAFFENFESIVYQAE QDAFVFAFDYKKFKDVENCWKTSWEVYSARRRLVYSRRSRQI EEINPTRLIKRALDKRGVVLSDGFDLKHYITSVEAVNENAAFFK DIFDAFGTTLQMRNSSGEEDYIESPVLNENGQFFDSRKAGKEWP |
| | | RDADSNGAYHIALKGLLRMKSGQTAKLKRLDWFEFVQRRGFIT E* |
| 38 | EpiCas039 | MNDLSEFVGLFPVSKTIKWELKPVGMTAERMREAGFLDRDRSK RDAYTVVKSMADRFYRGYIDERLSTLDLDWNHLGDAMSALKE SGETKDKRELENIKKSFKDSISKHIFTKKDDEDIFSAKVLARLRG TETDDEEVEALEKFDRFAGYFQDFFVNRRVFADTVRGSCAYRI VEDNFPRHFENCRKYQIIRRDNPELILELKRSMEGYLETDPDVLF SIDHFGKLLRQRDIDAYNRVVGSVFRSESDNSRGFNPILNEFHQ RNPDSPKMLMQKLQKQILSEPDSGSFRMRNYENEGQMFEDLQ NLWTGIETNRLLEIPASVLEEGEFDKSGVFISKAGVNTLSNWMF SDWERIGGLLQIFKINEMGDSASKSRKKIDKWLDSDSFSLDVLN RAVADETDIGIVPFLSRRLSDAAQQAREAYLGLEGCASMRREQ YQGRVKTFVDSLLRYRDILKLFSYDPDLPLDRGFYDTFQFVYEE MASAYRAYNRCRSFITRKEYSTEKFKMSFDCPHLADGWDLNK ESSYNSFLFRRDGLYYLGIRNPAWRYEDFSVKADPECDDFYEK AVYKQLQDPVKMLPKVFFSKKGLDKYRPPERILEGYENGLHRQ GDSFDLGFCHELIDFYRDAISQNRDWDFFDFKFKETSEYSSISEF YQDVESQGYQMRFIRIPAQTIDEMVDEGRLLLFQMYNKDYSRD KTPGGKPNLHTMYWEAAMSEDNLRDVRIKINGYAELFWRDAS IKDGTVHRKGSVLVNRIGKDRKPIPSEAYYDLCRYCNGIRDDLG AGKGYEDQIVCKNSRYDIEKDRRYCHDRMFLHIPLTFNFKSDG KTDVNRKVLEWIRRNDDVKFLGIaRGERNLIYLSLVDSDGRILW QRSLNTIVQKDGRGNTRALDYHEKLAVREGERRNAQQSWGPM DAIKNLKEGYVSAAVHEICRIAVEEKAVIVIaNLNYGFKNSRKK VEKQTYQTFERMLINKLQNLAFKDAEPGSPGSVLAPYQLCGPID SMEDIPNHCGMLIHVTAAFTSRIDPVTGFVSVFNLSDITNNAQR |
| | | ADFLSRMTSIRYNQDEDVFEFSFDYGDFRTRLTPKVRKWTVCT YGDRYKYNRKDRTLVRISPTTMIKDALTKAGIRYEDGSDIRKEI SGSDAVRQVFEAFEAILDMRVTTRDEDVIVSPVRDSSGRHFVSG TSGPGLPQDADANGAYCIALKGLLLARKIRASDEDRLPLLNNN DWVEFMQTRVM* |
| 39 | EpiCas040 | MIEQFVNRYPLSKTLRFGLIPQGNTEENFKIMQILEKDEALARN YGKAKEIIDRYHKFFINSCLADFHLTDLEKYALLYYEPNKTEEE VAAMDETAKTMRQAISGLFCKENKAIYDRLSKKDLIENFAMSV IETDEERTVLESFRKCTVYFTGFNQNRLNMYTGEGKSTEIAHRLI NQNLPKFLDNARVAKTILAALPKNDLDQIDVDFYDIFGINTQNIF EIPFYNQTLTQAGIDRYNQIIGGYTCSDGRKIKGYNEYINHFIQE TKQKLPKFKPLYKQILSDRETISFIPKAFQSDSEVLSALRNFYIKK NVETDKSIRDVITELKKLFQEMDSFDEKGIYITNGVAITDLSQAV CDNWFVIKKAMQAEYDAEHSSKNRNEDYEKKRGNDLKKVKS YSLSALQRYMDAEMEDKKSLAEYIKTHTTQLCDLTASAYERVR HLLETEYSPTKKLTADADAIGRIKDFLDPIMEVFHFAKIFLGSGE EAEKDDVFYGIFSPLYDQLADIIEIYDKVRNYVTQKPYKTDKIRL IFERGDFLSGWAQPTESQAKEAHLMEKDGRYYLFITVTAINKA DWERALYGKDNFQCACHIEYYKQKPDNKNTPRLFIRSKGDHY APAVEKYNLPIQEVIDLYDKGYYKAEFKKKDQKLYYTSLRKLI DYFKLGFTKHENYKDFAFAWKESDAYENITEFYQDTMNACYR LERVTVNWNGIMDLVEKGRGFLFQIYNKDFSDHTKGTPNLHT MYFKMLFDERNLENGCYRLLGGAEMFYRKASLKLSETTIHPKG VAIKNKNPRSDRAERVLDYDIIKDKRYTETQFSLHFPIMMNCLA QGNPKLNLDIRKALLKSEHQYIIGIaRGERNLLYVCVINEKGEIV EQMSLNEIIGDNGYTVDYHELLDRKEKERDAARKSWGTIENIK |
| | | ELKEGYMSQVIHKICELVLKYDAVIAMaDLNGGFMNSRIKIEKQ VYQKFEKMLTEKLSYLVDKKADPMSDGGLLRAYQLTNKPDPN KSVQHGMQDGIVFYIPAWCTSKIDPTTGFVDLLKPKYTNKEEA KDFFKRFQKICFNEVENMFEFHFHYPDFQRGITAGNTDWTVYT NGSRIRTFRNPEKNNQWDNTDIVLTDAFKALFAEYAIPCDADM KEAILAQDSPKFFKELCGLLSFTLQMRNSITGRTDVDYIISPVKN AAGRFYDSRKAASDLPCDADANGAYNIARKVLLFIRRLQAQEE SNLMNKDLEKITNAQWLSFVQNGHE* |
| 40 | EpiCas041 | MTKMTDFTKLYQLSHTLKFELRPIGKTSENLQNSGLLEEDFKRS QDYVEVKSVLDEVHKKFLQDVLSDCDLSWQELADALKIFQVN KDRKVLEDVQKKYRKLLADRFNKNKFFKTLTEATPSKLFKSLL AEPDMAERKVETFKRFACYFKGYQETRKNIYSAEAQQTAAAY RAVNENFVKYLRVVELYENFRKNFPELAEEIGRRTAELTDGELS QILRIENYNRFIAQSGIEYLNSLIAQINSAVNLYRQAHNVKLSFV PLLFKQILSDREQVFTLERIEKDADLDEKLRAFCEQHIGNVEIRG ETGDLFSRLKVLLTTLNDDCELCIDAASLSAIGNALSGNWSCFR DAMTAYANETLKNKSLREKYCKQPVYSFSEIKSWRVVRTDDD GNVQPVCLSDYWHGEKALELFATEKILRNKILEMVRVPHDKNF RERKNDVALVKDYLDAVVEILHLLKPLNVGSENGGDLDLLGIL TEFYEALSPVIPLYNQIRNYVTQKPSDIGKIKLMFSNPTLADGW DLNKEKDNTAVIFYRNGMYYLGIMNPKSKTDFDNLATDSSSDC YQKMVYKLLPGPNKMLPKVFFADKNIDFFAPSEELLRRYKAGE HLKGEKFDLAYCHKLIDFFKRSITRHEEWSKFDFQFSETSAYAGI DEFYREVAEQGYKMSFVNIPVKAVDQLVEDGKLFLFQIWNKDF SEKSTGTPNKFTLYWKALFDRQNLSDVVFKLNGEAELFLREPAI TEPVVHRSGEKLVNKTVVAAVDGNGMAIRNSIPDAVYTEIYKH |
| | | VNGMEEGTLSPAAREHLNRFPRLDWEPGMGLEATVKRTVVKD VKFQLVKDKRFTERKFSFHVPITVNFQSPGLKGAKLNEMVWEF LKDNPDVNVIGIaRGERNLIYVNCIDLKGRILLQKSFNIVGGFDY HQKLDMRERERGQARKSWNEIGTIKELKAGYLSGIVYEIARLM VEYNAIVVMaDLNGGFKRSRTGIEKQIYQKFEKALIDKLNYLVF KSESDWHVPGGLLNGYQLTGPLESFAKLGKQCGWLFYVPAAY TSKIDPSTGFVSLFKLSNYTNTEKRKAFFENFESIVYQTEQDAFV FAFDYKKFKDVESCWKTSWEVYSARRRLVYGRRSRQIEEINPT RLIKRALEQRGVVLSDGFDLKHYITSVEAVNENAAFFKDIFDAF GTTLQMRNSSGEEDYIESPVLNENGQFFDSRKAGKEWPRDADS NGAYHIALKGLLRMKSGQTAKLKRLDWFEFVQRRGFITE* |
| 41 | EpiCas042 | MRNRYSKILCLLLLFACCLPQEGNAFWPFKKKKKEDKRLRKLI AQNLKAHPLVDKLKVKNAFGKNGYLETLPLGKEEKEAVKVFA GFGGFFNNYNKNRENYFSTEEKSTAIANRIVNENFSKHFSNVEI VTKIQKEVPELIQIVEAQFKGYDAIFTVNGYNMALSQAGIDTYN EMVAIWNKEANLYAQKAGKLPDGHPLKKKRNYLLSALFKQIG SEKEHLIQIDRFDGDEEVIEALTGVKKMLQEADVFEKLNMLVE DMENWDYSKIYLSAQSLSNVSVFLNNLYEDERENSWNYLDNV LREKWQIELQGKKKGTDLEEAIRKKKQSFYSIEELQEAVNAIEE TDKCYNVSKWLLGAMKSERVIEEKKKDVEDFCTQWKNERNSL KETDITALKEYLEQWIFLARYCKSFYANGIEKKEKDEAFYHILE DVLYVLDEVIYFYNKVRNYVTKKPYSLEKMHLKFGHNELANG WSVNKEGNYGTAILRRNGKYYLAITNSLNKKMSIPTQLESTGN NYEKMVLNVFPNVFRMIPKCTTGRNDVKSCFERKEPNEYFFIDT PEFVNPFKVTREEYELNKITYDGVKKWQSDYSKNTQDEKGYKE AVTKWIQFCMRFLQSYKSTAIYDYSTLQQPEKYETVDSFYHDV |
| | | EKILYECHFEYVPANKIEQLEEEGRIFLFQIYNKDFSENRRPDSK KNLHTLYWEALFSEENRKAKVIQLNGKAEIFRREKSIEHPIVHK AGEVLVNKRTKDGEPIPDDIYKDLSNYFNGRNVTSEKEEYKEC LDKVYTSTKKYDITKDKRFTETKYEFHVPITLNYQADGVKYLN QKILHVLRDNPDVNIIGLaRGERNLISYVVLNREGKIVNNQQGSF NIVGKMDYQKKLYQKEKNRDKERKTWKNIETIKDLKEGYISQV VHELTDMAIRNNAIIVMaDLNFGFKRGRTKVERQVYQKFELAL LKKLHYLVTDKTEGEAMLKPGGVLQGYQLAREVKTLKEIGKQ CGCVFYVPPGYTSKIDPTTGFVDVFNMSGVTNREKKKAFFEKF DNMFYDEKRDMFGFSFNYEKFTTYQSSYRNDWTVYSNGSKYV WNSLKRTDELIDVTKELKLLFEKYAIDYRNEALFEQIMSQDTDK NNADFWNKLFWYFRVLLRLRNSSDELDQIVSPVLNQNGEFFET PKKITEKSYLSDYPMDADTNGAYHIALKGVYLIQEKIADESVDL DNKLPKDFYKISNAEWFMFRQKEK* |
| 42 | EpiCas043 | MVSNKEKTPTYNSLTNLYSVSKTLRFELRPQYSTLDHINEDQIV GKGEELKNHYKTFKKILDQVFSRIINDSLDKTYLDQKYISTYQD LAFKHRDQLTDKDRAELKTLKETLKKQIDKSLDHKDKKAIFSD PVKFLLDNESEFTDLIGDNRPSIEAFNRQKGYLSGYLQNRANIFD HTTNETSVAFRIVEENLAIFLNNRITLQHFLEKVAGKDGLLKSLQ EALFQLGFKLKLEDLLSLDYFNRTLSQSGIDQYNLLISGKTLEDG AKLQGINEILNQYLKQHQEEKLHKIKLKQLYKQILSESKTESFTL DFVEDNKGLAAMFLQFIDFANQLIEEKMPLLGMIQELKNSSVSA ELLSRLHLNRKNIKRLSNFIYKDYGYIEQALEENFLSTVEGKITK KVLGEHRKQDTFTIHEILVALQKQQYEKDGTLESADRLFLPSIV DFVYQNLNCEYSTLLEKVRSEKQPLLELFKERQLLEDQDTESHS SKYPDHPFNDHEIKIIKTTLDFYKNLQSNLALLQISDENLKLDSE |
| | | FYSEFDEFYQGLKNIIPVYNKSRNFLTKKPFSTEKTKLIFNSPQLL DGWSKSKESDCLGTIFIKDGKYYIGIINSATNAKKTLFEPHNFAN FDQSQYFEKMNLFFLSDLKRDFPKKYFSEKWHNQYPIPADLRE KYDYYRIDEHKDERKNDLEYHHQLIAYYQDCLKKDTEWQIYQ FKYKSPEEYSDINEFLSELTPNTYKMEFTKIPAEYIKKLVDDGKL YFFQIYSKDFSEFAKGKPNLHTLYLKAVFDQKNAEEFNYNYKIS GSAEIFYRPASIETRVTHPKNQPIKNKNKNNPKIESIFQYDLCKD RRYMSDKFFLHLPVELNRTPLLPNDSLVNSMVNQVVSSRNQNY FLGIaRGERHLIYLVLIDQSGKIIKQQTLNQITSSYQEKSNNQTVE VVTDYHDLLNDKEKIRKKNLQEWQSVENIKELKAGYLSNVVN EIGKLIAEYQPVIMLaNLNTGFKNSRIKIEKQVYQKFEKALIDKC NYFMRKDIDPSTIGGLYHALQLTKEYSKQYNGKQNGIIYYIPAS YTSNIDPTTGFISAFVQTRYENVEKAKALIEKFNDITYDADESLF CFTVDYKKFSPEAKLWQQNIWQIYTNGDRIYTFRDKEEWQSKN YILVEEFKDLFTKYQINYRNDLKTQILSQTDAGFFKQFLFLLRLT LQMRNSRTADSNEADIDTKKRENDYIISPVKNQYGKFYDSRQD YMDWPENADANGAYNIARKGLIMLKHLKEGLPEKRICDISTEE WVQFVEELNK* |
| 43 | EpiCas044 | MTRVFIFCLLYLKKTKYAPGVHKEGKMKINAFINCYSMSKTLR FKLVPEYETEKNLLEKGFLDRDKLRADDYDLMKKVIDKYHKH FIDKALEGFKFDLLQDYAEAFYSQSADDDGKKLEEIKKKMCKE LATCFSKQDEFKLLDKKELVEKLIPAAEFIEDEEKDIAKRFKGFT TYFTGFNENRQNLYAAELKHGTIAFRLIEENLPAFLYNCKKGVK IFEELDEGDVETLNNELGEILSIGNVKDILNVEYYNKTLTQNGID VYNQIIGGYTQEDGTKIKGVNEYVNLYNQTHDKKLPSLAKLKK QILSDSYSLSFLPAKFNDDSELLLSLKKFYSMVNEETGLSVEKAI |
| | | QEMRNVFLHIDDCDLHNVFIDAKFINKVSNDVFGNWSVLIDGIN AEYEKLNPFNGKNLGNYEEKRKAFLNKIESYSVDALQAYSGKE EKIADYVQKRAVELCDSVACAYENMSNKVINAREGKVKLYQD DEKTEIIKTFLDAVQEFKKFAEMFCYDGTDGDTTFYGEFANYY GQIAEIIPLYNKCRNYLTKKPYSEDKIKINFDNSQLLDGWDANK EKDYLTVLLLKGNNYYLGILDKNHKNVLIKDVPEKGQGEPCFK KMIYKLLPGPEKMLPKVVFSAKNIGFYNPDREILRIRENGTFKT GDGFNIDDCHKLIDFYKECISKNEDWKTFGFRFKETSEYKNIGQ FYNEVKEQGYKISFTDIPESYVKDLVNDGKLYLFRLANKDFSPY SKGKKNLHTMYFEGIFDPENIKEKVYALNGGGELFFRCASLNY DKPTHPKNVPIKNKTYEFRTDNAKKETSTFEYDLIKDKRYTKD QYTLHCPVTLNFKERGIERINDLVRQSLRESDDNYVIGIaRGERN LIYISVIDGKGKIVEQFSMNNLSSGNDVSIDFHKMLETRERERDA SRKNWNTIDNIKDLKQGYLSYVVKKICDLVVKYDAIVAMaDLN VGFKHGREKFERQVYQKFEKALVDKMSYIVNKNASPHSAGGL FRAYQLTNKKYNENEKQNGFIFYVRAWNTSKIDPTTGFVNMLP LKYQSKEKSKEFFDKFEDIFYDENKDMFGFTFRYDDFGINIDHK NEWTAYSNGERIITVRNSFGKWDKAKIALTPAFKKLFDDYNVD CRGDVKRQIMNVDDKDFFVRLYKLLSYTMQLRNSDDADDYIL SPVVNAEGMFFDSRNSDGSLPCDADANGAYHIAKKAMWAIGK IKEADEESFKKTSLAIDNKTWLEFVQKA* |
| 44 | EpiCas045 | MVSNNENTPTYNSLTNLYSVSKTLRFELKPQYSTLDHIRDDQIV DKGEDLKNHYKTFKKILDQVFSRIINDSLDKTYLDQKYISTYQD LAFKHRDQLTEKDRAELKTLKETLKKQIDKSLDHKDKKAIFSDP VKFLLDNESEFADLIGDNRPSIEAFNRQKGYLSGYLQNRANIFD RTTNETSVAFRIVEENLAIFLNNRITLQHFLEKVTDKDGLLKSLK |
| | | EALSQLGFKLKLEDLLSLDYFNRTLSQSGIDQYNLLISGKTLENG TKLQGINEILNQYLQQHQEEKLHKIKLKQLYKQILSESKTESFTL DFVEDNKGLAAMLLQFIDFANQLIEEKMPLLGMIQELKNSSVSV EFLSRLHLNRKNIKRLSNFIYKDYGYIEQSLEENFLSTIEGKITKK VLEEHHKQDTFTFHEILVALQKQQYEKNGTLESADQLLLPGIVN FIYQNLDCECSTLLEKARSEKQPLLDLFKEKQLLEDQNTESHAS KYSDRPFNDHEIKTIKTTLDFYKNLQSNLAIFRISDENLKLDSEF YSEFDEFYQGLKNIIPVYNKSRNFLTKKPFSTEKTKLIFNSPQLL DGWSKSKESDCLGTIFIKDGKYYIGIINSVTNVKKTLFEPHNFAN IDQSQYFEKMNLFFLSDLKRDFPKKYFSEKWHSQYPIPADLRKK YDYYRIDEHKDERKNDLEYHHQLIAYYQDCLKKDTEWQIYQF KYKSPEEYSDVNEFLSELTPSTYKMEFTKIPAEYIKKLVDDGKL YFFQIYSKDFSEFAKGKPNLHTLYLKAVFDQKNAEEFNYNYKIS GSAEIFYRPASIEVRVTHPKNQPIKNKNKNNPKTESVFQYDLYK DRRYMSDKFFLHLPVELNRTPLLPNDSSVNSMVNQVVSSRNQN YFLGIaRGERHLIYLVLIDQNGKIIKQQTLNQITSSYQEKSNNQTV EVVTDYHDLLDDKEKLRKKNLQEWQSVENIKELKAGYLSNVV NEIGKIIAEYQPVIMLaNLNTGFKNSRIKIEKQVYQKFEKALIDKF NYFMRKDIDPSTIGGLYHALQLTKEYSKQYNGKQNGIIYYIPAS YTSNIDPTTGFISAFVQTRYENVEKAKALIEKFNDITYDADESLF CFTADYKKFGPEAKLWQQNVWQIYTNGDRIYTFRDKEEWQSK SYILVEEFKDLFTKYQINYHNDLKTQILSQTDAGFFKQFLFLLRL TLQMRNSRTTDSNEADIDTKKRENDYIISPVKNQYGKFYDSRQ DYMEWPENADANGAYNIARKGLIMLKHLKEGLPEKRICDISTE EWVQFVEELNK* |
| 45 | EpiCas046 | MVSEKENTPTFNSLTNLYSVSKTLRFELRPQYSTLDHIKDDQIV DKGEELKNHYKTFKKILDQVFSRIINDSLDKTYLDQKYISTYQD LVFKHRDQLTDKDRAELKALKETLKKQIDKSLDHKDKKAIFSD PVKFLIDNESDFADLIGDNRPSIEAFNRQKGYLSGYLQNRANIFD HTTNETSVAFRIVEENLAIFLNNRLTLQHFLEKVADKDGLLKSL QETLSQLGFKLKLEDLLSLDYFNRTLSQPGIDQYNLLISGKALED GKKMQGINEILNQYLQQHQEEKLHKIKLKQLYKQILSESKTESF TLDFVEDNKGLAAMLLQFIDFVNKLIEEKMPLLDMIQGLKDSS VSSEFLSRLYFERKNIKRLSNFIYKDYGYIEQSLEENFLSTIEGKIT KKALEEHRKQDTFTIHEILVALQKQQYEKDGTLESADHLLLPG VVDFLYQNLDCKHSTLLEKVGSEKQPLLDLFNEKQLLEGQDAE SHASKYSDRPFNDHEIKVVKTALDFYKNLQSNFAIFQITDENLK LDSEFYSEFDEFYQGLKNIIPVYNKSRNFLTKKPFSTEKTKLIFN NPQLLDGWSKSKESDCLSTIFIKDGKYYVGIINSATNAKKILFEP YNFANLDQNQYFEKMNLFFLSDLKRDFPKKYFSEKWHNQHPV PADLREKYDYYRIDEHKDERKNDLKYHHQLIAYYQDCLKKDT EWQIYQFKYKAPEEYSDVNEFLSELTPNTYKMEFTKIPAEYIKK LVDDGKLYFFQIYSKDFSEFAKGKPNLHTLYLKAVFDQRNAEE FNYNYKISGSAEIFYRPASIETRVTHPKNQPIKNKNKNNPKAESV FQYDLCKDRRYMSDKFFLHLPIELNRIPLLANDSSVNSMVNQV VSSRNQNYFLGIaRGERHLIYLVLIDQNGRIIKQQTLNQITSSYQE KANNQTVEVITDYHDLLNDKEKLRKKNLQEWQSVENIKELKA GYLSNVVNEIGKIIVEYQPVIMLaNLNTGFKNSRIKIEKQVYQKF EKALIDKFNYFMRKDLDSSAIGGLYHALQLTKEYSKQYNGKQN GIIYYIPASYTSNIDPTTGFISAFIQTRYENVEKTKSLIEKFNDITY DAEESLFCFSADYKKFSPEAKLWQQTIWQIYTNGDRIYTFKNKE |
| | | EWQSKSYILVEEFKDLFAKYHIDYCRDLKAQILSQTDASFFKQF LFLLRLTLQMRNSRTTELNGTDADTKKRENDYIISPVKNQYGKF YDSRKDYVDWPENADANGAYNIARKGLIMLKHLKEGLPEKRI CDISTEEWVQFVEELNK* |
| 46 | EpiCas048 | MKSIYENFIGLESKNLTLRFALNPEAKTQENLKLYWDKLRDEER DRAYPIVKKILDKEYQQLISEGLKLLENQNVLDWTELAEYIRTS DLSKKKKEDKRLRKLIAQNLKAHPLVDKLKVKNAFGKNGYLE TLPLGKEEKEAVKVFAGFGGFFNNYNKNRENYFSTEEKSTAIA NRIVNENFSKHFSNVEIVTKIQKEVPELIQIVEAQFKGYDAIFTV NGYNMALSQAGIDTYNEMVAIWNKEANLYAQKAGKLPDGHP LKKKRNYLLSALFKQIGSEKEHLIQIDRFDGDEEVIEALTGVKK MLQEADVFEKLNMLVEDMENWDYSKIYLSAQSLSNVSVFLNN LYEDERENSWNYLDNVLREKWQIELQGKKKGTDLEEAIRKKK QSFYSIEELQEAVNAIEETDKCYNVSKWLLGAMKSERVIEEKKK DVEDFCTQWKNERNSLKETDITALKEYLEQWIFLARYCKSFYA NGIEKKEKDEAFYHILEDVLYVLDEVIYFYNKVRNYVTKKPYS LEKMHLKFGHNELANGWSVNKEGNYGTAILRRNGKYYLAITN SLNKKMSIPTQLESTGNNYEKMVLNVFPNVFRMIPKCTTGRND VKSCFERKEPNEYFFIDTPEFVNPFKVTREEYELNKITYDGVKK WQSDYSKNTQDEKGYKEAVTKWIQFCMRFLQSYKSTAIYDYS TLQQPEKYETVDSFYHDVEKILYECHFEYVPANKIEQLEEEGRIF LFQIYNKDFSENRRPDSKKNLHTLYWEALFSEENRKAKVIQLN GKAEIFRREKSIEHPIVHKAGEVLVNKRTKDGEPIPDDIYKDLSN YFNGRNVTSEKEEYKECLDKVYTSTKKYDITKDKRFTETKYEF HVPITLNYQADGVKYLNQKILHVLRDNPDVNIIGLaRGERNLISY VVLNREGKIVNNQQGSFNIVGKMDYQKKLYQKEKNRDKERKT |
| | | WKNIETIKDLKEGYISQVVHELTDMAIRNNAIIVMaDLNFGFKR GRTKVERQVYQKFELALLKKLHYLVTDKTEGEAMLKPGGVLQ GYQLAREVKTLKEIGKQCGCVFYVPPGYTSKIDPTTGFVDVFN MSGVTNREKKKAFFEKFDNMFYDEKRDMFGFSFNYEKFTTYQ SSYRNDWTVYSNGSKYVWNSLKRTDELIDVTKELKLLFEKYAI DYRNEALFEQIMSQDTDKNNADFWNKLFWYFRVLLRLRNSSD ELDQIVSPVLNQNGEFFETPKKITEKSYLSDYPMDADTNGAYHI ALKGVYLIQEKIADESVDLDNKLPKDFYKISNAEWFMFRQKEK * |
| 47 | EpiCas049 | MEKFPRCSHLKVKQHCGPFVEWYEDLQSDRQKYVSLFFPWIGI GYGERKDEGDDIDFISPEQTLERRKAELGKLEELVVRFVTVMLK GGSSASSCYSLFYEDYKGKDTAKKSTSKVNFIKALPLLSKKEIE DRVLGDNQLSLINLDEWMELGQSGLSADDIWDKVSQRLVTGE RSLGVDVRHTLKIRCQRPRDRGYCVVLMEMVARQLRSCYECY KNHLNELQELREKTTKQITESRDDLFSLVCDLATVLTEQDSGLS ERTLFSGRKQALKPQGKPSKIQAIVDELRQPQYSPLLEADDKEL RQAYKAWNSQRRLRDRETRRPYPCRPNWRNDYQVSLGLTGRG GFWMWMDGSDVVVDLQDCGRLTCSPSHYFGDLAIEGQLHQY DIRYRNKLKRRGKKLRKPPAYGEWIEATIKEIGIQRKGGDYYVY LLPVISHDSTNFQAAKKFFAAAAPKQEIIDALPDEMKVLALDLN ISNPVCSVKARIGRALDGPLSVLDYGQGEIVDGPKILVTDGPRC VALIELHSDVFFVKDAIREYKRTHGVAFNGAGMDDEMLERLSS IPSPSPSVRCQIQAWVAAIGKRLHLLHYGMRREGYKNLGEAIRL LAAMDAYDSMLQSYQRIHLKKGQSLPRQAKNDHSRAGLRETIT RRIAYAIAQEARDCNVVFVEDLKADFDADNDNNALLRLFAVKT LIRYIQEALEKIGVGLVLVAKDGTSKTNPMNGDLGWRDRNNKE |
| | | HLYFELNGEAVRCHADLVASLNVLLRGLDHSVCPYSFVTKDTN KKPGRADQKDKGYGKRLKRFFKDRFGSSNLRFVAKPDGEVTA TTRRPKDALIGERVYYHGGKLVTSEVHYRMENEIKHLLTLKSP AKELSLTEDGIRSYENFTHV |
| 48 | EpiCas050 | MVTPESQSDAKKAFADALFAGQEAAYGSFYAKYTPLPFPRKDS ALGRLTAELGTIGDEKYAKALKLNGLDPDEWAGYRNRAIAGEP PDALWEEIKVKVGVTNKATLASRGVGNLPTLRPDSKSYATALA YMLSSSLSSWEECAKLHRARREEIAAKAAVPDSMTEEYALVCQ LAQALEEAGSGLTAKMLNGAIREMAKTDSVYMVAITELADPK YKPLLALDKEDRARLMDTWQAERKLSTSLAAPKRPLFDKDYQI PFGMTGRGESFALRTDDSGRLWVKIGDHEEFSCAASDYFGNLK VEALPSDKGYKITYRKMVKNRRRQRAGYKSSVGGPLIEGEVKQ ILLQRRMNRRGKESFYVYLPHSIAVKDDRRHLSWFFKRADWK DGKGLEELPDQLIVSGIDLNIDQPVTVTKAIVSKNYTEGEINCPY GGGHLTGKPRSVCPDTPISQEAKLTHRKMRLLIEVIRESKACHK AGTPLSSEGSERLKILYADISKPPCRNNRESIQVVYSSLKKTYRR LKYELMQQGANDVSGCIRMLQLDDTHRALTACFDAFSGKPAL AGRNFGVARKKTSKGTARLNFREHVSHLYANAVLRACHGSDV VFIEDLSTSFDGNAHNSLKRLFAPGQLIDAITSLLESHGIAVVTV DPSGTSRRHPITGDIWYRDSMKKRELVGLIDGELVSVNDDLAAS INVLTKGLNHSVVPHKFKVDPSGRVNVKEDGGKRLKVFLREKG LSGRLPVGSADAAKSKKAKALTVYPSSSGLLSDRQVFEVLEQIK HLLAAGCKPRPVELMAGPGETYAAFSLKEVLEWSKSKSSEDLR HTGLGDETG |
| 49 | EpiCas051 | MSNIKTTNILIPKQQDNYFEIENMLDRQNKIANDVFKLLLTYQG HILDQHFFGEDSKEINFIQKRQELYLVAKQADPLVTEDVFNLTC |
| | | DGFVNQIASKSNMSNDLFKAFEVAYEELSEMKSYVAGKSEAKK LACKLKQWGVLPSFDNHIGEIKSGLSNAFGRFKSWYECDKMTQ ENFQKHVDSKSAYLEEMGDVISDRFQEFANELGNVYNSKGLDN FDYFGGKFLLFYEQILKPSFEKGEVCYSGSIEFSNGKKQQYSIYG GIIDLLFKYKELWETKSWSKKVKRKIETVEQCTFSRFYNIFEDFK YLRKNKQRAGFREFSSTSSASICLSNSNIIKYDITQVDGDRNNFY LTINGKQIQCRYRTKKIKYNKTTVEKTVENKCYLDNLEIEKQIK NDKPTGNYIFKFDVNKKRSNIATVNEPKIFKNRKGIYISLCMNID LDDGKYDENKNIAFYLKTKYFNTYTKEADTLPLLKNKTVRIMG VDTGIKPIYTYSILDYKNGKITDPNIHGKLEGKYEFTNYFNMKSL KNVIRDLPKYFQTDDHKELFKSHLFNDLTFTRDELFDELKNFNT LREYKSRKNKGCLIRREIKSYRHKIGRMKDTNFKSDVKDITNVF GIVKYIDSYKQVCSSWNYIGETVETKTQPNFAFADSHLAKYRE NLKDNMIKTLVFNIIDEAKKHDVDILAIEDFSSEYSTSTKRDNVL KALFAPSQIQSEIERCAKKEGIALMTVTRHYTSQVVFGKDIFGY RPKENDKTKLKWIENGEIKFVDADINAAKNIAAKLALKDVSPY TLDIEGDGKYFKSSKWDKSKVFKKVNDKFVNVSDQKPKNTKT KMKIFLNGDNYITKEKKNELVEEIKNYKIKT |
| 50 | EpiCas052 | MKSIKSIKSIKTKVVKNNELKLIELSTWCSSICEQLERYIFILGGK QIHDRDGVVVLDGAVERKIYCKKDKSLIAACEVVYKHFTDKSS KSRTFGSWFLGGKSEGDNTNKGRKSTKEKTEKQIAKQIADKKE LTDSLQLLWDKKLLPFPIDNKGYDFINTPRAKSYKWAITKTIHA KIKSYNEQCVETKKEYDALNAEINTYKTILFSGYSEKDIDDLQK FVDICEANNHRINYKFISFLKRKDLNFDEQTGKYRKEGKWIQHK NGKEVKSKYSMKDEIVEALYKYKSLTKNDVSVLCNEHQKEDE MGKVVHYNMKRYSDLLFRKKNKKEIPSYTKISLATSKIELGLN |
| | | NVKYNVEQVEDKLIWTICDQTGKDIQFVTVYTRKKEDNRTNG KKGAGFYKGKHHQLEDLKIVPVGDIGTYDISFKVNGKRPFTGT LKEPNIICRGGKVFVQMPININIDKTLNDARKKVLYAYRETYSG SVNGKKQKMIKIENSKIAESLKSLGRDAIVLGVDLGLRGLATAV VSHNGKNETVKSSQYIKGDIVEWEKYRVFNDNIREVKKYIFLTK KSYTATTEEYTEFYKECSKPEQDYLDSLKTYKDKNVKLNELKY TKNAWSVSKMFEDVSKMFETLKQDRLKYYDIFNMPYWAASLK NYMSLMKSYNYVGVDIKVSKEYMSKYQSLYNNIKEDYAKKIG SYIVQLAVAKNCDIIVLEELKSNLGSVDRKSKRDNEMSLMWNC GRIKTHVENMAKDYGMFIDEVPEYGTSQVYHKTGNYGYRDED NREIFWYEDNKDVAYIHADENAAINIAKRFLSQHTDNSSFSVIL KGDAYYLNIASNSKRMRAAALKTFGDLNKPFKINANDKNGNL YKKTRIFKSDSRWIGVNDKDLYIEHIKSLRNLRVRQ |
| 51 | EpiCas053 | MNHFTQNLAVTKIDSSVQTLYDDFQEDFRKWYSILCSWAKVPY RVEDDNGNITHVHAKQRAAELSSLYSAQEQLLIERFMRVFLGA SKEASSSYSLFYDKYAPNGAKKTAALKEFLGEIPTLDDSQVEKK ANTNKNCKRITVKEWRDFAAQFNKLGIDAIWGKLKEKITSPDR GVGLQVRGFVSERCKHPENAGYCRQLCQKLTEQVKSTVRICEL YIQETADLQSEIGNKTKQPEVKKCFDLLCQLSDELRDVNSGLSG RVLFQSIKDIQGGKDSDRKNKVCRTVMSDDKYLCLIDSDKKIV QRAFQAWKLSITLKKRQEKLYPKQRSHQNDVPMPFGLTGLASF DVSPDGSKFTFSEGSVETKPSLYFDGLRFQKNKHSFDMEYRVA YASRRTKKGRRPKRVGRLRKATLKQVELFNKGGKFVIKLPHGI ELPEHRAKLATFLMCADPKEKGYSKGGLVDEFRALAIDVNLSN PLAACVADVSKHGDGPIQALDYGRAKLCVDPFFLVANTPVCGQ LIKLCKECKAVVQAIKEFKECQAAGSDFSFETDLFLPAGHSPRH |
| | | RIQSKVQELSRVLRDWKFKLQNTNQVAEMIRLLELMDAYQSMI SSYQRIHLKAGQTLPPQKKNSTKRANYRKDISRRLAFVVADYA QSLNVDVVFGEDLKADFDRDKNTNSLSRLFAGGMFLQHLENA LAIRGIAFVKCAKNGTSITDPVSGVFGWRDKFNKERLWIERDGE LGWVNSDHAACLNVLLVGLMHSVVPYRVYVKAGRKTTDDVE VFEEDHPIGPRKKRFFELCFGENKPKFEVVENRVILDNAGTYQG FIYAHCLDAMQSEKQHWAEYDRIKELALAETNPKQFGLTPKKG EGYRNFCQPDIHEQVEVVGNKRVAATKQILESLADEKIEAT |
| 52 | EpiCas054 | MSQEGEVKFKSNSDMKDRGVVTGYDYSLINAFCSKYTNLWEE RDDYDSTYLDKGGRKRQACIMTDRYYISHMIKRLKYMTKKHV NVPLFEQGKKAPCQLFGKNYMSYTLRQDEETGTFEFSFTDPTSE SEGRAVKKFNASLAKNGYLNNLKIVESSNKDGVYKVSFNTAVE NKDTLTIQTTYTGYIKEPRIRFNLEKNVTYLDLCISGIESDNDTFN KILSEEQKENKEAIFKGFSSYFKASYAKGRDGSQKGSLDVDVFR AMGVDLGLNPLAALSIYEVKKSDKGEDIGGQKVKIKHLTDHKS SDINKANKDCRVKKAKRTLKFIEGVKGLISVANHHCNYLEDVE ATGEGKEVNVGYMEMLCGDVIENHTPFKDLEDYMKYIENNLY GVDRKEWKKKTSDWVVPSLIQQAKKKISEMRREYFDAEDKHY HTTKVNHVCNLQLHKIQAVKEMISTLNSFSTLGLNDEEKQAHA DNGRPCADLWEYINGLRNYLSKGIASIIINHALRNSVNIIFIEDLD MKASSFDSKEENKLKSLWSASQIMDYLEKFAAKHTIAVQEVNK FMTSQFSSENGTLGVRGASGNKSQFLYEDKGEVCDVDSDINAA RNIAIRGITRHSNMPMFKVKVASDKEFILEMPKAAGMQKLSSM MKYLDSDNPRYVKFVKANNGTLKASVLEKEEFNKLKKQAIAW KKDNKDADSEVSILRSGKHFYIRKELEAIVKAKLCPEVEEETVK AK |
| 53 | EpiCas055 | MATRTSHKSSKIRILPTGTTKKALVEYNLLFHDQVAKIMLDLLT MRGVPYVLRDKDGEHEITATQIREAQWKLINSGRDSGDCIRLSQ SIIDMMAQIYGGIMETGNPTKYRANLYLKKTNSGNEIGQGRSSA SFMLFKDVSRGLSRPEFTRQIKDKNGKIKKPFAWATEERLMELY DAVRNDEMQPIEVEAERKSLEPDVTMENLVKLRKVGILPLPLPG GHLYQGRGLGKLLAMPFDAAVGRLKSHYQANLAAVEARAAL DKVVADKKDNVPAPLYALFLEFAEECRQNGFGVSKRFAKKLH GLNIPKTDFEPGDPFIVGLEIIKQYKYRPLWPQIGTLAGVYLAGV KAEKTKGYAAITIADAYRSPALIRFGITNYARFEAEVNDQQGID FSLQIPVDDQWTTVTVSGMPSNYFQNPELIRQTSNYALSFTKGN RQQRRMGVILKEPLLTVRYSSDPEQEPRFYVHLAYNYDKTSYR MIGLDKIRAFFSSNLSKGAKLPHGTPYGLRTLSIDLGIRPIISSHL MELVSPHDIEAAQESRNGIEPIEVPDMGFARLVDSREMGGVWN SGLVGRMIDASRRCKSARHMILVSKLMRDGKPLNTHQQFLIEK YVKMYGVFLPPDDWARKDIIGEFIRQILADYRVIKDTMAKRNH PDHKGKFPDIICSDVFRMLFLIKDVISMLKSWARYKRKTGDKEI QGQNPTELNQYWRYYNNLKDDLLKKIVHSLVRYADEHGVHLL LLEDITGNRDSTKHRKENSMFAIWSPTLIRTRLEEEFEACGMLV QAINPSFTSQTHYMTGEFGFRDPRQKECLYFKREGRLYLIDSDG NSSMGLQVRFWTRHRDLWTLKTVKARRDRAAKLLKLDPKRA ECTFVAKPTFEYAKSYLKRNLASPVAAFVPDDNGNFILHKLGV NAANQLTEGQERGETFFRHGTDWITHASHKKRMSAIEAMVEK VKKKGDKMTEADLVRLKV |
| 54 | EpiCas056 | MSSDVVRPYNTKLLPDNRKHNMVVDTMNWCDNACSVFFDLF VGLYGGVKIGDVTNFLTEKQDTTTDADLLCAINWFRAMPVSNP DAVVGPEQLVVRFRDYSGHNPSPMASMYLSAPVNPRYTWVDC |
| | | RFLYQQMCQTLGANLDVDISTLVQTGLLPIQKDTQLNAYSFCS NVFGEGVKEDPSAKVTSYTAAANALSGATNPIITYEDYRQILSS AITNRPSRKGAKDILAADLDSTKTGVLPGNYVSQRIAMYQASA ASKARGVVLPNRLRVKAYLQNLVGLNYICTTWAAPMLRAVGD IKSKNSNNYRFVGERLERQQEIDNLLAVSNIRSAQAILQQFRAN ESNQFYIKKRHIGKGLPSLYDMWDLMDEKEGIDNYIQSLKGDK TERLPIRELLQIIYPHRKSVSAELFLSAAELTDVEDRQNRCMVHP TVRGSLVINFGKSSTIFGAITPPDTLIKFRGEMVPAGQTGMIWFT MHLVDGGKWVKHHIPCHSSRYFEEIYAWKKDLPTLPFPRRSLY GYAIGNKISDTRKIDLRRKKASKQYLRTLENMTHNVEFDPTTQF MVDQELNVTITARVKKIPPVRDVPVGSRIMGLNKNQTIRDCYSI WERVEANTSGSFQHPVVQDVWLRLVADGHVTSTVNGPNGQID QMSYSGPDVANCGNWMQERSNFITSVDDADQSIMKHFLWNNR VKLYAWNCVYAGTLMKAISKQMTTANRNLFRNEILSFVGGRF GCRLGSLAQVSLDLLRRMKSVISCYFSRNNAFTIEERSAFDKEL MELFESLDNKMNNKRKEKVNRTLSSIFQVAKQHNVVRLVVTK NLQTPDANVRSVMNQRSVDWCARKIEEKIVDLGNVIGIYVDLV EALDLSHLDPFIYIAKGDVGKECQFDEVESSSLLPIHTSMFRDW HGLVNKGRTTTPIYHSALRSFASHYGLRFDDLARIKAAELAKM LPDQKVFIPHRGGRFFMSTYPVTSTSRPVSYDGRQRFLNSGDMV APVNTMLRGFRRARS |
| 55 | EpiCas057 | MGITMSDTTFRPYKSKLNPNERKMPMVVDTMNWCDNACSVFF DLFVGLYGGVKIGDVTNFLTEKQDTTTDADLLCAINWFRAMPV SNPDAVVGPEQLVVRFRDYSGHNPSPMASMYLSAPVNPRYTW VDCRFLYQQMCQTLGANLDVDISTLVQTGLLPIQKDTQLNAYS FCSNVFGEGVKEDPSAKVTSYTAAANALSGATNPIITYEDYRQI |
| | | LSSAITNRPSRKGAKDILAADLDSTKTGVLPGNYVSQRIAMYQA SAASKARGVVLPNRLRVKAYLQNLVGLNYICTTWAAPMLRAV GDIKSKNSNNYRFVGERLERQQEIDNLLAVSNIRSAQAILQQFR ANESNQFYIKKRHIGKGLPSLYDMWDLMDEKEGIDNYIQSLKG DKTERLPIRELLQIIYPHRKSVSAELFLSAAELTDVEDRQNRCMV HPTVRGSLVINFGKSSTIFGAITPPDTLIKFRGEMVPAGQTGMIW FTMHLVDGGKWVKHHIPCHSSRYFEEIYAWKKDLPTLPFPRRS LYGYAIGNKISDTRKIDLRRKKASKQYLRTLENMTHNVEFDPTT QFMVDQELNVTITARVKKIPPVRDVPVGSRIMGLNKNQTIRDC YSIWERVEANTSGSFQHPVVQDVWLRLVADGHVTSTVNGPNG QIDQMSYSGPDVANCGNWMQERSNFITSVDDADQSIMKHFLW NNRVKLYAWNCVYAGTLMKAISKQMTTANRNLFRNEILSFVG GRFGCRLGSLAQVSLDLLRRMKSVISCYFSRNNAFTIEERSAFD KELMELFESLDNKMNNKRKEKVNRTLSSIFQVAKQHNVVRLV VTKNLQTPDANVRSVMNQRSVDWCARKIEEKIVDLGNVIGIYV DLVEALDLSHLDPFIYIAKGDVGKECQFDEVESSSLLPIHTSMFR DWHGLVNKGRTTTPIYHSALRSFASHYGLRFDDLARIKAAELA KMLPDQKVFIPHRGGRFFMSTYPVTSTSRPVSYDGRQRFLNSGD MVAPVNTMLRGFRRARS |
| 56 | EpiCas058 | MGITMSDTTFRPYKSKLNPNERKMPMARNNKKLEGLVKSYKF QAKNVDIMIDILEEYSEYYNRVSNWINDNLMTLTIGDIALKCNT SQSVGYMQIALSDDYKQLPMYKLFEKKYPSTHCLNLLYAYITN NNIDNYTGNIWGLSTTNYRKYGYINLVINNYRSKFANLNFNVK RSKIDINSNVEEIEKQCLYERIRLGLIKIEDWKKHIDSLKRQEIINE KALHRFNILSDYFNKNGESINERQSLLTIEQLQKFNGCQMKNKS MRIQVQNFKLNEKPNSLGYILQIHLNKKLFNIELLGNRQIRKGN |
| | | KDNYTTLVDIINNHGIDIIFKLENNKLYVILTSTDTTFNKTITTPN KICGIDINIKHALLNTSERDSMNIQGYVNLYKRVLENDNFVSLLS KEEYETFSNMASYLTFCPIEFNYLYSRISNDEYTEKERLFSKILID IQKEYENKKGCDLEKNYIHSVNKLRAKIIAYHKLKTIYTQKKQE YDIKMGFVDDSTLSKDGMDKRRFESPFNNTDCAKQIKSKLDNV LQDIEGCRKNIITYVYKIFETNGYVTIGLENLDNSNFEYHKPLCSI KSLLNYHKLLNKTIEQVKENNSVADLIEKDYYIFTFDENKNIIDA KFSDKGFYKINKSWFINQLIKVLHFASIKNYFITLSNNGKTQIVL VPSEFTSQINSLTHKLPMIKNDKGENVRPTKFRVRKTQEKHYNG LNADINATLNIKYIVENEEWRNVLCKSPNKKEYKDARGYNFPT LIATQKHYNVSKQLEKLGGIEFIQ* |
| 57 | EpiCas059 | MEETRTPEKIACQQDRSAFFKLGKLPQKTTSAIYHLMRCSKRYI DNVSETISNNIEKAAMTSDNHIFRSALKEEYKKIYREKFYPDVT GQPFPDKGPDNTHIVERTVENLCGQYISALKGVMKVRKPKWQ DDIFEDESDKVIAEMLMASGNEADIHPKLEKKIASAKKKDKMD EWQKVLDRYDYIKSKTDIEWTYTTLLYDHIARECARSMFEDGN KPKFCYTNGNKFAKQVVDELWGSERVEYEYEKYLHCRTLEAY WNEENKYFFLENQKKSKKTYEARQIIDHFIENEDAVKAETEEY MAKVAKEFGENGGCKFRTENILYLVNSVSINKKQDEERCPIFKV TDIPQYAEAGIDRIGECKYVIKLKLFTVGERYGTTYMPEAFSGL YDRRTNQRYDLIDRACKGSYVVYLPIYISDKDSRKWSDRLKNTF TMSAFSISERKTKKDGRVLCVNLPYTHIQHKPRNYFKVYPTYG VDINVKHSLFSTNATNLNGYIDVNGELIDFYNSIGRPDKLKNINL AVTLGDKGGTAGNHTNFLLVSDLGDWDDHGKKRNDRFVKRE DGTYYSRPDLLADGLNFLKKKYSGDRKSYDYINTTIAFRNNQT RLRCIDKEWNDRKRAYDEEHGKPEKGERSGFYPTEDAERLTME |
| | | RQALRREIAENTRQVIAYMLWLFQENGIKSIALEHLDCGSWKN FRDQLKPKWFCNNCKYWAEKYGIPYEATKYYRSNRKYFDIED GELIPENVKLNALGEAENDKRYFQTSIAKAVGMSSIKQMFSIVS QNYDIQVNYTEQAYTSQTDSRNGTVDKDFRVSQECYIGSDGTK MNCDFNAAVNIADRISDDTLREEFNKELPMKEFVKYGATQIVS RNRRWNAENAERVTMAAFQKRKNADGGAHTAAVKPTSKTEE SSLKLVAE |
| 58 | EpiCas060 | MIDRNETHLSKKSTRSVEFKLAVPNRIAPVIDEMLDLSITMSNAI SKDISEHLTDWVQRAEVTKEQKRPYDYLYASEKEGIRYWYKHS GLRDKMYFDFCSKYFGYPVLKNKNAHFLTRTIENVAAAYEGRF TRFQEETKIRATDQSQLTEEEELLKKNIKEILNIMCSLKPGQTVD QILVRRSKTGKNQEKYQKLLSFYVDVTSDEKSDKYINFDILFAD MCYRNSATFILKTMEISVDDSGVVNFGMVSPLVELPTKIGSSEE ATMRHILENRSVRLINEVNDMIFELTVAQYIKSSCRYRFVDELT EKANSKDSVFHKNLLMYYTSHRDKVDEGYKQYLHSVNESFGK KQIVWKNSTLKLNYEEKVEKDRQVPIKMISSIKQFADLGITSISG CKYVLRVHMILDDEKKFKDQHLFCDLYDKRTGRRFDLLDHET KGYAYIPIMVPENDRRTYDISDFDCQMTEISSRDGMYFAKLTRD IDYVKPVNRFGEEQVCGVDINIRHAMIDVSERSIMNSEKYVDVE GFIIDYFRNVKNLPSVADDLVTNRYLGEAEEAGKHTFFGLMSSI NKSISRETGGLKNRFVKNVDGTYTILPDCFSEPLNVLMKKYEQG SLQYRYVASVKALRVQLAQMHVLCMEHDDRRQVYDNLYGKP YIDDHPYEDPDFVYAHRKLHQKIETNTQFIVSYAIQLIQEAGFSC ISVEQIDKKNLGKMLNLKSAKVFYNGCIYASSENGQAIADTRYF RTNCRYFDYPSDLNLFDIDHIKLNGVGEAYNINAKLMNSLGKY VGFASFKHMFAHIGTAHDVQVVFVEPAFTSQVDSKTGELRADK |
| | | KEMRPYHHTYKHTDGTVSHADENGAINAKKIVTEEILRTRLLK ENSVKDIMEYGGVQLERSGKSINGVRKVYEEVYAEKNGLV* |

In certain embodiments, the Cas enzyme has a catalytically active domain capable of binding to a target DNA strand and/or a catalytically active domain capable of cleaving the target DNA strand.

In certain embodiments, the catalytically active domain comprising an alteration of one or more amino acids such that the Cas enzyme has only the activity of binding to the target DNA strand, or has the activity of binding to the target DNA strand and the activity of cleaving a single strand of the target DNA.

In another aspect, the present application provides a fusion molecule, which comprises the Cas enzyme described in the present application and one or more heterologous functional domains.

In certain embodiments, the one or more heterologous functional domains are capable of regulating expression of one or more gene products.

In certain embodiments, the one or more heterologous functional domains are directly or indirectly fused to the Cas enzyme.

In certain embodiments, the one or more heterologous functional domains are selected from helicases, nucleases, helicase-nucleases, DNA methyltransferases, DNA hydroxymethylases, histone methylases, histone demethylases, histone acetyltransferases, histone deacetylases, phosphatases, kinases, transcriptional (co-) activators, transcriptional repressors, DNA binding proteins, DNA structural proteins, marker proteins, reporter proteins, fluorescent proteins, ligand binding proteins, signal peptides, subcellular localization sequences, antibody epitopes, and affinity purification tags.

In certain embodiments, the one or more heterologous functional domains have one or more of the following activities: methylase activity, demethylase activity, deaminase activity, transcriptional activation activity, transcriptional repression activity, transcriptional release factor activity, reverse transcriptase activity, histone modification activity, RNA cleavage activity, and nucleic acid binding activity.

In another aspect, the present application provides an engineered, programmable, non-naturally occurring CRISPR-Cas system, the system comprises the Cas enzyme of the present application or the fusion molecule of the present application, and one or more guide RNAs; the one or more guide RNAs target a locus of a nucleic acid molecule encoding one or more gene products in a cell, thereby directing the Cas enzyme or the fusion molecule to bind to and/or cleave the locus of the nucleic acid molecule encoding the one or more gene products; and the Cas enzyme or the fusion molecule does not naturally co-occur with the guide RNA.

In another aspect, the present application provides an engineered, non-naturally occurring vector system, the vector system comprises one or more vectors comprising: a) a first regulatory element operably linked to one or more guide RNAs capable of hybridizing to a target sequence in a locus of a nucleic acid molecule encoding one or more gene products, and b) a second regulatory element operably linked to the Cas enzyme of the present application or the fusion molecule of the present application, wherein the component a) and the component b) are located on the same vector or different vectors of the vector system, and the guide RNA targets the locus of the nucleic acid molecule encoding the one or more gene products in a cell, thereby directing the Cas enzyme or the fusion molecule to bind to and/or cleave the locus of the nucleic acid molecule encoding the one or more gene products; and the Cas enzyme or the fusion molecule does not naturally co-occur with the guide RNA.

In certain embodiments, the expression of the one or more gene products is altered.

In certain embodiments, the expression of the gene product is decreased or increased.

In certain embodiments, the gene product is a protein.

In certain embodiments, the cell is a eukaryotic cell.

In certain embodiments, the eukaryotic cell is a mammalian cell. For example, the mammalian cell includes, but are not limited to, cells of mice, monkeys, humans, farm animals, sports animals, and pets.

In certain embodiments, the mammalian cell is a human cell.

In certain embodiments, the Cas enzyme is codon-optimized for expression in eukaryotic cells.

In certain embodiments, the guide RNA comprises a guide sequence fused to a tracr sequence.

In certain embodiments, the guide RNA comprises a direct repeat sequence and a spacer sequence, and the spacer sequence binds to a nucleic acid molecule targeted by the guide RNA.

In certain embodiments, the direct repeat sequence is 10 to 70 nucleotides in length.

In certain embodiments, the direct repeat sequence is 31 to 36 nucleotides in length.

In certain embodiments, the direct repeat sequence comprises a nucleotide sequence as set forth in any one of SEQ ID NO: 63-88 and 90-99, or comprises a nucleotide sequence having at least 95% sequence identity to the nucleotide sequence as set forth in any one of SEQ ID NOs: 63-88 and 90-99.

In certain embodiments, the spacer sequence is 16 to 24 nucleotides in length.

In certain embodiments, the nucleic acid molecule targeted by the guide RNA comprises a nucleotide sequence capable of complementary pairing with the spacer sequence.

In certain embodiments, the vector or the Cas enzyme of the system further comprises one or more nuclear localization sequences (NLS).

In certain embodiments, the system is introduced into the cell by a delivery system, and the delivery system is selected from a virion, a liposome, a lipid nanoparticle, electroporation, microinjection, and conjugation.

In another aspect, the present application provides a method for altering the expression of one or more gene products, the method comprises introducing an engineered, non-naturally occurring CRISPR-Cas system into a cell comprising and expressing a nucleic acid molecule encoding the one or more gene products, and the system comprises the Cas enzyme of the present application or the fusion molecule of the present application, and one or more guide RNAs, and the one or more guide RNAs target a locus of the nucleic acid molecule encoding the one or more gene products, thereby directing the Cas enzyme or the fusion molecule to bind to and/or cleave the locus, thus altering the expression of the one or more gene products; and the Cas enzyme or the fusion molecule does not naturally co-occur with the guide RNA.

In another aspect, the present application provides a method for altering the expression of one or more gene products, wherein the method comprises introducing an engineered, non-naturally occurring vector system into a cell comprising and expressing a nucleic acid molecule encoding the one or more gene products, the system comprises one or more vectors, and the one or more vectors comprises: a) a first regulatory element operably linked to one or more guide RNAs capable of hybridizing to a target sequence in a locus of the nucleic acid molecule encoding the one or more gene products, and b) a second regulatory element operably linked to the Cas enzyme of the present application or the fusion molecule of the present application, wherein the component a) and the component b) are located on the same vector or different vectors of the vector system, and the guide RNA targets the locus of the nucleic acid molecule encoding the one or more gene products in the cell, thereby directing the Cas enzyme or the fusion molecule to bind to and/or cleave the locus, thus altering the expression of the one or more gene products; and the Cas enzyme or the fusion molecule does not naturally co-occur with the guide RNA.

In certain embodiments, the expression of the gene product is decreased or increased.

In certain embodiments, the gene product is a protein.

In certain embodiments, the cell is a eukaryotic cell.

In certain embodiments, the eukaryotic cell is a mammalian cell. For example, the mammalian cell includes, but are not limited to, cells of mice, monkeys, humans, farm animals, sports animals, and pets.

In certain embodiments, the mammalian cell is a human cell.

In certain embodiments, the Cas enzyme is codon-optimized for expression in eukaryotic cells.

In certain embodiments, the guide RNA comprises a guide sequence fused to a tracr sequence.

In certain embodiments, the guide RNA comprises a direct repeat sequence and a spacer sequence, and the spacer sequence binds to a nucleic acid molecule targeted by the guide RNA.

In certain embodiments, the direct repeat sequence is 10 to 70 nucleotides in length.

In certain embodiments, the direct repeat sequence is 31 to 36 nucleotides in length.

In certain embodiments, the direct repeat sequence comprises a nucleotide sequence as set forth in any one of SEQ ID NO: 63-88 and 90-99, or comprises a nucleotide sequence having at least 95% sequence identity to the nucleotide sequence as set forth in any one of SEQ ID NOs: 63-88 and 90-99.

In certain embodiments, the spacer sequence is 16 to 24 nucleotides in length.

In certain embodiments, the nucleic acid molecule targeted by the guide RNA comprises a nucleotide sequence capable of complementary pairing with the spacer sequence.

In certain embodiments, the vector or the Cas enzyme of the system further comprises one or more nuclear localization sequences (NLS).

In certain embodiments, the method comprises introducing the CRISPR-Cas system or the vector system into the cell by a delivery system, and the delivery system is selected from a virion, a liposome, a lipid nanoparticle, electroporation, microinjection, and conjugation.

In another aspect, the present application provides a nucleic acid encoding the Cas enzyme of the present application, the fusion molecule of the present application, or the CRISPR-Cas system of the present application.

In another aspect, the present application provides a cell comprising the Cas enzyme of the present application, the fusion molecule of the present application, the CRISPR-Cas system of the present application, the vector system of the present application and/or the nucleic acid of the present application.

In another aspect, the present application provides a kit comprising the Cas enzyme of the present application, the fusion molecule of the present application, the CRISPR-Cas system of the present application, the vector system of the present application, the nucleic acid of the present application and/or the cell of the present application.

In certain embodiments, the kit further comprises a container for placing the Cas enzyme, the fusion molecule, the CRISPR-Cas system, the vector system, the nucleic acid and/or the cell, and an instruction for use.

Those skilled in the art may easily discern other aspects and advantages of the present application from the following detailed description. Only exemplary embodiments of the present application are shown and described in the following detailed description. As those skilled in the art will realize, the contents of the present application enable those skilled in the art to make modifications to the specific embodiments disclosed without departing from the spirit and scope of the invention to which the present application relates. Accordingly, the drawings and descriptions in the specification of the present application are illustrative only and not limited.

### BRIEF DESCRIPTION OF DRAWINGS

The specific features of the invention to which the present application relates are as shown in the appended claims. The features and advantages of the invention to which the present application relates may be better understood by reference to the exemplary embodiments and the drawings described in detail below. The accompanying drawings are briefly described as follows:
FIGs. 1A-1B show a schematic process diagram and fluorescence results of the assay of cleavage activity of the Cas enzyme of the present application in eukaryotic cells, and the PAM motif required for gRNA binding of the Cas enzyme.
FIGs. 2A-2B show a schematic process diagram of the assay of cleavage activity of the Cas enzyme of the present application in eukaryotic cells, and the comparison results of green fluorescent cells of different Cas enzymes.
FIGs. 3A-3B show a schematic process diagram of the assay of the in vitro cleavage activity of the Cas enzyme of the present application and the PAM sequence of the Cas enzyme, as well as sequencing results.
FIG. 4 shows site information and amplification detection results of detecting the Cas enzyme activity of the present application at endogenous sites.
FIGs. 5A-5C show the GFP fluorescence results after cleavage activity assay of different variants of the Cas enzyme of the present application in eukaryotic cells.
FIGs. 6A-6B show the results of the proportion of green fluorescent cells after cleavage activity assay of the Cas enzyme of the present application combined with sgRNAs processed by different Direct Repeat (DR) region optimization schemes in eukaryotic cells.
FIG. 7 shows the results of the proportion of green fluorescent cells after cleavage activity assay of the Cas enzyme of the present application combined with different sgRNAs with different spacer lengths in eukaryotic cells.
FIGs. 8A-8B show the results of the proportion of green fluorescent cells after cleavage activity assay of different variants of the Cas enzyme of the present application in eukaryotic cells. FIG. 8C shows the results of cleavage activity assays (proportion of green fluorescent cells) of different variants of the Cas enzyme of the present application in different PAM reporter systems.
FIGs. 9A-9C show schematic structural diagrams of cytosine base editors comprising the Cas enzyme of the present application, and the results of testing the base editing efficiency thereof at two endogenous sites, EMX1 and VEGFA.
FIG. 10 shows the activation effect of the gene activation epigenetic tool comprising the Cas enzyme of the present application on detecting target site gene expression at the endogenous site of CXCR4.

### DETAILED DESCRIPTION

The embodiments of the invention of the present application will be illustrated below with specific examples. Those skilled in the art may easily understand other advantages and effects of the invention of the present application from the disclosure of the specification.

### DEFINITION OF TERMS

In the present application, the term "identity" is used interchangeably with "homology", which generally refers to the relationship between two or more polypeptide molecules or two or more nucleic acid molecule sequences, which relationship is determined by comparing these sequences. In the art, "identity" also refers to the degree of relatedness of nucleic acid molecules or polypeptide sequences, as determined by the match between two or more nucleotides or two or more amino acids. In the present application, percent identity (%) of amino acid sequences is defined as the percentage of amino acid residues in a candidate sequence that are identical to the amino acid residues in a reference polypeptide sequence to the total number of residues, after aligning the sequences and introducing gaps, if necessary, to achieve the maximum percent sequence identity, and not considering any conservative substitutions as part of the sequence identity. Alignment for the purpose of determining percent amino acid sequence identity can be achieved in a variety of ways within the skill in the art, for example, using publicly available computer software such as BLAST, BLAST-2, ALIGN, or Megalign (DNASTAR) software. Those skilled in the art can determine appropriate parameters for aligning sequences, including any algorithm required to achieve maximum alignment over the full length of the sequences being compared. In certain embodiments, the calculation of the percent identity (%) of a polypeptide or nucleic acid sequence can also determine the total number of residues based on the type of sequence mutation. Types of mutations include insertions (extensions) at either or both ends of the sequence, deletions (truncations) at either or both ends of the sequence, substitutions/replacements of one or more amino acids/nucleotides, insertions within the sequence, and deletions within the sequence. Taking the amino acid sequence of a polypeptide as an example (the same applies to the nucleotide sequence), if the type of mutation is one or more of the following: substitution/replacement of one or more amino acids/nucleotides, insertion within the sequence, and deletion within the sequence, the total number of residues is calculated based on the larger one in the compared molecules. If the type of mutation also includes insertions (extensions) at either or both ends of the sequence or deletions (truncations) at either or both ends of the sequence or insertions within the sequence and deletions within the sequence, the number of amino acids inserted or deleted at either or both ends or within the sequence (e.g. less than 20 insertions or deletions at both ends) is not counted in the total number of residues. In calculating percent identity, the sequences being compared can be aligned in a manner that produces the greatest match between the sequences, and gaps in the alignment, if present, can be resolved by a specific algorithm.

In the present application, a "catalytically active domain" refers to an identifiable or determinable conserved structural entity within a Cas protein (enzyme) that exhibits a significant secondary structure content, and the conserved structure is the region where the Cas protein (enzyme) performs functions such as binding and/or cleaving polynucleotides. An exemplary catalytically active domain may be a Cas9 family protease having two catalytically active domains, one being HNH-like, which functions to cleave a single-stranded polynucleotide (target strand) paired with a guide RNA, and the other being RuvC-like, which functions to cleave the complementary strand of the target strand.

In the present application, the term "binding" (e.g., with respect to a target DNA binding (catalytically active) domain of a polypeptide or protease) generally refers to a non-covalent interaction between macromolecules (e.g., between proteins and nucleic acids). A macromolecule is referred to as "associated" or "interacting" or "bound" when in a state of non-covalent interaction (e.g., when molecule X is referred to as interacting with molecule Y, it is meant that molecule X binds molecule Y in a non-covalent manner). It will be appreciated that not all binding interaction components need to be sequence-specific (e.g., contact with phosphate residues in the DNA backbone), but some portions of the binding interaction may be sequence-specific.

In the application, the term "fusion molecule" generally refers to a molecule composed of at least two moieties (bipartite molecule) comprising an enzyme (protein or peptide) of the present application coupled to at least one additional moiety to form a single entity. The enzyme and the at least one additional moiety may be separated by a linker, or may be directly coupled. The at least one additional moiety may be fused to an enzyme of the present application at any amino acid other than the N-terminal amino acid, C-terminal amino acid, or terminal amino acids. The additional moiety may be fused to moieties already included in the fusion molecule. The optimal order and/or combination of assays for determining moieties in the fusion molecules of the present application are well known to those skilled in the art. Generally, when the fusion molecule comprises an enzyme of the present application and at least one additional peptide, the term does not include such fusion molecules in which the fusion produces a naturally occurring peptide.

In the present application, the term "heterologous" generally refers to a nucleotide or polypeptide sequence that is not found in a naturally occurring nucleic acid or protein, respectively. In certain embodiments of the present application, the term "heterologous functional domain" may refer to about or more than about 1, 2, 3, 4, 5, 6, 7, 8, 9, 10 or more domains other than the Cas enzyme of the present application, or a portion of the fusion molecule of the present application. Examples of heterologous functional domains that can be included in the fusion molecules of the present application or can be fused to the Cas enzymes of the present application include, but are not limited to, epitope tags, reporter gene sequences, and protein domains having one or more of the following activities: methylase activity, demethylase activity, transcriptional activation activity, transcriptional repression activity, transcriptional release factor activity, histone modification activity, RNA cleavage activity, and nucleic acid binding activity. Non-limiting examples of epitope tags include histidine (His) tags, V5 tags, FLAG tags, influenza virus hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT), beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). The Cas enzyme can be fused to a gene sequence encoding a protein or protein fragment that binds to a DNA molecule or binds to other cellular molecules including, but not limited to, maltose binding protein (MBP), S-tag, Lex A DNA binding domain (DBD) fusions, GAL4 DNA binding domain fusions, and herpes simplex virus (HSV) BP16 protein fusions. Additional domains that can form part of a fusion molecule comprising a Cas enzyme are described in US 20110059502, which is incorporated herein by reference.

In the present application, the term "expression" generally refers to the process by which a DNA template is transcribed into a polynucleotide (e.g., into mRNA or other RNA transcript) and/or the process by which the transcribed mRNA is subsequently translated into a peptide, polypeptide or protein. The transcript and the encoded polypeptide may be collectively referred to as a "gene product". If the polynucleotide is derived from genomic DNA, expression may include splicing of mRNA in eukaryotic cells.

In the present application, the terms "polynucleotide," "nucleotide," "nucleotide sequence," "nucleic acid," and "oligonucleotide" are used interchangeably. They generally refer to a polymeric form of nucleotides (either deoxyribonucleotides or ribonucleotides, or analogs thereof) of any length. A polynucleotide may have any three-dimensional structure and may perform any function, known or unknown. The following are non-limiting examples of polynucleotides: coding or non-coding regions of a gene or gene fragment, multiple loci (a locus) defined by linkage analysis, exons, introns, messenger RNA (mRNA), transfer RNA, ribosomal RNA, short hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. Polynucleotides may include one or more modified nucleotides, such as methylated nucleotides and nucleotide analogs. If present, modifications to the nucleotide structure may be conducted before or after polymer assembly. The sequence of nucleotides may be interrupted by non-nucleotide components. Polynucleotides may be further modified after polymerization, for example, by conjugation with a labeling moiety.

In the present application, the terms "non-naturally occurring" and "engineered" are used interchangeably. When they refer to nucleic acid molecules, polypeptides, or combinations thereof and systems thereof, it is generally meant that the nucleic acid molecules or polypeptides are at least substantially free from at least another component with which they are associated in nature or as found in nature.

In the present application, the term "vector" generally refers to a nucleic acid molecule that is capable of transporting another nucleic acid molecule to which it is linked. Vectors include, but are not limited to, single-stranded, double-stranded, or partially double-stranded nucleic acid molecules; nucleic acid molecules comprising one or more free ends, or no free ends (e.g., circular); nucleic acid molecules comprising DNA, RNA, or both; and a wide variety of other polynucleotides known in the art. One type of vector is a "plasmid", which refers to a circular double-stranded DNA loop into which additional DNA fragments can be inserted, for example, by standard molecular cloning techniques. Another type of vector is a viral vector in which a virus-derived DNA or RNA sequence is present in a vector used to package viruses (e.g., retroviruses, replication defective retroviruses, adenoviruses, replication defective adenoviruses, and adeno-associated viruses). Viral vectors also include polynucleotides carried by a virus for transfection into a host cell. Certain vectors (e.g., bacterial vectors having a bacterial origin of replication and episomal mammalian vectors) are capable of autonomous replication in a host cell into which they are introduced. Other vectors (e.g., non-episomal mammalian vectors) are integrated into a host cell genome upon introduction into the host cell, and thereby replicate along with the host genome. Furthermore, certain vectors are capable of directing the expression of the genes to which they are operably linked. Such vectors are referred to herein as "expression vectors". Common expression vectors used in recombinant DNA technology are typically in the form of plasmids. The recombinant expression vectors may comprise the nucleic acid of the present application in a form suitable for expression of the nucleic acid in a host cell, meaning that these recombinant expression vectors comprise one or more regulatory elements selected based on the host cell to be used for expression, and the regulatory elements is operably linked to the nucleic acid sequence to be expressed. In a recombinant expression vector, "operably linked" is intended to mean that a nucleotide sequence of interest is linked to the one or more regulatory elements in a manner that allows the expression of the nucleotide sequence (for example, in an in vitro transcription/translation system or in a host cell when the vector is introduced into the host cell).

In the present application, the term "regulatory element" is generally intended to include promoters, enhancers, internal ribosome entry sites (IRES), and other expression control elements (e.g., transcription termination signals such as polyadenylation signals and polyU sequences). Such regulatory sequences are described, for example, in Goeddel, GENE EXPRESSION TECHNOLOGY: METHODS IN ENZYMOLOGY 185, Academic Press, San Diego, CA (1990). In certain embodiments, regulatory elements may include those sequences that direct constitutive expression of a nucleotide sequence in many types of host cells as well as those sequences that direct expression of the nucleotide sequence only in certain host cells (e.g., tissue-specific regulatory sequences). A tissue-specific promoter may primarily direct expression in a desired tissue of interest, such as muscle, neurons, bone, skin, blood, specific organs (e.g. liver, pancreas), or specific cell types (e.g. lymphocytes). The regulatory elements may also direct expression in a timing-dependent manner, such as in a cell cycle-dependent or developmental stage-dependent manner, which may or may not be tissue- or cell type-specific. In certain embodiments, a vector may comprise one or more pol III promoters (e.g., 1, 2, 3, 4, 5, or more pol III promoters), one or more pol II promoters (e.g., 1, 2, 3, 4, 5, or more pol II promoters), one or more pol I promoters (e.g., 1, 2, 3, 4, 5, or more pol I promoters), or a combination thereof. Examples of pol III promoters include, but are not limited to, the U6 and H1 promoters. Examples of pol II promoters include, but are not limited to, the reverse transcription Rous sarcoma virus (RSV) LTR promoter (optionally with an RSV enhancer), the cytomegalovirus (CMV) promoter (optionally with a CMV enhancer), the SV40 promoter, the dihydrofolate reductase promoter, the beta-actin promoter, the phosphoglycerol kinase (PGK) promoter, and the EF1α promoter. The term "regulatory element" may also encompass enhancer elements such as WPRE, CMV enhancers, R-U5' fragments in the LTR of HTLV-I, SV40 enhancers; and the intron sequence between exons 2 and 3 of rabbit β-globin (Proc. Natl. Acad. Sci. USA., Vol. 78 (3), pp. 1527-31, 1981).

Those skilled in the art will appreciate that the design of the expression vector may depend on factors such as the choice of the host cell to be transformed, the desired expression level, and the like. A vector can be introduced into a host cell to thereby produce a transcript, protein, or peptide, including a fusion molecule or enzyme encoded by a nucleic acid of the present application (e.g., a clustered regularly interspaced short palindromic repeat (CRISPR) transcript, protein, enzyme, mutant form thereof, fusion molecule or fusion protein thereof, etc.). Advantageous vectors include lentiviruses as well as adeno-associated viruses, and vectors of this type can also be selected to target specific types of cells.

In the present application, the term "codon optimization" generally refers to a method of modifying a nucleic acid sequence in order to enhance expression in a host cell of interest by replacing at least one codon of the native sequence, for example about or more than about 1, 2, 3, 4, 5, 10, 15, 20, 25, 50, or more codons, with a codon that is more frequently or most frequently used in a gene of the host cell, while maintaining the native amino acid sequence. Different species exhibit specific preferences for certain codons with specific amino acids. Codon preference (differences in codon usage between organisms) is often associated with the translation efficiency of messenger RNA (mRNA), which is believed to depend, among other things, on the nature of the codon being translated and the availability of a particular transport RNA (tRNA) molecule. The dominance of selected tRNAs within a cell generally reflects the codons most frequently used for peptide synthesis. Thus, genes can be tailored to optimal gene expression in a given organism based on codon optimization. Codon utilization tables are readily available, for example, in a Codon Usage Database ("Codon Usage Database"), and these tables can be adapted in different ways. For example, see Nakamura Y. (Nakamura Y.) et al., Codon usage tabulated from the international DNA sequence databases: status for the year 2000, Nucl. Acids Res. 28: 292 (2000). In silico algorithms for codon optimization of specific sequences for expression in specific host cells are also available, such as Gene Forge (Aptagen, Jacobus, PA). In certain embodiments, one or more codons (e.g., 1, 2, 3, 4, 5, 10, 15, 20, 25, 50, or more, or all codons) in the sequence encoding the Cas enzyme correspond to the codon most frequently used for a particular amino acid.

In the present application, the term "guide RNA" is used interchangeably with "gRNA", and generally refers to a group of nucleic acid molecules that facilitate the specific direction of an RNA-guided nuclease or other effector molecule (usually complexed with a gRNA molecule) onto a target sequence. In nature, crRNA and tracrRNA usually exist as two independent RNA molecules that make up gRNA. The term "tracrRNA" generally refers to a scaffold RNA that can bind to a Cas nuclease, and the term "crRNA", also referred to as a CRISPR RNA, generally refers to a stretch of nucleotide sequence that is complementary to the target DNA being targeted. The crRNA and tracRNA may also be fused into a single strand, in which case the gRNA may also be referred to as a single-stranded guide RNA (sgRNA). The sgRNA has become the most common form of gRNA used by those skilled in the art in CRISPR technology, and thus the terms "sgRNA" and "gRNA" may have the same meaning herein. The sgRNA can be synthesized artificially or can be prepared in vitro or in vivo from a DNA template. The sgRNA can bind to a Cas nuclease or target a target DNA, and can direct the Cas nuclease to cleave a DNA site complementary to the gRNA.

As used in the present application, a crRNA typically comprises a spacer sequence that mediates target recognition and a direct repeat sequence (also referred to herein as a "Direct repeat" or "DR sequence") that forms a complex with the CRISPR-Cas effector protein. In certain instances, a spacer sequence (also referred to as a guide sequence) is any polynucleotide sequence that has sufficient complementarity to a target sequence to hybridize to the target sequence and direct specific binding of the CRISPR-Cas system complex to the target sequence. In certain embodiments, the degree of complementarity between the spacer sequence and its corresponding target sequence when optimally aligned is at least 50%, at least 60%, at least 70%, at least 80%, at least 90%, at least 95%, at least 96%, at least 97%, at least 98%, or at least 99%. Determining the optimal alignment is within the capabilities of one of ordinary skill in the art. For example, there are publicly and commercially available alignment algorithms and programs such as, but not limited to, ClustalW, the Smith-Waterman algorithm in Matlab, Bowtie, Geneious, Biopython, and SeqMan.

In certain embodiments, the spacer sequence is at least 5, at least 10, at least 15, at least 16, at least 17, at least 18, at least 19, at least 20, at least 21, at least 22, at least 23, at least 24, at least 25, at least 26, at least 27, at least 28, at least 29, at least 30, at least 35, at least 40, at least 45, or at least 50 nucleotides in length. In certain embodiments, the spacer sequence is no more than 50, 45, 40, 35, 30, 25, 24, 23, 22, 21, 20, 15, 10, or fewer nucleotides in length. In certain embodiments, the spacer sequence is 10-30, 15-25, 15-22, 16-24, 19-25, or 19-22 nucleotides in length. In certain preferred embodiments, the spacer sequence is 20 nucleotides in length.

In certain embodiments, the direct repeat sequence is at least 10, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 40, at least 45, at least 50, at least 55, at least 56, at least 57, at least 58, at least 59, at least 60, at least 61, at least 62, at least 63, at least 64, at least 65, or at least 70 nucleotides in length. In certain embodiments, the direct repeat sequence is no more than 70, 65, 64, 63, 62, 61, 60, 59, 58, 57, 56, 55, 50, 45, 40, 35, 34, 33, 32, 31, 30, 29, 28, 27, 26, 25, 24, 23, 22, 21, 20, 15, 10, or fewer nucleotides in length. In certain embodiments, the direct repeat sequence is 55-70 nucleotides, e.g. 55-65 nucleotides, e.g. 60-65 nucleotides, e.g. 62-65 nucleotides, e.g. 63-64 nucleotides in length. In certain embodiments, the direct repeat sequence is 15-40 nucleotides, e.g. 15-25 nucleotides, e.g. 20-30 nucleotides, e.g. 22-36 nucleotides, e.g. 31 nucleotides in length.

In the present application, the terms "comprise" and "include" are used interchangeably, and generally refer to the inclusion of explicitly specified features, but not the exclusion of other elements. The term "at least" generally refers to cases where the stated number itself is included.

In the present application, the term "about" or "approximately" means within an acceptable error range for a particular value as determined by one of ordinary skill in the art, which depends in part on how the value is measured or determined, i.e., the limitations of the measurement system. For example, "about" or "approximately" may mean within 1 or more than 1 standard deviation according to practice in the art. Alternatively, "about" or "approximately" means a range of up to 10% or 20% (i.e., ±10% or ±20%).

In the present application, the term "selected from" generally means including the selected objects and all combinations thereof. For example, "selected from (:) A, B and C" is intended to include all combinations of A, B and C, for example, A, B, C, A+B, A+C, B+C or A+B+C.

Without wishing to be bound by any theory, the following examples are only for illustrating the enzymes of the present application and their fusion molecules, and are not used to limit the scope of the invention of the present application.

### EXAMPLES

### Example 1

### Determination of the cleavage activity of the Cas enzyme of the present application in eukaryotic cells

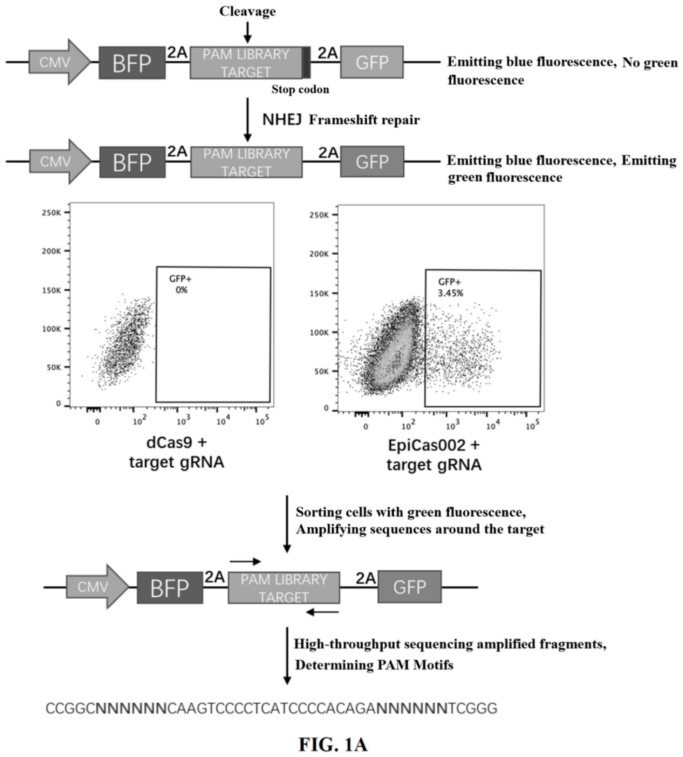

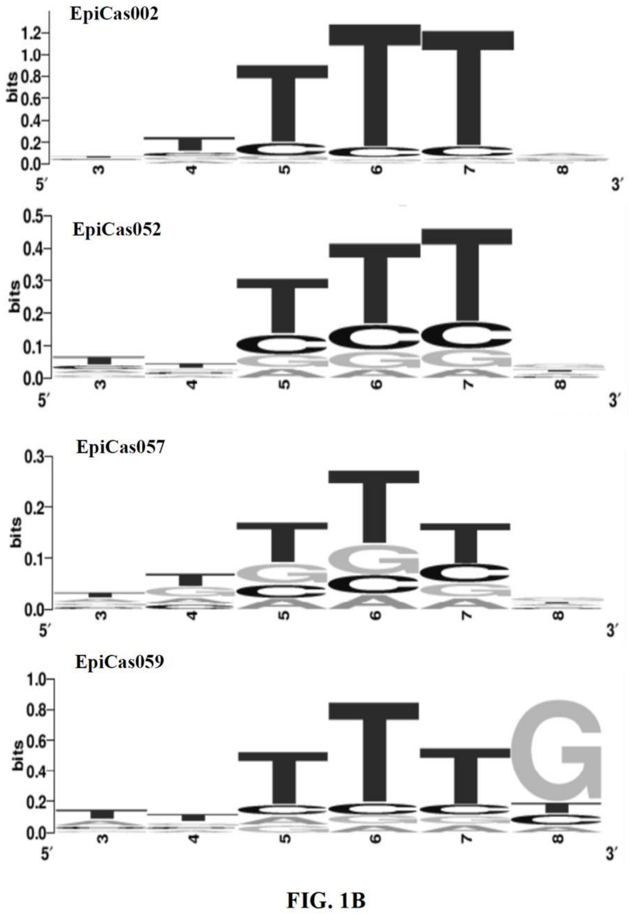

This example tested the activity of Cas enzymes provided in the present application using stably transfected 293T cells with a blue-green reporter system. As shown in FIG. 1, the reporter system carries a continuously expressed CMV promoter, a sequence encoding a blue fluorescent protein, a sequence encoding a green fluorescent protein, and a gRNA targeting sequence inserted in between; the gRNA targeting sequence is flanked by random N-base sequences that can be used to screen Cas enzymes with different protospacer adjacent motif (PAM) preferences.

The cleavage activity of the Cas enzyme of the present application would be determined by the fluorescence results displayed by the reporter system. The reporter system in which no cleavage occurs would stably express the blue fluorescent protein and emit blue fluorescence. However, the green fluorescent protein could not be expressed due to the presence of a stop codon before the sequence encoding the green fluorescent protein, and the reporter system would not emit green fluorescence. Only after DNA cleavage occurs near the gRNA targeting sequence, and the cell undergoes frameshift mutation in the process of nick repair, could the blue fluorescent protein be stably expressed and the green fluorescent protein be expressed at the same time. The fluorescence results of FIG. 1A indicated that after transfection with the Cas enzyme provided in the present application (SEQ ID NO: 2), the cells in the experimental group with cleavage activity would produce a population of cells clearly expressing green fluorescence.

The green fluorescent cell population was enriched by flow sorting, the genome was extracted and then the target region was amplified, and the PAM motif required for gRNA binding of the Cas enzyme provided in the present application could be determined by high-throughput sequencing (as shown in FIG. 1B).

In this example, an exemplary nucleotide sequence encoding the reporter system is shown below (blue and green fluorescent protein sequence in bold, linker sequence in italics, 2A cleavage peptide sequence underlined, and exemplary gRNA targeting sequence comprising random N bases (SEQ ID NO: 59) in bold and underlined):

ATGAGCGAGCTGATTAAGGAGAACATGCACATGAAGCTGTACATGGAGGGCA
CCGTGGACAACCATCACTTCAAGTGCACATCCGAGGGCGAAGGCAAGCCCTAC

GAGGGCACCCAGACCATGAGAATCAAGGTGGTCGAGGGCGGCCCTCTCCCCTT
CGCCTTCGACATCCTGGCTACTAGCTTCCTCTACGGCAGCAAGACCTTCATCAA
CCACACCCAGGGCATCCCCGACTTCTTCAAGCAGTCCTTCCCTGAGGGCTTCAC
ATGGGAGAGAGTCACCACATACGAAGACGGGGGCGTGCTGACCGCTACCCAGG
ACACCAGCCTCCAGGACGGCTGCCTCATCTACAACGTCAAGATCAGAGGGGTGA
ACTTCACATCCAACGGCCCTGTGATGCAGAAGAAAACACTCGGCTGGGAGGCC
TTCACCGAGACGCTGTACCCCGCTGACGGCGGCCTGGAAGGCAGAAACGACAT
GGCCCTGAAGCTCGTGGGCGGGAGCCATCTGATCGCAAACATCAAGACCACATA
TAGATCCAAGAAACCCGCTAAGAACCTCAAGATGCCTGGCGTCTACTATGTGGA
CTACAGACTGGAAAGAATCAAGGAGGCCAACAACGAGACCTACGTCGAGCAGC
ACGAGGTGGCAGTGGCCAGATACTGCGACCTCCCTAGCAAACTGGGGCACAAG
CTTAATGGATCCGGCGCAACAAACTTCtctctgctgaaacaagccggagatgTCGAAGAGAATCCT
GGACCGGCTGAGCAGGCTCGTTGGGAAGCAGCCTCCAAAGAAACCATCAAGAAAACCAC
CAAGCCCTGTCCCCGCTGCCATGTACCAGTGGAAAAAAATGGAGGCTGCATGCACATGAA
GTGTCCGCAGCCCCAGTGCAGGCTCGAGTGGTGCTGGAACTGTGGCTGCGAGcccCCGG
CNNNNNNCAAGTCCCCTCATCCCCACAGANNNNNNTCgggTtaGGGGACCACTGGTTC
GACGTGAGCGGTTCTGGCGAAGGGCGAGGTTCCTTGCTGACCTGTGGCGATGTAGAA
GAAAATCCCGGCCCCGGATCCGTGAGCAAGGGCGAGGAGCTGTTCACCGGGGTG
GTGCCCATCCTGGTCGAGCTGGACGGCGACGTAAACGGCCACAAGTTCAGCGT
GTCCGGCGAGGGCGAGGGCGATGCCACCTACGGCAAGCTGACCCTGAAGTTCA
TCTGCACCACCGGCAAGCTGCCCGTGCCCTGGCCCACCCTCGTGACCACCCTG
ACCTACGGCGTGCAGTGCTTCAGCCGCTACCCCGACCACATGAAGCAGCACGAC
TTCTTCAAGTCCGCCATGCCCGAAGGCTACGTCCAGGAGCGCACCATCTTCTTC
AAGGACGACGGCAACTACAAGACCCGCGCCGAGGTGAAGTTCGAGGGCGACAC
CCTGGTGAACCGCATCGAGCTGAAGGGCATCGACTTCAAGGAGGACGGCAACA
TCCTGGGGCACAAGCTGGAGTACAACTACAACAGCCACAACGTCTATATCATGG
CCGACAAGCAGAAGAACGGCATCAAGGTGAACTTCAAGATCCGCCACAACATCG

AGGACGGCAGCGTGCAGCTCGCCGACCACTACCAGCAGAACACCCCCATCGGC
GACGGCCCCGTGCTGCTGCCCGACAACCACTACCTGAGCACCCAGTCCGCCCT
GAGCAAAGACCCCAACGAGAAGCGCGATCACATGGTCCTGCTGGAGTTCGTGA
CCGCCGCCGGGATCACTCTCGGCATGGACGAGCTGTACAAGTAA (SEQ ID NO:
61).

### Example 2

### Determination of the strength of the cleavage activity of the Cas enzyme of the present application in eukaryotic cells

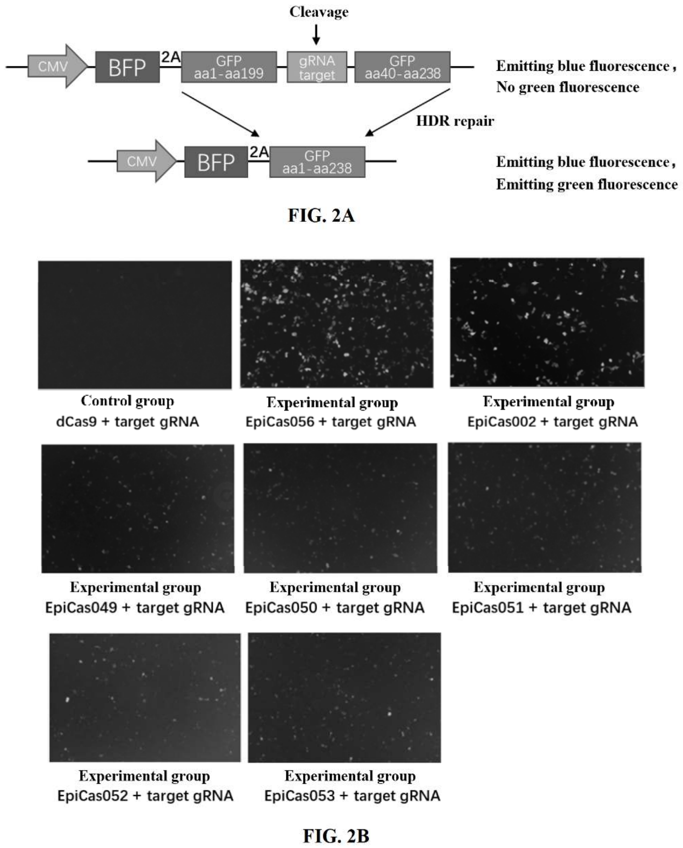

The Cas enzymes provided in the present application (SEQ ID NOs: 2, 47-51 and 54), the corresponding gRNA and the reporter plasmid with gRNA targeting site were co-transfected into HEK293T cells, and the reporter systems required for different Cas enzymes could be quickly constructed by modifying the targeting sequence on the reporter plasmid. As shown in FIG. 2A, the reporter system cells without cleavage only expressed blue fluorescent protein, and the green fluorescent protein could not be normally expressed because it was blocked. At this time, the reporter system only emitted blue fluorescence. After cleavage occurs at the gRNA targeting site, the cells would repair the DNA through homologous recombination, thereby obtaining a complete green fluorescent protein expression frame, which caused the reporter system to emit green fluorescence.

The Cas enzyme with stronger cleavage activity would produce a higher proportion of DNA cleavage, thereby obtaining more copies of the repaired green fluorescent protein, and the cells exhibited stronger green fluorescence. Therefore, this example quickly compared the cleavage activities of different Cas enzymes by comparing the proportion and fluorescence intensity of green fluorescent cells. The comparison results are shown in FIG. 2B.

In this example, the exemplary nucleotide sequence encoding the reporter system is shown below (blue and green fluorescent protein sequences in bold, linker sequence in italics, 2A cleavage peptide sequence underlined; exemplary gRNA targeting sequence (SEQ ID NO: 60) in bold and underlined, where the TTTG and TGG at both ends of the sequence would be set to different sequences according to the PAM preferences of different Cas enzymes):

ATGAGCGAGCTGATTAAGGAGAACATGCACATGAAGCTGTACATGGAGGGCA
CCGTGGACAACCATCACTTCAAGTGCACATCCGAGGGCGAAGGCAAGCCCTAC
GAGGGCACCCAGACCATGAGAATCAAGGTGGTCGAGGGCGGCCCTCTCCCCTT
CGCCTTCGACATCCTGGCTACTAGCTTCCTCTACGGCAGCAAGACCTTCATCAA
CCACACCCAGGGCATCCCCGACTTCTTCAAGCAGTCCTTCCCTGAGGGCTTCAC
ATGGGAGAGAGTCACCACATACGAAGACGGGGGCGTGCTGACCGCTACCCAGG
ACACCAGCCTCCAGGACGGCTGCCTCATCTACAACGTCAAGATCAGAGGGGTGA
ACTTCACATCCAACGGCCCTGTGATGCAGAAGAAAACACTCGGCTGGGAGGCC
TTCACCGAGACGCTGTACCCCGCTGACGGCGGCCTGGAAGGCAGAAACGACAT
GGCCCTGAAGCTCGTGGGCGGGAGCCATCTGATCGCAAACATCAAGACCACATA
TAGATCCAAGAAACCCGCTAAGAACCTCAAGATGCCTGGCGTCTACTATGTGGA
CTACAGACTGGAAAGAATCAAGGAGGCCAACAACGAGACCTACGTCGAGCAGC
ACGAGGTGGCAGTGGCCAGATACTGCGACCTCCCTAGCAAACTGGGGCACAAG
CTTAATGGATCCGGCGCAACAAACTTCtctctgctgaaacaagccggagatgTCGAAGAGAATCCT
GGACCGGCTgtgagcaagggcgaggagcTGTTCACCGGGGTGGTGCCCATCCTGGTCGAG
CTGGACGGCGACGTAAACGGCCACAAGTTCAGCGTGTCCGGCGAGGGCGAGGG
CGATGCCACCTACGGCAAGCTGACCCTGAAGTTCATCTGCACCACCGGCAAGCT
GCCCGTGCCCTGGCCCACCCTCGTGACCACCCTGACCTACGGCGTGCAGTGCT
TCAGCCGCTACCCCGACCACATGAAGCAGCACGACTTCTTCAAGTCCGCCATGC
CCGAAGGCTACGTCCAGGAGCGCACCATCTTCTTCAAGGACGACGGCAACTACA
AGACCCGCGCCGAGGTGAAGTTCGAGGGCGACACCCTGGTGAACCGCATCGAG
CTGAAGGGCATCGACTTCAAGGAGGACGGCAACATCCTGGGGCACAAGCTGGA
GTACAACTACAACAGCCACAACGTCTATATCATGGCCGACAAGCAGAAGAACGG
CATCAAGGTGAACTTCAAGATCCGCCACAACATCGAGGACGGCAGCGTGCAGCT
CGCCGACCACTACCAGCAGAACACCCCCATCGGCGACGGCCCCGTGctgctgcccgac

aaccacTGAtaaggatccTTTGAAGTCCCCTCATCCCCACAGTGGtaggatatcggcaagctgaccctga
aGTTCATCTGCACCACCGGCAAGCTGCCCGTGCCCTGGCCCACCCTCGTGACCA
CCCTGACCTACGGCGTGCAGTGCTTCAGCCGCTACCCCGACCACATGAAGCAGC
ACGACTTCTTCAAGTCCGCCATGCCCGAAGGCTACGTCCAGGAGCGCACCATCT
TCTTCAAGGACGACGGCAACTACAAGACCCGCGCCGAGGTGAAGTTCGAGGGC
GACACCCTGGTGAACCGCATCGAGCTGAAGGGCATCGACTTCAAGGAGGACGG
CAACATCCTGGGGCACAAGCTGGAGTACAACTACAACAGCCACAACGTCTATAT
CATGGCCGACAAGCAGAAGAACGGCATCAAGGTGAACTTCAAGATCCGCCACA
ACATCGAGGACGGCAGCGTGCAGCTCGCCGACCACTACCAGCAGAACACCCCC
ATCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGAGT
TCGTGACCGCCGCCGGGATCACTCTCGgcatggacgagctgtacAAGtaa (SEQ ID NO: 62).

### Example 3

### Determination of the in vitro cleavage activity and PAM sequence of the Cas enzyme of the present application

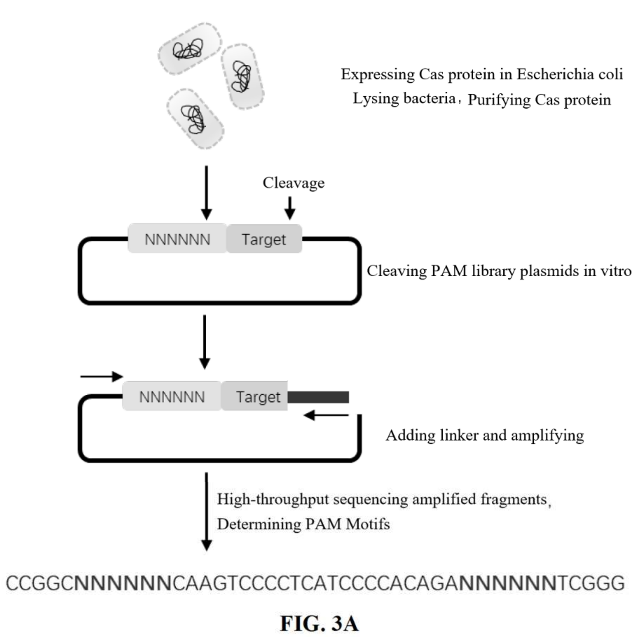

As shown in the experimental design flow chart of this embodiment in FIG. 3A, the Cas enzyme provided in the present application was expressed and purified by E. coli, and reacted with a plasmid library containing a PAM library together with an in vitro transcribed gRNA (the gRNA targeting sequence was SEQ ID NO: 59 flanked by random N-base sequences to determine PAM preference). The Cas enzyme having cleavage activity would cleave the plasmid library to form linearized DNA. By in vitro ligation, the linker fragment was ligated to the library fragment after linearization of DNA, and then the vector and the linker ligated library fragment were amplified by specific PCR primers and high-throughput sequencing was performed.

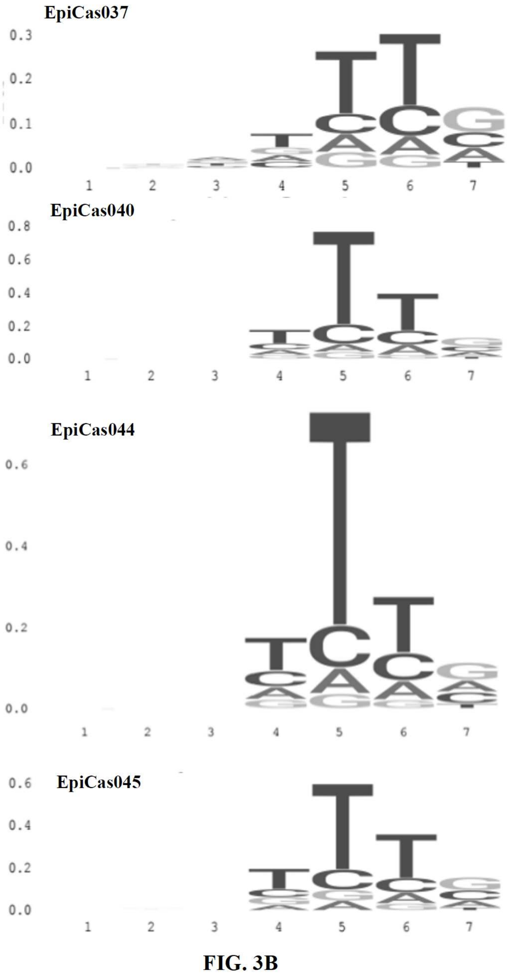

The analytical sequencing results shown in FIG. 3B indicated the preference of Cas enzymes with cleavage activity for PAM motifs. These results indicated that the four proteins EpiCas037 (SEQ ID NO: 36), EpiCas044 (SEQ ID NO: 43), EpiCas040 (SEQ ID NO: 39) and EpiCas045 (SEQ ID NO: 44) all had cleavage activity and preferred T-rich PAM motifs.

### Example 4

### Detection of the activity of the Cas enzyme of the present application at endogenous sites

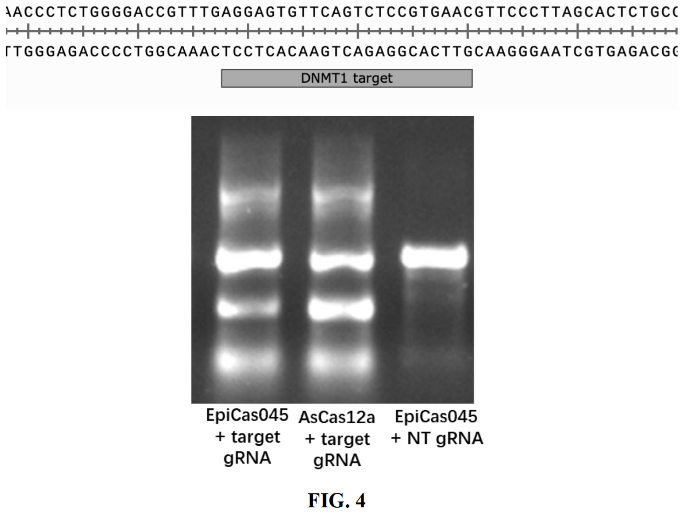

According to the PAM motifs of different Cas enzymes determined in Example 3, eligible sites were found at endogenous sites of HEK293T, and gRNA (gRNA targeting sequence was SEQ ID NO: 89) was constructed. The Cas enzyme (SEQ ID NOs: 44) provided in the present application and the corresponding gRNA were co-transfected into HEK293T cells, and the transfection-positive cells were enriched by flow sorting 3 days after transfection, and the target region was amplified after lysing the cells. The amplified PCR fragment was reacted with T7 endonuclease. If the Cas enzyme cleaved the target site, various randomly repaired sequences would be formed, and these sequences would be cleaved by T7 enzyme to produce bands other than the target band. Therefore, the results shown in FIG. 4 indicated that the Cas enzyme had cleavage activity at the endogenous sites.

### Example 5

### Determination of the strength of the cleavage activity of the Cas enzyme mutant of the present application in eukaryotic cells

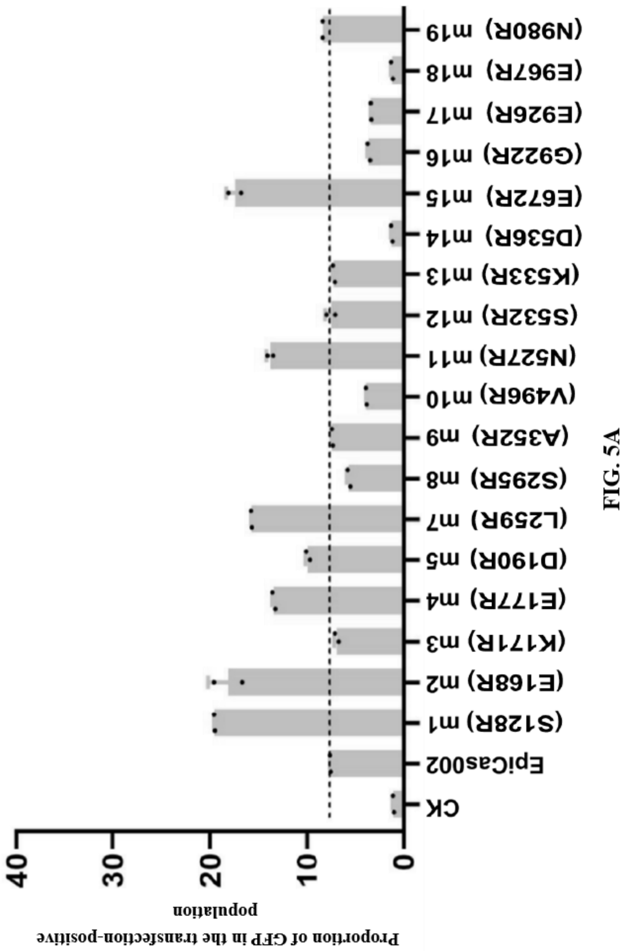

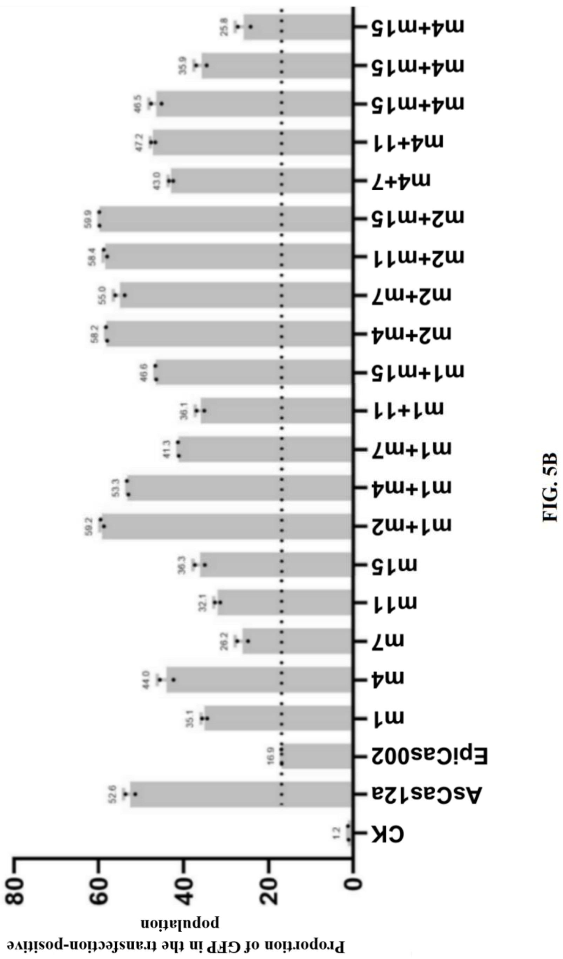

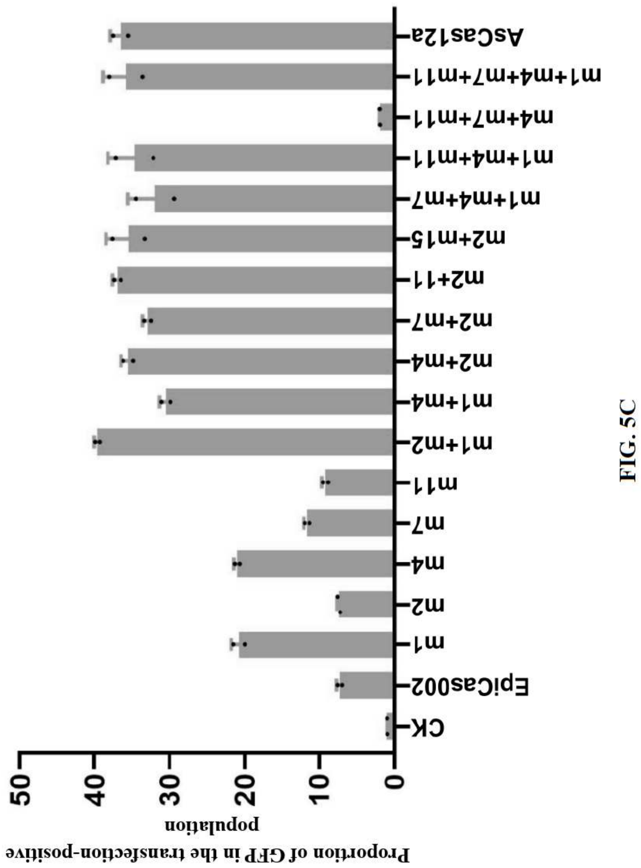

By site-directed mutagenesis, different sites of the Cas enzyme (SEQ ID NO: 2) provided in the present application were mutated to target amino acids (see the table below), and then the detection as described in Example 2 was performed. Among them, the DR (direct repeat) region sequence and spacer sequence of the gRNA were as set forth in SEQ ID NOs: 64 and 60, respectively. The results shown in FIG. 5A indicated that the activities of the m1, m2, m4, m5, m7, m11, and m15 mutants were significantly increased compared to the wild type. After superimposing these effective mutations, the activity detection by the same method was performed. The results shown in FIG. 5B indicated that the double mutants formed by superimposing two mutations selected from the following table could further enhance the activity, and the activity of some double mutants was comparable to that of AsCas12a; the results shown in FIG. 5C indicated that the triple mutants and quadruple mutants formed by further superimposing the effective single mutations and double mutations still maintain the original activities, and the activity is comparable to that of AsCas12a. The amino acid sequence of AsCas12a for control is as set forth in SEQ ID NO: 127.

| | |
|---|---|
| EpiCas002-m1 | S128R |
| EpiCas002-m2 | E168R |
| EpiCas002-m3 | K171R |
| EpiCas002-m4 | E177R |
| EpiCas002-m5 | D190R |
| EpiCas002-m6 | E248R |
| EpiCas002-m7 | L259R |
| EpiCas002-m8 | S295R |
| EpiCas002-m9 | A352R |
| EpiCas002-m10 | V496R |
| EpiCas002-m11 | N527R |
| EpiCas002-m12 | S532R |
| EpiCas002-m13 | K533R |
| EpiCas002-m14 | D536R |
| EpiCas002-m15 | E672R |
| EpiCas002-m16 | G922R |
| EpiCas002-m17 | E926R |
| EpiCas002-m18 | E967R |
| EpiCas002-m19 | N980R |

### Example 6

### Optimization of CRISPR/Cas editing system

This example optimized an editing system comprising the Cas enzyme provided (SEQ ID NO: 57) and gRNA in the present application. The Cas enzyme activity was detected using the reporter system and detection method as described in Example 2.

### gRNA Optimization:

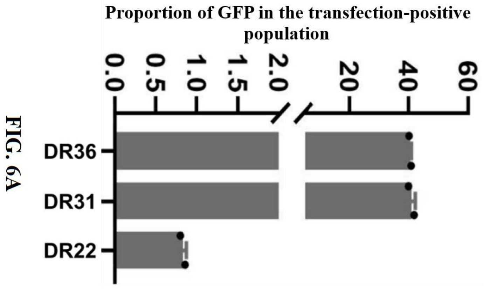

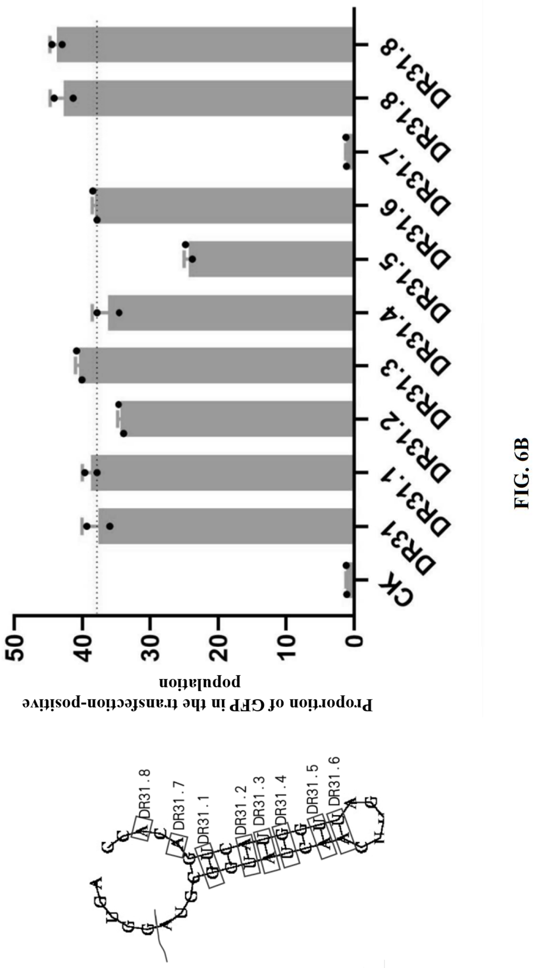

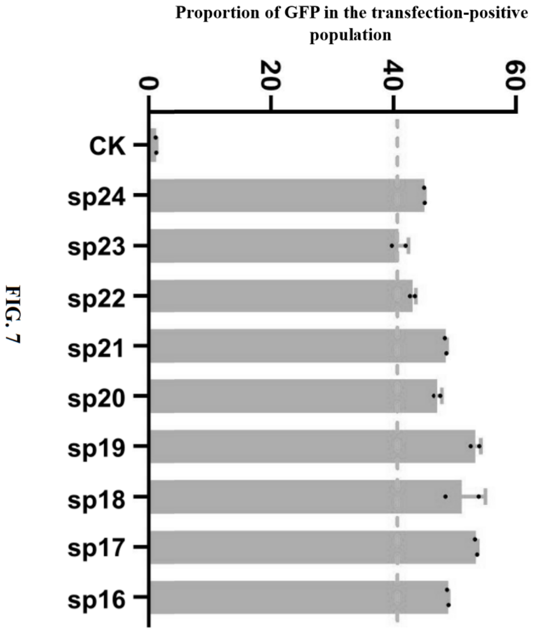

The DR (direct repeat) region of the gRNA was changed by molecular cloning to 36 nt (SEQ ID NO: 87), 31 nt (SEQ ID NO: 91), 22 nt (SEQ ID NO: 90). The results shown in FIG. 6A indicated that a DR of 31 nt length could maintain 100% Cas enzyme activity. When the base pairs of the DR region were altered by molecular cloning to improve its stability (the nucleotide sequences are as set forth in SEQ ID NOs: 92-99 for DRs of 31.1 to 31.8), the results shown in FIG. 6B indicated that most of the alterations had little effect on activity, whereas the alterations in 31.8 were the most effective in improving activity. In this example, the length of the spacer of the gRNA used to target DNA was changed by molecular cloning to improve its binding efficiency, thereby enhancing Cas enzyme activity. As shown in FIG. 7, when the spacer length varied from 24 nt to 16 nt (SEQ ID NOs: 100-103, 60 and 104-107), the Cas enzymes provided in the present application could maintain their activity.

### Cas enzyme mutant:

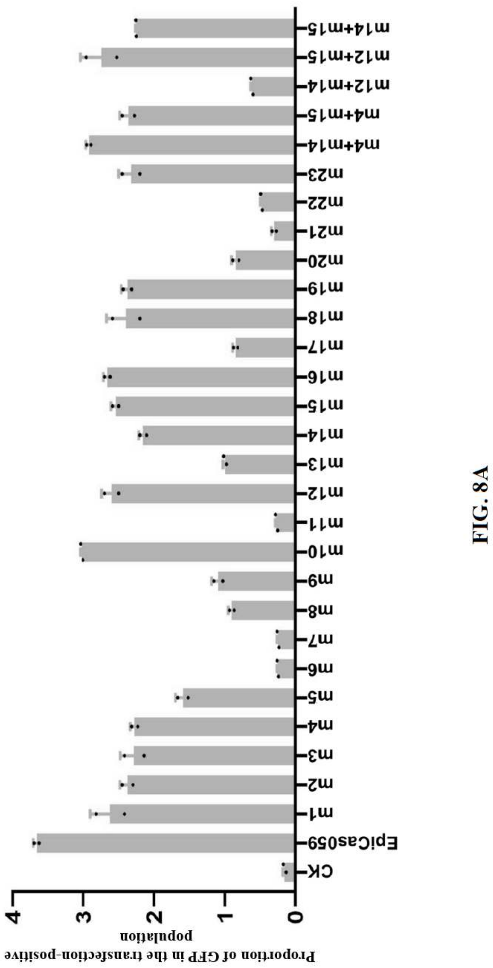

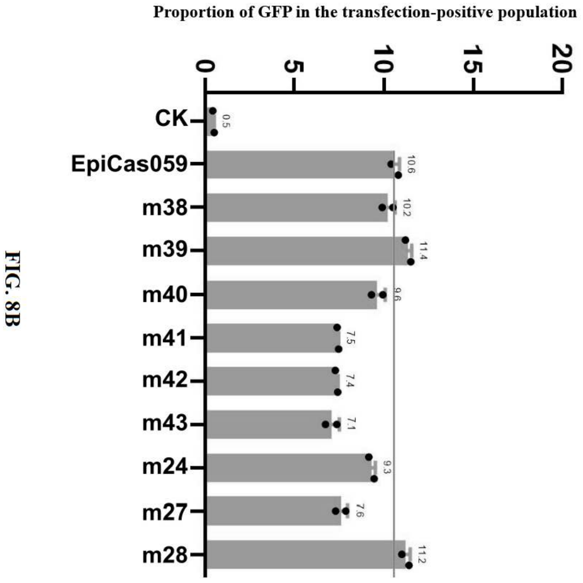

By site-directed mutagenesis, different sites of the Cas enzyme (SEQ ID NO: 57) provided in the present application were mutated to target amino acids (see the table below), and then the detection as described in Example 2 was performed. Among them, the DR (direct repeat) region sequence and spacer sequence of the gRNA were as set forth in SEQ ID NOs: 91 and 60, respectively. The results shown in FIGs. 8A-8B indicated that most single mutations had little effect on Cas enzyme activity, and if the single mutations with better activity optimization effect were superimposed, it could be found that the formed double mutants could still maintain high activity.

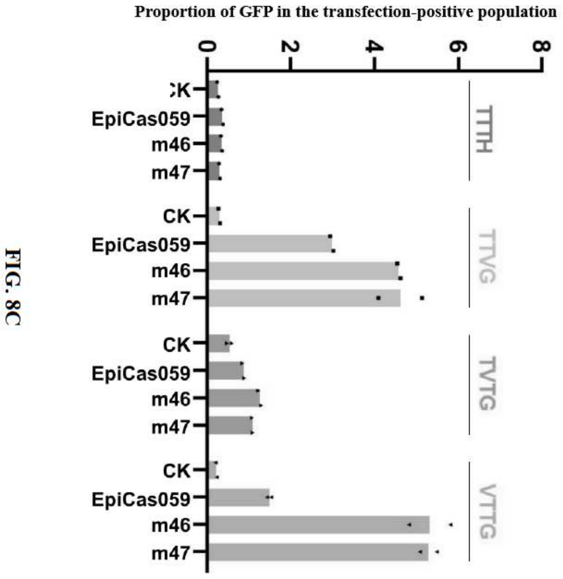

In this example, on the basis of the reporter system described in Example 2, TTTG was mutated into TTTH, TTVG, TVTG and VTTG respectively (H stands for A/C/T, V stands for A/C/G, and 3 plasmids were mixed in equal proportions and then transfected) to construct a new PAM reporter system. The results shown in FIG. 8C indicated that some mutants could broaden the range of PAM recognition, especially in the recognition of PAM targets of VTTG and TTVG. Compared with the wild type, the activity of the Cas enzyme provided in the present application was significantly improved.

| | |
|---|---|
| EpiCas059-m1 | K57R |
| EpiCas059-m2 | Y258R |
| EpiCas059-m3 | K264R |
| EpiCas059-m4 | K272R |
| EpiCas059-m5 | D621R |
| EpiCas059-m6 | Q649R |
| EpiCas059-m7 | E681R |
| EpiCas059-m8 | Y754R |
| EpiCas059-m9 | S767R |
| EpiCas059-m10 | Q770R |
| EpiCas059-m11 | D823R |
| EpiCas059-m12 | K850R |
| EpiCas059-m13 | V861R |
| EpiCas059-m14 | K186R |
| EpiCas059-m15 | T197R |
| EpiCas059-m16 | V242R |
| EpiCas059-m17 | K304R |
| EpiCas059-m18 | T319R |
| EpiCas059-m19 | E397R |
| EpiCas059-m20 | Y364R |
| EpiCas059-m21 | S421R |
| E piCas059-m22 | V453R |
| EpiCas059-m23 | P627R |
| EpiCas059-m24 | K9R |
| EpiCas059-m25 | I10R |
| EpiCas059-m26 | C39R |
| EpiCas059-m27 | N53R |
| EpiCas059-m28 | H65R |
| EpiCas059-m29 | S69R |
| EpiCas059-m30 | E81R |
| EpiCas059-m31 | K95R |
| EpiCas059-m32 | D98R |
| EpiCas059-m33 | V103R |
| EpiCas059-m34 | G112R |
| EpiCas059-m35 | I115R |
| EpiCas059-m36 | G120R |
| EpiCas059-m37 | K123R |
| EpiCas059-m38 | A153N |
| EpiCas059-m39 | A153R |
| EpiCas059-m40 | T346S |
| EpiCas059-m41 | T346R |
| EpiCas059-m42 | D347N |
| EpiCas059-m43 | D347R |
| EpiCas059-m44 | D183Y/T458K/G528C |
| EpiCas059-m45 | E144K/S633N |
| EpiCas059-m46 | E144K/D183Y/T458K/G528C/S633N |
| EpiCas059-m47 | E144K/T458K |

### Example 7

### Detection of the effects of inactivated Cas enzyme (dCas) at endogenous sites

### Base editing effect of dCas-CBE:

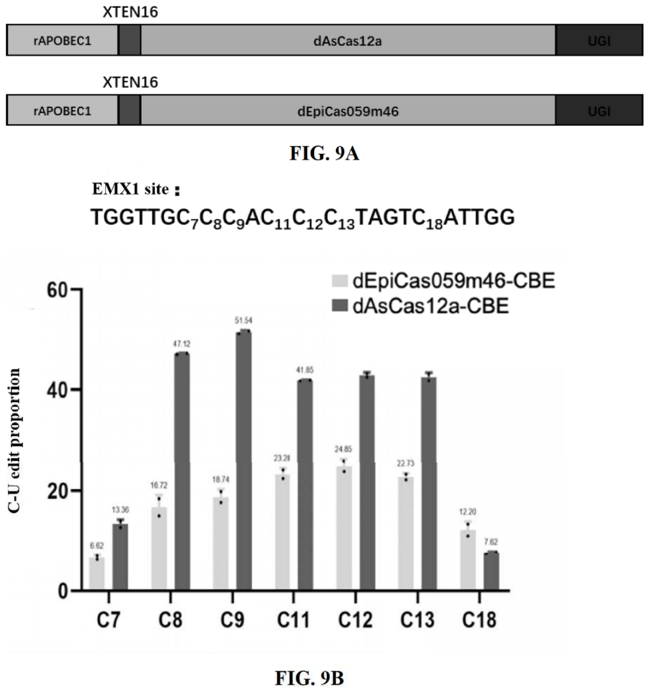

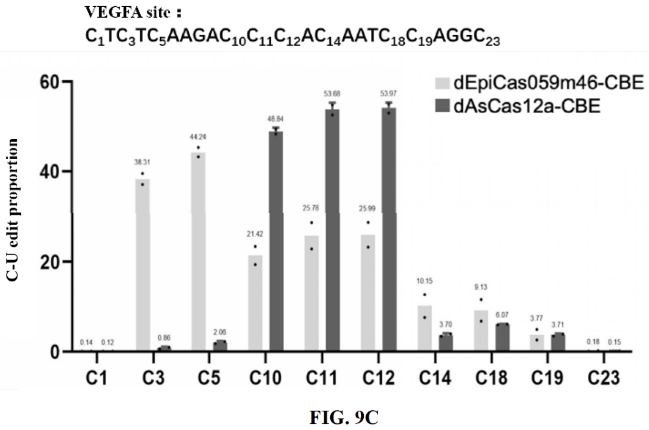

The Cas enzyme provided in the present application (SEQ ID NO: 57) was inactivated and then fused with rAPOBEC1 and UGI (FIG. 9A) to obtain a cytosine base editor based on the Cas enzyme provided in the present application. The cytosine base editor was co-transfected into HEK293T cells with the corresponding gRNA (the DR sequence is as set forth in SEQ ID NO: 91, and the spacer sequences targeting the EMX1 site and the VEGFA site are as set forth in SEQ ID NOs: 108 and 109, respectively). 48 hours after transfection, transfection-positive cells were sorted, the genome was extracted, the target site was amplified, and the base editing efficiency was determined by Sanger sequencing. The results are shown in FIGs. 9B-9C.

The amino acid sequence of the dEpiCas059m46-CBE editor is shown below (SEQ ID NO: 116, NLS in italics, rAPOBEC1 in italics and bold, dEpiCas059 in bold, UGI in italics and underlined, P2A cleavage peptide underlined, and mCherry marker in < >; the plasmid sequence is SEQ ID NO: 117):

MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSRAI
TEFLSRYPHVTLFIYIARLYHHADPRNRQGLRDLISSGVTIQIMTEQESGYCWRNFVNYS
PSNEAHWPRYPHLWVRLYVLELYCIILGLPPCLNILRRKQPQLTFFTIALQSCHYQRLPPH
ILWATGLKSGSETPGTSESATPESGSEETRTPEKIACQQDRSAFFKLGKLPQKTTSAIYH
LMRCSKRYIDNVSETISNNIEKAAMTSDNHIFRSALKEEYKKIYREKFYPDVTGQPFP
DKGPDNTHIVERTVENLCGQYISALKGVMKVRKPKWQDDIFEDESDKVIAKMLMA
SGNEADIHPKLEKKIASAKKKDKMDEWQKVLDRYYYIKSKTDIEWTYTTLLYDHIA
RECARSMFEDGNKPKFCYTNGNKFAKQVVDELWGSERVEYEYEKYLHCRTLEAY
WNEENKYFFLENQKKSKKTYEARQIIDHFIENEDAVKAETEEYMAKVAKEFGENGG
CKFRTENILYLVNSVSINKKQDEERCPIFKVTDIPQYAEAGIDRIGECKYVIKLKLFTV

GERYGTTYMPEAFSGLYDRRTNQRYDLIDRACKGSYVVYLPIYISDKDSRKWSDRLK
NTFTMSAFSISERKTKKDGRVLCVNLPYKHIQHKPRNYFKVYPTYGVAINVKHSLFS
TNATNLNGYIDVNGELIDFYNSIGRPDKLKNINLAVTLGDKCGTAGNHTNFLLVSDL
GDWDDHGKKRNDRFVKREDGTYYSRPDLLADGLNFLKKKYSGDRKSYDYINTTIA
FRNNQTRLRCIDKEWNDRKRAYDEEHGKPEKGERNGFYPTEDAERLTMERQALRR
EIAENTRQVIAYMLWLFQENGIKSIALAHLDCGSWKNFRDQLKPKWFCNNCKYWA
EKYGIPYEATKYYRSNRKYFDIEDGELIPENVKLNALGEAENDKRYFQTSIAKAVGM
SSIKQMFSIVSQNYDIQVNYTEQAYTSQTDSRNGTVDKDFRVSQECYIGSDGTKMNC
AFNAAVNIADRISDDTLREEFNKELPMKEFVKYGATQIVSRNRRWNAENAERVTMA
AFQKRKNADGGAHTAAVKPTSKTEESSLKLVAEPKKKRKVSGGSTNLSDIIEKETGKQL
VIQESILMLPEEVEEVIGNKPESDILVHTAYDESTDENVMLLTSDAPEYKPWALVIQDSNGENKI
KMLSGGSPKKKRKVGSKRTGSGATNFSLLKQAGDVEENPGPVN<MVSKGEEDNMAIIKE
FMRFKVHMEGSVNGHEFEIEGEGEGRPYEGTQTAKLKVTKGGPLPFAWDILSPQFMYGS
KAYVKHPADIPDYLKLSFPEGFKWERVMNFEDGGVVTVTQDSSLQDGEFIYKVKLRGTN
FPSDGPVMQKKTMGWEASSERMYPEDGALKGEIKQRLKLKDGGHYDAEVKTTYKAKK
PVQLPGAYNVNIKLDITSHNEDYTIVEQYERAEGRHSTGGMDELYK>*

The amino acid sequence of the dAsCas12a-CBE editor is shown below (SEQ ID NO: 118, NLS in italics, rAPOBEC1 in italics and bold, dAsCas12a in bold, UGI in italics and underlined, P2A cleavage peptide underlined, and mCherry marker in < >; the plasmid sequence is SEQ ID NO: 119):

MKRTADGSEFESPKKKRKVSSETGPVAVDPTLRRRIEPHEFEVFFDPRELRKETCLLY
EINWGGRHSIWRHTSQNTNKHVEVNFIEKFTTERYFCPNTRCSITWFLSWSPCGECSRAI
TEFLSRYPHVTLFIYIARLYHHADPRNRQGLRDLISSGVTIQIMTEQESGYCWRNFVNYS
PSNEAHWPRYPHLWVRLYVLELYCIILGLPPCLNILRRKQPQLTFFTIALQSCHYQRLPPH
ILWATGLKSGSETPGTSESATPESGSSKLEKFTNCYSLSKTLRFKAIPVGKTQENIDNKR
LLVEDEKRAEDYKGVKKLLDRYYLSFINDVLHSIKLKNLNNYISLFRKKTRTEKENK
ELENLEINLRKEIAKAFKGNEGYKSLFKKDIIETILPEFLDDKDEIALVNSFNGFTTAF

TGFFDNRENMFSEEAKSTSIAFRCINENLTRYISNMDIFEKVDAIFDKHEVQEIKEKIL
NSDYDVEDFFEGEFFNFVLTQEGIDVYNAIIGGFVTESGEKIKGLNEYINLYNQKTKQ
KLPKFKPLYKQVLSDRESLSFYGEGYTSDEEVLEVFRNTLNKNSEIFSSIKKLEKLFK
NFDEYSSAGIFVKNGPAISTISKDIFGEWNVIRDKWNAEYDDIHLKKKAVVTEKYED
DRRKSFKKIGSFSLEQLQEYADADLSVVEKLKEIIIQKVDEIYKVYGSSEKLFDADFV
LEKSLKKNDAVVAIMKDLLDSVKSFENYIKAFFGEGKETNRDESFYGDFVLAYDILL
KVDHIYDAIRNYVTQKPYSKDKFKLYFQNPQFMGGWDKDKETDYRATILRYGSKY
YLAIMDKKYAKCLQKIDKDDVNGNYEKINYKLLPGPNKMLPKVFFSKKWMAYYNP
SEDIQKIYKNGTFKKGDMFNLNDCHKLIDFFKDSISRYPKWSNAYDFNFSETEKYKD
IAGFYREVEEQGYKVSFESASKKEVDKLVEEGKLYMFQIYNKDFSDKSHGTPNLHT
MYFKLLFDENNHGQIRLSGGAELFMRRASLKKEELVVHPANSPIANKNPDNPKKTT
TLSYDVYKDKRFSEDQYELHIPIAINKCPKNIFKINTEVRVLLKHDDNPYVIGIARGE
RNLLYIVVVDGKGNIVEQYSLNEIINNFNGIRIKTDYHSLLDKKEKERFEARQNWTSI
ENIKELKAGYISQVVHKICELVEKYDAVIALADLNSGFKNSRVKVEKQVYQKFEKM
LIDKLNYMVDKKSNPCATGGALKGYQITNKFESFKSMSTQNGFIFYIPAWLTSKIDPS
TGFVNLLKTKYTSIADSKKFISSFDRIMYVPEEDLFEFALDYKNFSRTDADYIKKWK
LYSYGNRIRIFRNPKKNNVFDWEEVCLTSAYKELFNKYGINYQQGDIRALLCEQSDK
AFYSSFMALMSLMLQMRNSITGRTDVAFLISPVKNSDGIFYDSRNYEAQENAILPKN
ADANGAYNIARKVLWAIGQFKKAEDEKLDKVKIAISNKEWLEYAQTSVKHPKKKRK
VSGGSTNLSDIIEKETGKQLVIQESILMLPEEVEEVIGNKPESDILVHTAYDESTDENVMLLTSD
APEYKPWALVIQDSNGENKIKMLSGGSPKKKRKVGSKRTGSGATNFSLLKQAGDVEENPGP
VN<MVSKGEEDNMAIIKEFMRFKVHMEGSVNGHEFEIEGEGEGRPYEGTQTAKLKVTK
GGPLPFAWDILSPQFMYGSKAYVKHPADIPDYLKLSFPEGFKWERVMNFEDGGVVTVTQ
DSSLQDGEFIYKVKLRGTNFPSDGPVMQKKTMGWEASSERMYPEDGALKGEIKQRLKL
KDGGHYDAEVKTTYKAKKPVQLPGAYNVNIKLDITSHNEDYTIVEQYERAEGRHSTGG
MDELYK>*

### Gene activation effect of dCas-SPH:

The Cas enzyme provided in the present application (SEQ ID NO: 55) was subjected to inactivation treatment and then fused with 10 × GCN4 to recruit a fusion peptide, i.e., scFV-P65-HSF1, thereby obtaining a gene activation tool based on the Cas enzyme provided in the present application. The principle of this tool was based on the fact that GCN4 could spontaneously recognize and bind to scFV, thereby enriching P65 and HSF1 effectors with transcriptional activation function near the target site of the Cas enzyme, and then activating gene expression at the target site.

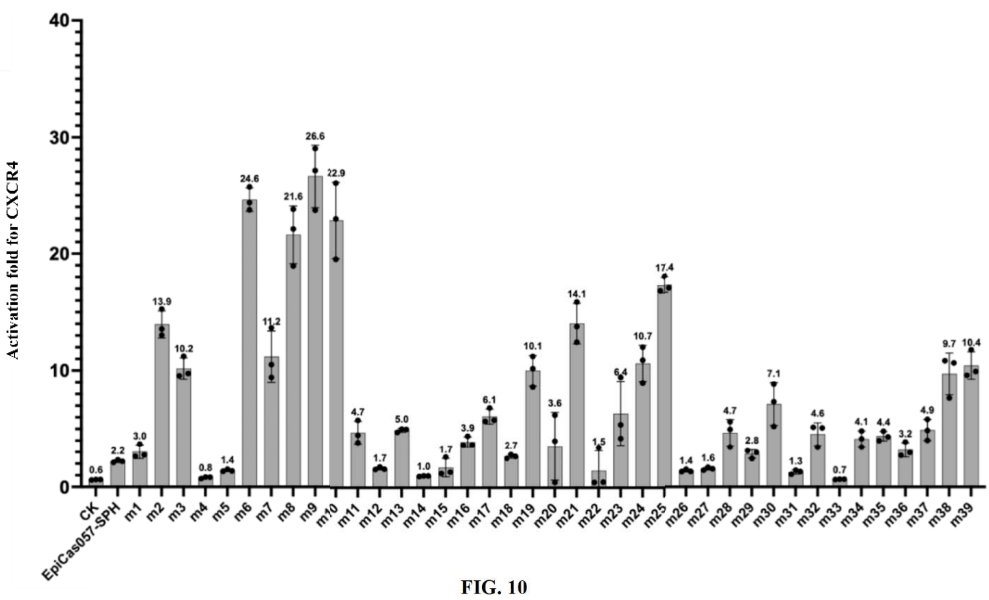

The transcription activation tool and gRNA targeting CXCR4 (spacer sequence is as set forth in SEQ ID NOs: 120-123, and the 4 gRNAs was mixed in equal proportions) were co-transfected into HEK293T cells. 48 hours after transfection, the cells were collected and stained with PE antihuman CXCR4 antibody (BioLegendg, 306506). The fluorescence intensity of the PE channel was detected by flow cytometry to reflect the expression intensity of CXCR4. The activation efficiency (MFI fold change) was calculated by dividing the mean fluorescence intensity of PE in the transfection-positive group by the mean fluorescence intensity of PE in the transfection-negative group, and used to represent the activation intensity of different activation tools (FIG. 10) and, therefore, the DNA binding efficacy of different tools.

The amino acid sequence of the scFV-P65-HSF1 fusion peptide is shown below (SEQ ID NO: 124, NLS in italics, P65 and HSF1 in italics and bold, scFV in bold, HA tag in italics and underlined, linker peptide underlined, and Flag marker in < >):

MGPDIVMTQSPSSLSASVGDRVTITCRSSTGAVTTSNYASWVQEKPGKLFKGLIG
GTNNRAPGVPSRFSGSLIGDKATLTISSLQPEDFATYFCALWYSNHWVFGQGTKVEL
KRGGGGSGGGGSGGGGSSGGGSEVKLLESGGGLVQPGGSLKLSCAVSGFSLTDYG
VNWVRQAPGRGLEWIGVIWGDGITDYNSALKDRFIISKDNGKNTVYLQMSKVRSD
DTALYYCVTGLFDYWGQGTLVTVSSYPYDVPDYAGGGGSGGGGSGGGGSGGGGSPK
KKRKVAAAPSGQISNQALALAPSSAPVLAQTMVPSSAMVPLAQPPAPAPVLTPGPPQSLS
APVPKSTQAGEGTLSEALLHLQFDADEDLGALLGNSTDPGVFTDLASVDNSEFQQLLNQ
GVSMSHSTAEPMLMEYPEAITRLVTGSQRPPDPAPTPLGTSGLPNGLSGDEDFSSIADMD

FSALLSQISSSGQGGGGSGFSVDTSALLDLFSPSVTVPDMSLPDLDSSLASIQELLSPQEP
PRPPEAENSSPDSGKQLVHYTAQPLFLLDPGSVDTGSNDLPVLFELGEGSYFSEGDGFAE
DPTISLLTGSEPPKAKDPTVSASEGRGSLLT<CDYKDDDDK>*

The amino acid sequence of the dEpiCas057-10 × GCN4 fusion peptide is shown below (SEQ ID NO: 125, NLS in italics, dEpiCas057 in bold, and GCN4 in < >):

MKRTADGSEFESPKKKRKVHRRRRVREGITMSDTTFRPYKSKLNPNERKMPMVVD
TMNWCDNACSVFFDLFVGLYGGVKIGDVTNFLTEKQDTTTDADLLCAINWFRAMP
VSNPDAVVGPEQLVVRFRDYSGHNPSPMASMYLSAPVNPRYTWVDCRFLYQQMCQ
TLGANLDVDISTLVQTGLLPIQKDTQLNAYSFCSNVFGEGVKEDPSAKVTSYTAAAN
ALSGATNPIITYEDYRQILSSAITNRPSRKGAKDILAADLDSTKTGVLPGNYVSQRIA
MYQASAASKARGVVLPNRLRVKAYLQNLVGLNYICTTWAAPMLRAVGDIKSKNSN
NYRFVGERLERQQEIDNLLAVSNIRSAQAILQQFRANESNQFYIKKRHIGKGLPSLYD
MWDLMDEKEGIDNYIQSLKGDKTERLPIRELLQIIYPHRKSVSAELFLSAAELTDVE
DRQNRCMVHPTVRGSLVINFGKSSTIFGAITPPDTLIKFRGEMVPAGQTGMIWFTM
HLVDGGKWVKHHIPCHSSRYFEEIYAWKKDLPTLPFPRRSLYGYAIGNKISDTRKID
LRRKKASKQYLRTLENMTHNVEFDPTTQFMVDQELNVTITARVKKIPPVRDVPVGS
RIMGLNKNQTIRDCYSIWERVEANTSGSFQHPVVQDVWLRLVADGHVTSTVNGPNG
QIDQMSYSGPDVANCGNWMQERSNFITSVDDADQSIMKHFLWNNRVKLYAWNCVY
AGTLMKAISKQMTTANRNLFRNEILSFVGGRFGCRLGSLAQVSLDLLRRMKSVISC
YFSRNNAFTIEERSAFDKELMELFESLDNKMNNKRKEKVNRTLSSIFQVAKQHNVV
RLVVTKNLQTPDANVRSVMNQRSVDWCARKIEEKIVDLGNVIGIYVDLVEALDLSH
LDPFIYIAKGDVGKECQFDEVESSSLLPIHTSMFRDWHGLVNKGRTTTPIYHSALRSF
ASHYGLRFDDLARIKAAELAKMLPDQKVFIPHRGGRFFMSTYPVTSTSRPVSYDGR
QRFLNSGAMVAPVNTMLRGFRRARSHHHHHHPAAKRVKLDDYKDHDGDYKDHDID
YKDDDDKGSM<EELLSKNYHLENEVARLKK>GSGSG<EELLSKNYHLENEVARLKK>GS
GSG<EELLSKNYHLENEVARLKK>GSGSG<EELLSKNYHLENEVARLKK>GSGSG<EELL
SKNYHLENEVARLKK>GSGSG<EELLSKNYHLENEVARLKK>GSGSG<EELLSKNYHLE

NEVARLKK>GSGSG<EELLSKNYHLENEVARLKK>GSGSG<EELLSKNYHLENEVARLK
K>GSGSG<EELLSKNYHLENEVARLKK>GSGSGTAVNIGGGTGPMDLQRPQGGGGPKKK
RKVGSGVRLQRR*

The fusion peptides scFV-P65-HSF1 and dEpiCas057-10 × GCN4 could be expressed together from the plasmid sequence as set forth in SEQ ID NO: 126.

The above experiments demonstrate that the Cas enzyme provided in the present application, which loses cleavage activity by mutation, is suitable for application scenarios of base editing and epigenetic editing, and is not limited to other application scenarios based on DNA targeting, such as gene activation, gene silencing, chromosome imaging, and the like.

## Claims

1. An isolated Cas enzyme, wherein the Cas enzyme comprises an amino acid sequence as set forth in any one of SEQ ID NOs: 1-58 or a sequence having at least about 80% identity to the amino acid sequence as set forth in any one of SEQ ID NOs: 1-58.

2. The Cas enzyme according to claim 1, wherein the Cas enzyme has a catalytically active domain capable of binding to a target DNA strand and/or a catalytically active domain capable of cleaving the target DNA strand.

3. The Cas enzyme according to any one of claims 1-2, wherein the catalytically active domain comprises an alteration of one or more amino acids such that the Cas enzyme has only the activity of binding to the target DNA strand, or has the activity of binding to the target DNA strand and the activity of cleaving a single strand of the target DNA.

4. A fusion molecule, wherein the fusion molecule comprises the Cas enzyme according to any one of claims 1-3 and one or more heterologous functional domains.

5. The fusion molecule according to claim 4, wherein the one or more heterologous functional domains are capable of regulating the expression of one or more gene products.

6. The fusion molecule according to any one of claims 4-5, wherein the one or more heterologous functional domains are directly or indirectly fused to the Cas enzyme.

7. The fusion molecule according to any one of claims 4-6, wherein the one or more heterologous functional domains are selected from helicases, nucleases, helicase-nucleases, DNA methyltransferases, DNA hydroxymethylases, histone methylases, histone demethylases, histone acetyltransferases, histone deacetylases, phosphatases, kinases, transcriptional (co-) activators, transcriptional repressors, DNA binding proteins, DNA structural proteins, marker proteins, reporter proteins, fluorescent proteins, ligand binding proteins, signal peptides, subcellular localization sequences, antibody epitopes, and affinity purification tags.

8. The fusion molecule according to any one of claims 4-7, wherein the one or more heterologous functional domains have one or more of the following activities: methylase activity, demethylase activity, deaminase activity, transcriptional activation activity, transcriptional repression activity, transcriptional release factor activity, reverse transcriptase activity, histone modification activity, RNA cleavage activity, and nucleic acid binding activity.

9. An engineered, programmable, non-naturally occurring CRISPR-Cas system, wherein the system comprises the Cas enzyme according to any one of claims 1-3 or the fusion molecule according to any one of claims 4-8, and one or more guide RNAs; the one or more guide RNAs target a locus of a nucleic acid molecule encoding one or more gene products in a cell, thereby directing the Cas enzyme or the fusion molecule to bind to and/or cleave the locus of the nucleic acid molecule encoding the one or more gene products; and the Cas enzyme or the fusion molecule does not naturally co-occur with the guide RNA.

10. An engineered, non-naturally occurring vector system, wherein the vector system comprises one or more vectors, and the one or more vectors comprise:
a) a first regulatory element, the first regulatory element is operably linked to one or more guide RNAs, and the one or more guide RNAs are capable of hybridizing to a target sequence in a locus of a nucleic acid molecule encoding one or more gene products, and
b) a second regulatory element, the second regulatory element is operably linked to the Cas enzyme according to any one of claims 1-3 or the fusion molecule according to any one of claims 4-8,
wherein the component a) and the component b) are located on the same vector or different vectors of the vector system, and the guide RNA targets the locus of the nucleic acid molecule encoding the one or more gene products in a cell, thereby directing the Cas enzyme or the fusion molecule to bind to and/or cleave the locus of the nucleic acid molecule encoding the one or more gene products; and the Cas enzyme or the fusion molecule does not naturally co-occur with the guide RNA.

11. The system according to any one of claims 9-10, wherein the expression of the one or more gene products is altered.

12. The system according to any one of claims 9-11, wherein the expression of the gene product is decreased or increased.

13. The system according to any one of claims 9-12, wherein the gene product is a protein.

14. The system according to any one of claims 9-13, wherein the cell is a eukaryotic cell.

15. The system according to any one of claims 9-14, wherein the eukaryotic cell is a mammalian cell.

16. The system according to any one of claims 9-15, wherein the mammalian cell is a human cell.

17. The system according to any one of claims 9-16, wherein the Cas enzyme is codon-optimized for expression in a eukaryotic cell.

18. The system according to any one of claims 9-17, wherein the guide RNA comprises a guide sequence fused to a tracr sequence.

19. The system according to any one of claims 9-18, wherein the guide RNA comprises a direct repeat sequence and a spacer sequence, and the spacer sequence binds to a nucleic acid molecule targeted by the guide RNA.

20. The system according to claim 19, wherein the direct repeat sequence is 10 to 70 nucleotides in length.

21. The system according to claim 19 or 20, wherein the direct repeat sequence is 31 to 36 nucleotides in length.

22. The system according to any one of claims 19-21, wherein the direct repeat sequence comprises a nucleotide sequence as set forth in any one of SEQ ID NOs: 63-88 and 90-99, or comprises a nucleotide sequence having at least 95% sequence identity to the nucleotide sequence as set forth in any one of SEQ ID NOs: 63-88 and 90-99.

23. The system according to claim 19, wherein the spacer sequence is 16 to 24 nucleotides in length.

24. The system according to claim 19 or 23, wherein the nucleic acid molecule targeted by the guide RNA comprises a nucleotide sequence capable of complementary pairing with the spacer sequence.

25. The system according to any one of claims 9-24, wherein the vector or the Cas enzyme of the system further comprises one or more nuclear localization sequences (NLS).

26. The system according to any one of claims 9-25, wherein the system is introduced into the cell by a delivery system, and the delivery system is selected from a virion, a liposome, a lipid nanoparticle, electroporation, microinjection, and conjugation.

27. A method for altering the expression of one or more gene products, wherein the method comprises introducing an engineered, non-naturally occurring CRISPR-Cas system into a cell comprising and expressing a nucleic acid molecule encoding the one or more gene products, and the system comprises the Cas enzyme according to any one of claims 1-3 or the fusion molecule according to any one of claims 4-8, and one or more guide RNAs, and the one or more guide RNAs target a locus of the nucleic acid molecule encoding the one or more gene products, thereby directing the Cas enzyme or the fusion molecule to bind to and/or cleave the locus, thus altering the expression of the one or more gene products; and the Cas enzyme or the fusion molecule does not naturally co-occur with the guide RNA.

28. A method for altering the expression of one or more gene products, wherein the method comprises introducing an engineered, non-naturally occurring vector system into a cell comprising and expressing a nucleic acid molecule encoding the one or more gene products, the system comprises one or more vectors, and the one or more vectors comprises:
a) a first regulatory element, the first regulatory element is operably linked to one or more guide RNAs, and the one or more guide RNAs are capable of hybridizing to a target sequence in a locus of the nucleic acid molecule encoding the one or more gene products, and
b) a second regulatory element, the second regulatory element is operably linked to the Cas enzyme according to any one of claims 1-3 or the fusion molecule according to any one of claims 4-8,
wherein the component a) and the component b) are located on the same vector or different vectors of the vector system, and the guide RNA targets the locus of the nucleic acid molecule encoding the one or more gene products in the cell, thereby directing the Cas enzyme or the fusion molecule to bind to and/or cleave the locus, thus altering the expression of the one or more gene products; and the Cas enzyme or the fusion molecule does not naturally co-occur with the guide RNA.

29. The method according to claim 27 or 28, wherein the expression of the gene product is decreased or increased.

30. The method according to any one of claims 27-29, wherein the gene product is a protein.

31. The method according to any one of claims 27-30, wherein the cell is a eukaryotic cell.

32. The method according to any one of claims 27-31, wherein the eukaryotic cell is a mammalian cell.

33. The method according to any one of claims 27-32, wherein the mammalian cell is a human cell.

34. The method according to any one of claims 27-33, wherein the Cas enzyme is codon-optimized for expression in a eukaryotic cell.

35. The method according to any one of claims 27-34, wherein the guide RNA comprises a guide sequence fused to a tracr sequence.

36. The method according to any one of claims 27-35, wherein the guide RNA comprises a direct repeat sequence and a spacer sequence, and the spacer sequence binds to a nucleic acid molecule targeted by the guide RNA.

37. The method according to claim 36, wherein the direct repeat sequence is 10 to 70 nucleotides in length.

38. The method according to claim 36 or 37, wherein the direct repeat sequence is 31 to 36 nucleotides in length.

39. The method according to any one of claims 36-38, wherein the direct repeat sequence comprises a nucleotide sequence as set forth in any one of SEQ ID NO: 63-88 and 90-99, or comprises a nucleotide sequence having at least 95% sequence identity to the nucleotide sequence as set forth in any one of SEQ ID NOs: 63-88 and 90-99.

40. The method according to claim 36, wherein the spacer sequence is 16 to 24 nucleotides in length.

41. The method according to claim 36 or 40, wherein the nucleic acid molecule targeted by the guide RNA comprises a nucleotide sequence capable of complementary pairing with the spacer sequence.

42. The method according to any one of claims 27-41, wherein the vector or the Cas enzyme of the system further comprises one or more nuclear localization sequences (NLS).

43. The method according to any one of claims 27-42, wherein the method comprises introducing the CRISPR-Cas system or the vector system into the cell by a delivery system, and the delivery system is selected from a virion, a liposome, a lipid nanoparticle, electroporation, microinjection, and conjugation.

44. A nucleic acid, wherein the nucleic acid encodes the Cas enzyme according to any one of claims 1-3, the fusion molecule according to any one of claims 4-8, or the CRISPR-Cas system according to any one of claims 9 and 11-26.

45. A cell, wherein the cell comprises the Cas enzyme according to any one of claims 1-3, the fusion molecule according to any one of claims 4-8, the CRISPR-Cas system according to any one of claims 9 and 11-26, the vector system according to any one of claims 10-26 and/or the nucleic acid according to claim 44.

46. A kit, wherein the kit comprises the Cas enzyme according to any one of claims 1-3, the fusion molecule according to any one of claims 4-8, the CRISPR-Cas system according to any one of claims 9 and 11-26, the vector system according to any one of claims 10-26, the nucleic acid according to claim 44 and/or the cell according to claim 33.

47. The kit according to claim 46, wherein the kit further comprises a container for placing the Cas enzyme, the fusion molecule, the CRISPR-Cas system, the vector system, the nucleic acid and/or the cell, and an instruction for use.
